(19) Europäisches Patentamt
European Patent Office
Office européen des brevets

(11) EP 0 472 722 B1

(12) **EUROPEAN PATENT SPECIFICATION**

(45) Date of publication and mention of the grant of the patent: **21.05.2003 Bulletin 2003/21**

(21) Application number: **91907317.1**

(22) Date of filing: **14.03.1991**

(51) Int Cl.[7]: **C12N 1/21**, C12N 15/29, C12N 15/82, C07H 15/12

(86) International application number: **PCT/US91/01746**

(87) International publication number: **WO 91/013972 (19.09.1991 Gazette 1991/22)**

(54) **DNAS ENCODING PLANT DESATURASES AND THEIR USES**

DNAS, DIE FÜR PFLANZLICHE DESATURASEN KODIEREN UND DEREN ANWENDUNGEN

ADN CODANT POUR DES DESATURASES DE PLANTES ET LEURS EMPLOIS

(84) Designated Contracting States:
**AT BE CH DE DK ES FR GB GR IT LI LU NL SE**

(30) Priority: **16.03.1990 US 494106**
**13.08.1990 US 567373**
**14.11.1990 US 615784**

(43) Date of publication of application:
**04.03.1992 Bulletin 1992/10**

(73) Proprietor: **Calgene LLC**
**Davis, California 95616 (US)**

(72) Inventors:
- **THOMPSON, Gregory, A.**
  **Davis, CA 95616 (US)**
- **KNAUF, Vic, C.**
  **Winters, CA 95694 (US)**

(74) Representative: **Bosch, Henry et al**
**Monsanto Europe S.A.**
**Avenue de Tervuren 270-272**
**PO Box 1**
**1150 Bruxelles (BE)**

(56) References cited:

| | |
|---|---|
| **EP-A- 0 255 377** | **EP-A- 0 255 378** |
| **EP-A- 0 323 753** | **WO-A-90/12084** |
| **WO-A-91/18985** | **NL-A- 8 800 794** |
| **US-A- 4 394 443** | **US-A- 4 446 235** |

- **JOURNAL OF BIOLOGICAL CHEMISTRY. vol. 257, no. 20, 1982, BALTIMORE US pages 12141 - 12147; MCKEON, T. A., ET AL.: 'Purification and characterization of the stearoyl-acyl carrier protein desaturase and acyl-acyl carrier protein thioesterase from maturing seeds of safflower'**
- **JOURNAL OF THE AMERICAN OIL CHEMISTS SOCIETY, vol. 67, no. 4, April 1990, pages 217 - 225; BAFOR, M.,ET AL.: 'Properties of the glycerol acylating enzymes in microsomal preparations from the developing seeds of safflower (Carthamus tinctorius) and turnip rape (Brassica campestris) and their ability to assemble cocoa-butter type fats'**
- **J. CELL. BIOCHEM. SUPPL. vol. 15A, 1991, page 133; KATER, M., ET AL.: 'Purification and cloning of Brassica napus stearoyl-acp desaturase'**
- **J. CELL. BIOCHEM. SUPPL. vol. 14E, 1990, page 266; KNAUF, V. C., ET AL.: 'Reprogramming levels of fatty acid synthesis enzymes in developing embryos of rapeseed'**
- **PLANT PHYSIOLOGY. vol. 90, 1989, ROCKVILLE, MD, USA. pages 760 - 764; CHEESBOROUGH, T. M.: 'Changes in the enzymes for fatty acid synthesis and desaturation during acclimation of developing soybean seeds to altered growth temperature'**
- **CHEMICAL ABSTRACTS, vol. 105, 1986, Columbus, Ohio, US; abstract no. 169077, BROWSE, J.,ET AL.: 'A mutant of Arabidopsis deficient in C18:3 and C16:3 leaf lipids' & PLANT PHYSIOLOGY, vol. 81, no. 3, 1986, ROCKVILLE, MD, USA, pages 859-864**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 472 722 B1

- WORLD SOYBEAN RESEARCH CONFERENCE III, Proceedings (Westview Press): Shibles (ed); Published 1985; GOODMAN et al: "Biotechnology and its impact on future improvements in soybean production and use"; pages 261-271
- JOURNAL OF LIPID RESEARCH, vol. 26, 1985; MATTSON et al.; "Comparison of effects of dietary saturated, monounsaturated, and poly-unsaturated fatty acids on plasma and lipids and lipoprotein in man"; pages 194-202
- TRENDS IN BIOTECHNOLOGY; Volume 5; Issued February 1987; KNAUF "The application of genetic engineering to oilseed crops"; pages 40-47
- TRENDS IN BIOTECHNOLOGY; Volume 7; Issued May 1989; BATTEY et al; "Genetic engineering for plant oils: potential and limitations"; pages 122-126
- METHODS IN ENZYMOLOGY; Volume 71; Issued 1981; McKEON et al; "Stearoyl-acyl carrier protein desaturase from safflower seeds"; pages 275-281
- ARCHIVES OF BIOCHEMISTRY & BIOPHYSICS; Volume 162; Issued 1974; JAWORSKI et al; "Fat metabolism in higher plants, properties of a soluble stearylacyl carrier protein destaurase from maturing Carthamus tinctorius"; pages 158-165
- PROCEEDINGS OF THE FLAX INSTITUTE USA; Volume 41, Number 3; Issued 1971, DOWNEY et al; "Genetic control of fatty acid composition in oilseed crops"; pages 1-3
- JOURNAL OF THE AMERICAN OIL CHEMISTS SOCIETY; Volume 61, Number 1; Issued January 1984; WILCOX et al; "Genetic alteration of soybean oil composition by a chemical mutagen"; pages 97-100
- JOURNAL OF THE AMERICAN OIL CHEMISTS SOCIETY; Volume 59, Number 5; Issued May 1982; WOLF et al: "Effect of temperature on soybean seed constituents: oil, protein, moisture, fatty acids, amino acids and sugars"; pages 200-222
- LIPIDS; Volume 4, Number 6; Issued 1969; INKPEN et al; "Desaturation of palmitate and stearate by cell-free fractions from soybean cotyledons"; pages 539-543
- JOURNAL OF BIOLOGICAL CHEMISTRY; Volume 241; Issued 25 April 1966; NAGAI et al; "Enzymatic desaturation of stearyl acyl carrier protein"; pages 1925-1927
- JOURNAL OF HEREDITY; Volume 80, Number 3; Issued March 1989; MOORE et al; "The inheritance of high oleic acid in peanut"; pages 252-253
- CROP SCIENCE; Volume 24; Issued November-December 1984; CARVER et al; "Developmental changes in acyl-composition of soybean seed selected for high oleic acid concentration"; pages 1016-1019
- BODMAN et al., "Soybeans and Soybean Products: Processing of edible soybean oil" published 1951 by Interscience Publishers, Inc. (N.Y.), pages 649-725

## Description

Technical Field

The present invention is directed to desaturase enzymes relevant to fatty acid synthesis in plants, enzymes, amino acid and nucleic acid sequences and methods related thereto, and novel plant entities and/or oils and methods related thereto.

## INTRODUCTION

Background

Novel vegetable oils compositions and/or improved means to obtain or manipulate fatty acid compositions, from biosynthetic or natural plant sources, are needed. Depending upon the intended oil use, various different oil compositions are desired. For example, edible oil sources containing the minimum possible amounts of saturated fatty acids are desired for dietary reasons and alternatives to current sources of highly saturated oil products, such as tropical oils, are also needed.

One means postulated to obtain such oils and/or modified fatty acid compositions is through the genetic engineering of plants. However, in order to genetically engineer plants one must have in place the means to transfer genetic material to the plant in a stable and heritable manner. Additionally, one must have nucleic acid sequences capable of producing the desired phenotypic result, regulatory regions capable of directing the correct application of such sequences, and the like. Moreover, it should be appreciated that to produce a desired modified oils phenotype requires that the Fatty Acid Synthetase (FAS) pathway of the plant is modified to the extent that the ratios of reactants are modulated or changed.

Higher plants appear to synthesize fatty acids via a common metabolic pathway in plant plastid organelles (i. e., chloroplasts, proplastids, or other related organelles) as part of the FAS complex. Outside of plastid organelles, fatty acids are incorporated into triglycerides and used in plant membranes and in neutral lipids. In developing seeds, where oils are produced and stored as sources of energy for future use, FAS occurs in proplastids.

The production of fatty acids begins in the plastid with the reaction between Acyl Carrier Protein (ACP) and acetyl-CoA to produce acetyl-ACP. Through a sequence of cylical reactions, the acetyl-ACP is elongated to 16- and 18- carbon fatty acids. The longest chain fatty acids produced by the FAS are 18 carbons long. Monunsaturated fatty acids are also produced in the plastid through the action of a desaturase enzyme.

Common plant fatty acids, such as oleic, linoleic and α-linolenic acids, are the result of sequential desaturation of stearate. The first desaturation step is the desaturation of stearoyl-ACP (C18:0) to form oleoyl-ACP (C18:1) in a reaction often catalyzed by a Δ-9 desaturase, also often referred to as a "stearoyl-ACP desaturase" because of its high activity toward stearate the 18 carbon acyl-ACP. The desaturase enzyme functions to add a double bond at the ninth carbon in accordance with the following reaction (I):

$$\text{Stearoyl-ACP} + \text{ferredoxin(II)} + O_2 + 2H^+ \text{->}$$

$$\text{oleoyl-ACP} + \text{ferredoxin (III)} + 2H_2O.$$

Δ-9 desaturases have been studied in partially purified preparations from numerous plant species. Reports indicate that the protein is a dimer, perhaps a homodimer, displaying a molecular weight of 68 kD (±8 kD) by gelfiltration and a molecular weight of 36 kD by SDS-polyacrylamide gel electrophoresis.

In subsequent sequential steps for triglyceride production, polyunsaturated fatty acids may be produced. These desaturations occur outside of the plastid as a result of the action of membrane-bound enzymes. Additional double bonds are added at the twelve position carbon and thereafter, if added, at the 15 position carbon through the action of Δ-12 desaturase and Δ-15 desaturase, respectively.

Obtaining nucleic acid sequences capable of producing a phenotypic result in FAS, desaturation and/or incorporation of fatty acids into a glycerol backbone to produce an oil is subject to various obstacles including but not limited to the identification of metabolic factors of interest, choice and characterization of a protein source with useful kinetic properties, purification of the protein of interest to a level which will allow for its amino acid sequencing, utilizing amino acid sequence data to obtain a nucleic acid sequence capable of use as a probe to retrieve the desired DNA sequence, and the preparation of constructs, transformation and analysis of the resulting plants.

Thus, the identification of enzyme targets and useful plant sources for nucleic acid sequences of such enzyme targets capable of modifying fatty acid compositions are needed. Ideally, an enzyme target will be amenable to one or more applications alone or in combination with other nucleic acid sequences relating to increased/decreased oil production, the ratio of saturated to unsaturated fatty acids in the fatty acid pool, and/or to novel oils compositions as a result of the modifications to the fatty acid pool. Once enzyme target(s) are identified and qualified, quantities of protein and purification protocols are needed for sequencing. Ultimately, useful nucleic acid constructs having the necessary elements to provide a phenotypic modification and plants containing such constructs are needed.

Relevant Literature

A 200-fold purification of *Carthamus tinctorius* ("safflower") stearoyl-ACP desaturase was reported by McKeon & Stumpf in 1982, following the first publication of their protocol in 1981. McKeon, T. & Stumpf, P. *J.Biol.Chem.* (1982) *257*:12141-12147; McKeon, T. & Stumpf, P .*Methods in Enzymol.* (1981) *71*:275-281.

## BRIEF DESCRIPTION OF THE DRAWINGS

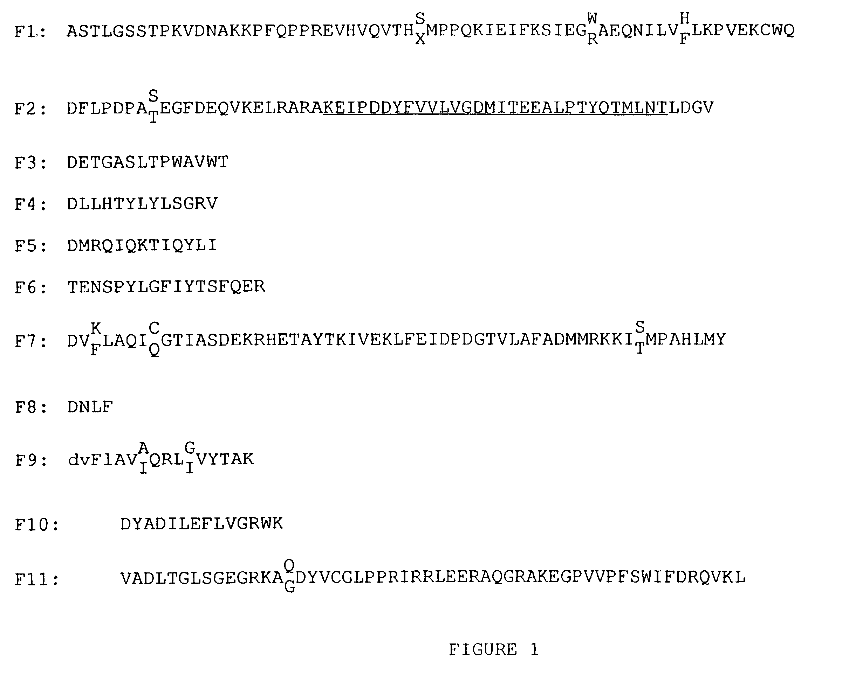

Fig. 1 provides amino acid sequence of fragments relating to *C. tinctorius* desaturase. Fragments F1 through F11 are also provided in the sequence listing as SEQ ID NO: 1 through SEQ ID NO: 11, respectively. Each fragment represents a synthesis of sequence information from peptides originating from different digests which have been matched and aligned. In positions where there are two amino acids indicated, the top one corresponds to that found in the translation of the cDNA; the lower one was detected either as a second signal at the same position of one of the sequenced peptides, or as a single unambiguous signal found in one or more of the overlapping peptides comprising the fragment. Residues in F9 shown in lower case letters represent positions where the called sequence does not agree with that predicted from the cDNA, but where the amino acid assignment is tentative because of the presence of a contaminating peptide. The standard one letter code for amino acid residues has been used. X represents a position where no signal was detectable, and which could be a modified residue. F1 corresponds to the N-terminal sequence of the mature protein. The underlined region in F2 is the sequence used in designing PCR primers for probe synthesis.

Fig. 2 provides a cDNA sequence (SEQ ID NO: 12) and the corresponding translational peptide sequence (SEQ ID NO: 13) derived from *C. tinctorius* desaturase. The cDNA sequence includes both the plastid transit peptide encoding sequence and the sequence encoding the mature protein.

Fig. 3 provides cDNA sequence of *Ricinus communis* desaturase. Fig. 3A provides preliminary partial cDNA sequence of a 1.7 kb clone of *R. communis* desaturase (SEQ ID NO: 14). The sequence is from the 5' end of the clone. Fig. 3B provides the complete cDNA sequence of the approximately 1.7 kb clone (SEQ ID NO: 15) and the corresponding translational peptide sequence (SEQ ID NO: 16).

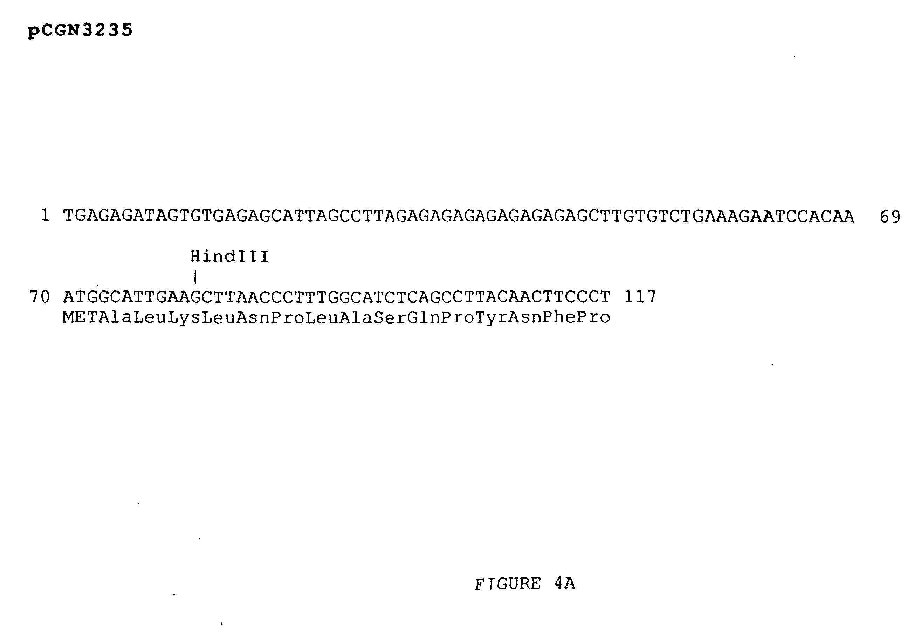

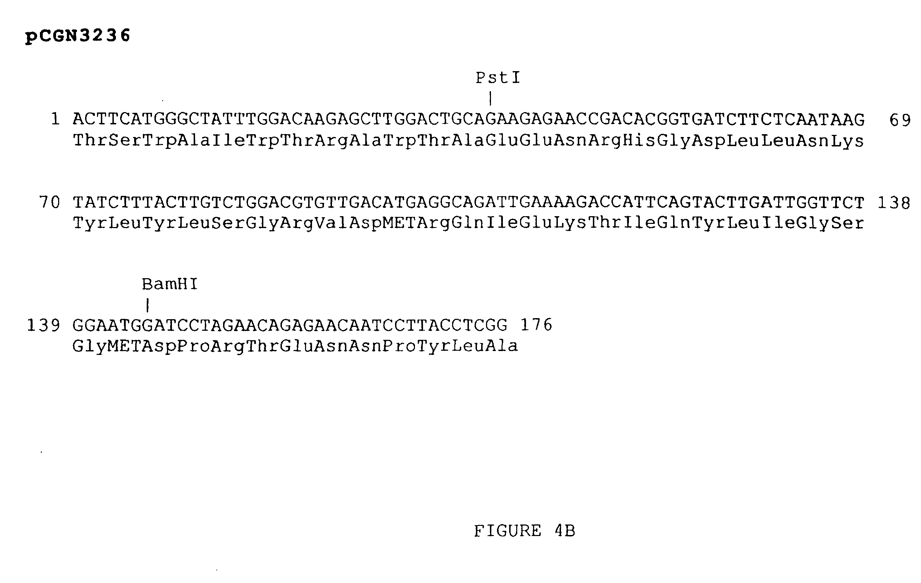

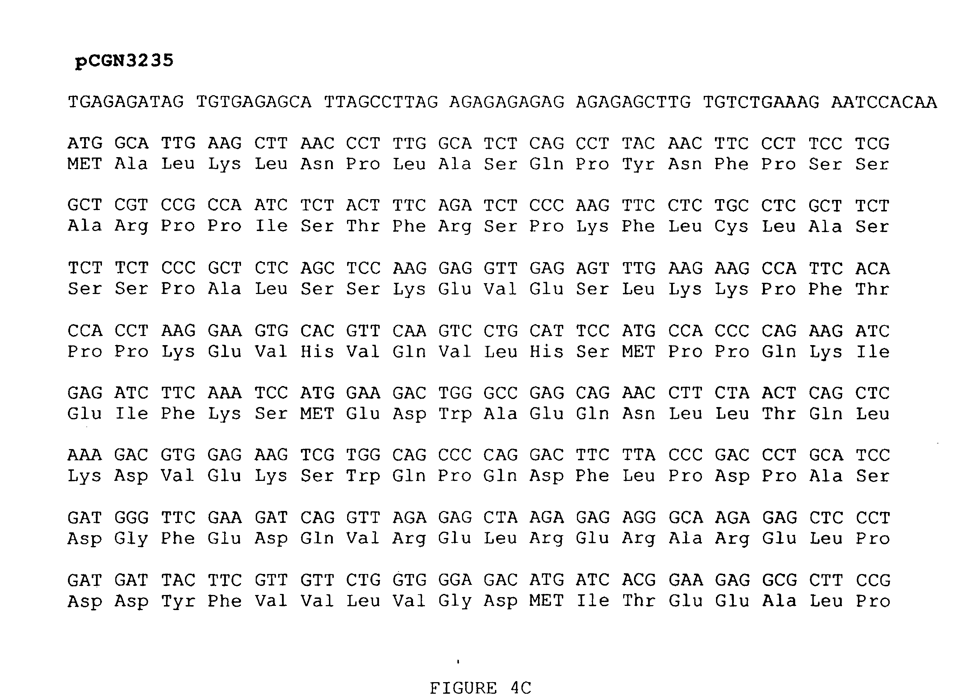

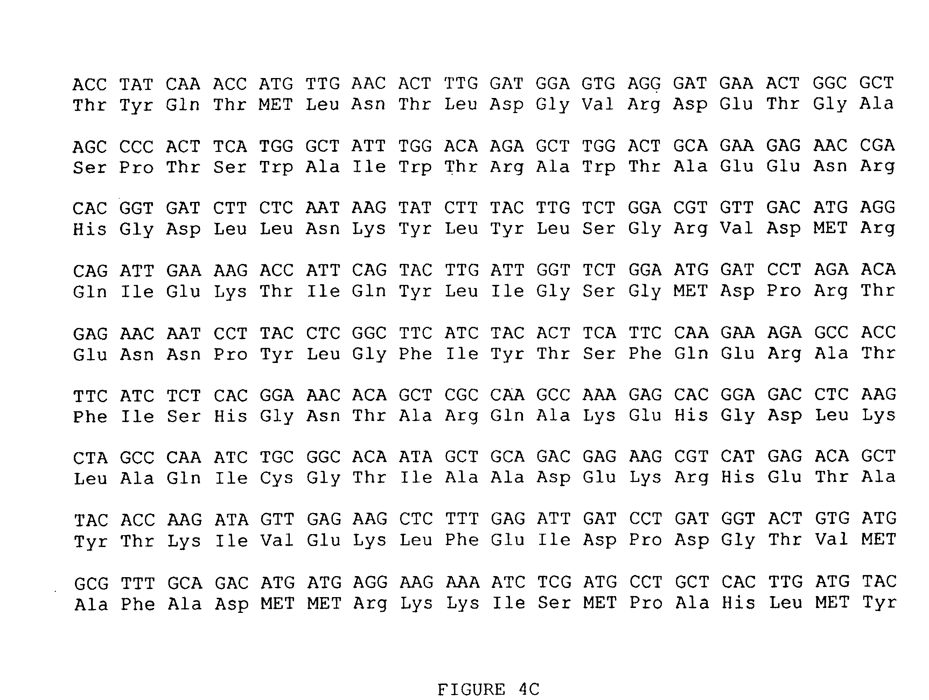

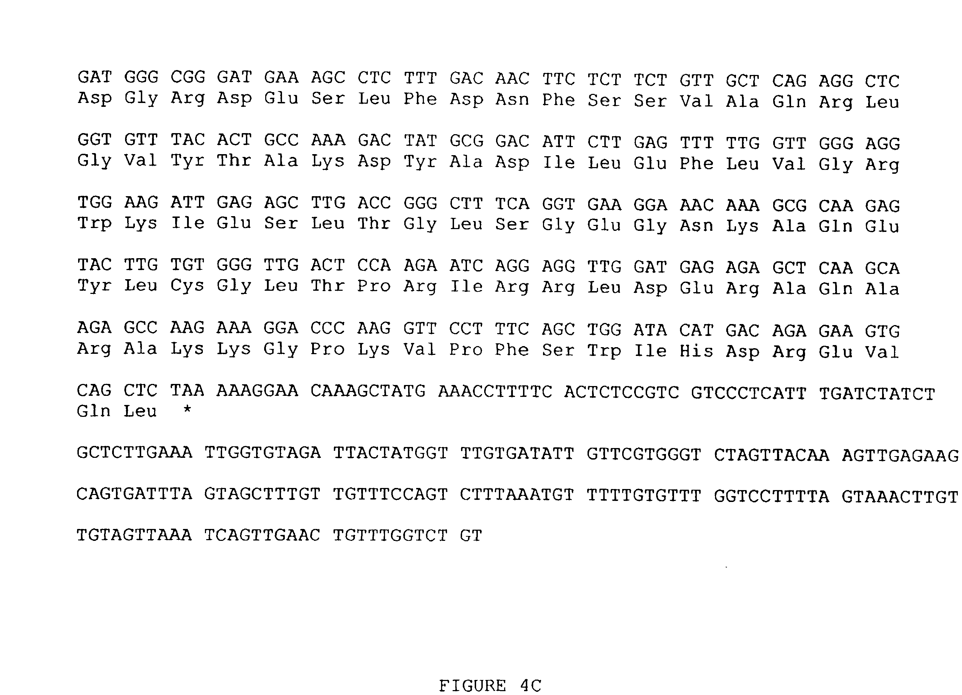

Fig. 4 provides sequence of *Brassica campestris* desaturase. Fig. 4A represents partial DNA sequence of a 1.6 kb clone pCGN3235 (SEQ ID NO: 17), from the 5' end of the clone. Fig. 4B represents partial DNA sequence of a 1.2 kb clone, pCGN3236, from the 5' end of the clone (SEQ ID NO: 18). Initial sequence for the 3' ends of the two *B. campestris* desaturase clones indicates that pCGN3236 is a shorter cDNA for the same clone as pCGN3235. Fig. 4C provides complete cDNA sequence of *B. campestris* desaturase above, pCGN3235 (SEQ ID NO: 19) and the corresponding translational peptide sequence (SEQ ID NO: 20).

Fig. 5 provides preliminary partial cDNA sequence of *Simmondsia chinensis* desaturase (SEQ ID NO: 43). The translated amino acid sequence is also shown.

Fig. 6 shows the design of forward and reverse primers (SEQ ID NO: 21 through SEQ ID NO: 26) used in polymerase chain reaction (PCR) from the sequence of *C. tinctorius* desaturase peptide "Fragment F2" (SEQ ID NO: 2).

Fig. 7 provides maps of desaturase cDNA clones showing selected restriction enzyme sites. Fig. 7A represents a *C. tinctorius* clone, Fig. 7B represents a *R. communis* clone, and Fig. 7C represents a *B. campestris* clone.

Fig. 8 provides approximately 3.4 kb of genomic sequence of Bce4 (SEQ ID NO: 27).

Fig. 9 provides approximately 4 kb of genomic sequence of Bcg 4-4 ACP sequence (SEQ ID NO: 28).

Fig. 10 provides a restriction map of cloned λCGN 1-2 showing the entire napin coding region sequence as well as extensive 5' upstream and 3' downstream sequences (SEQ ID NO: 29).

## SUMMARY OF THE INVENTION

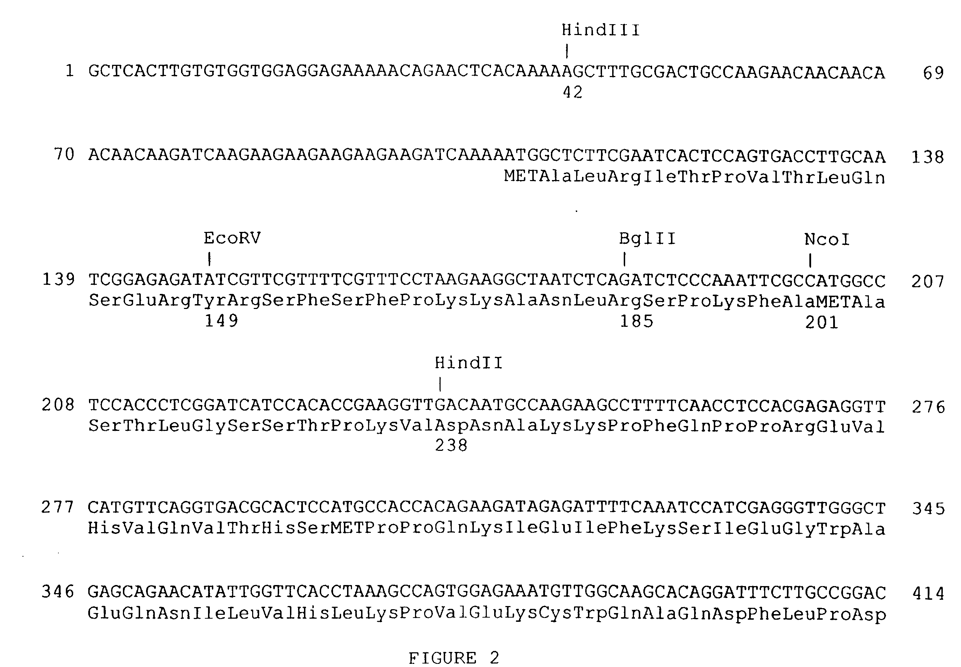

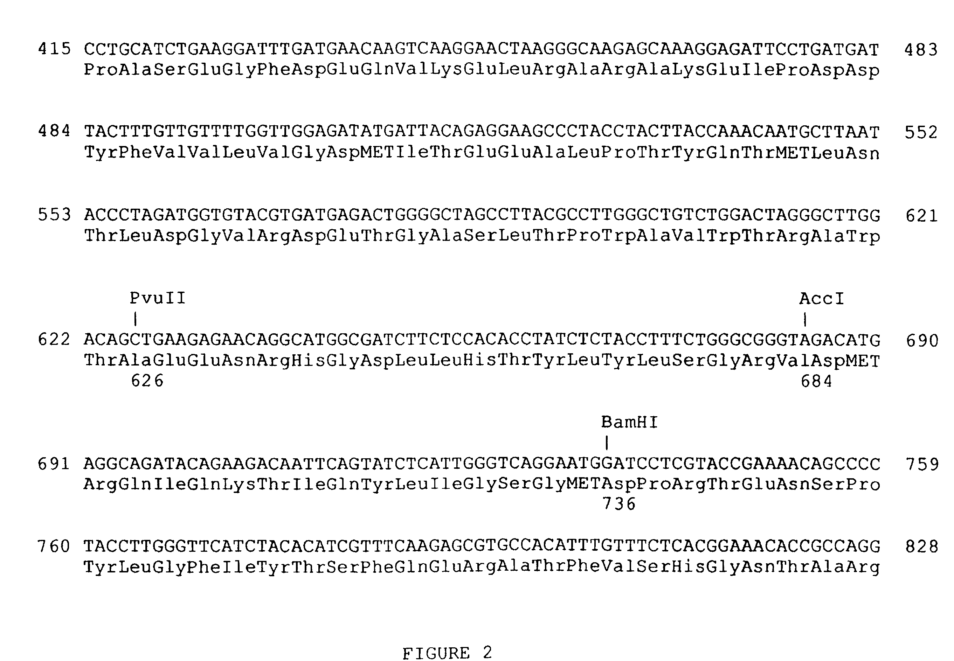

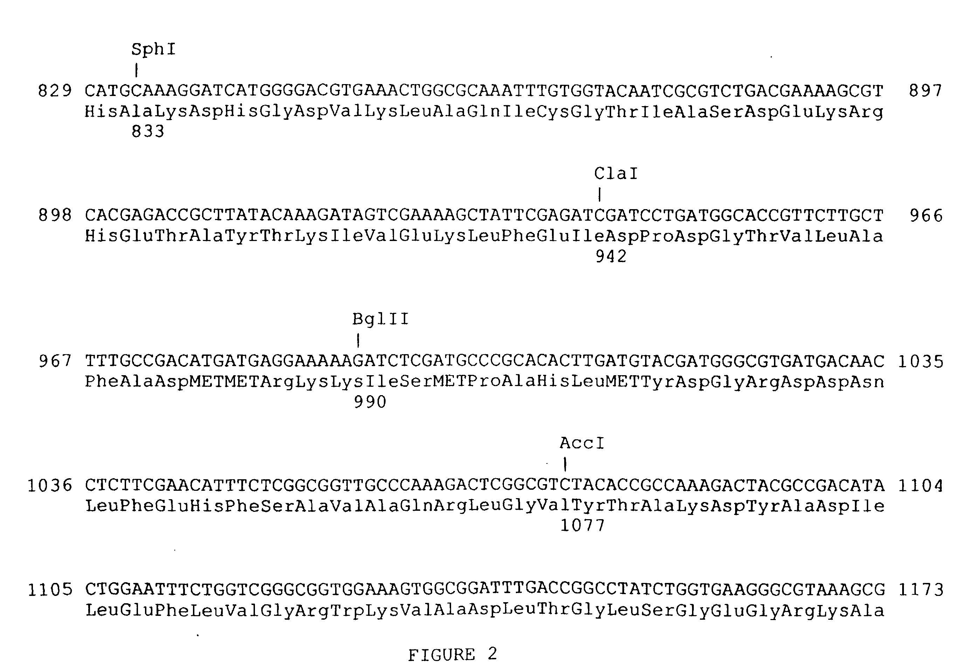

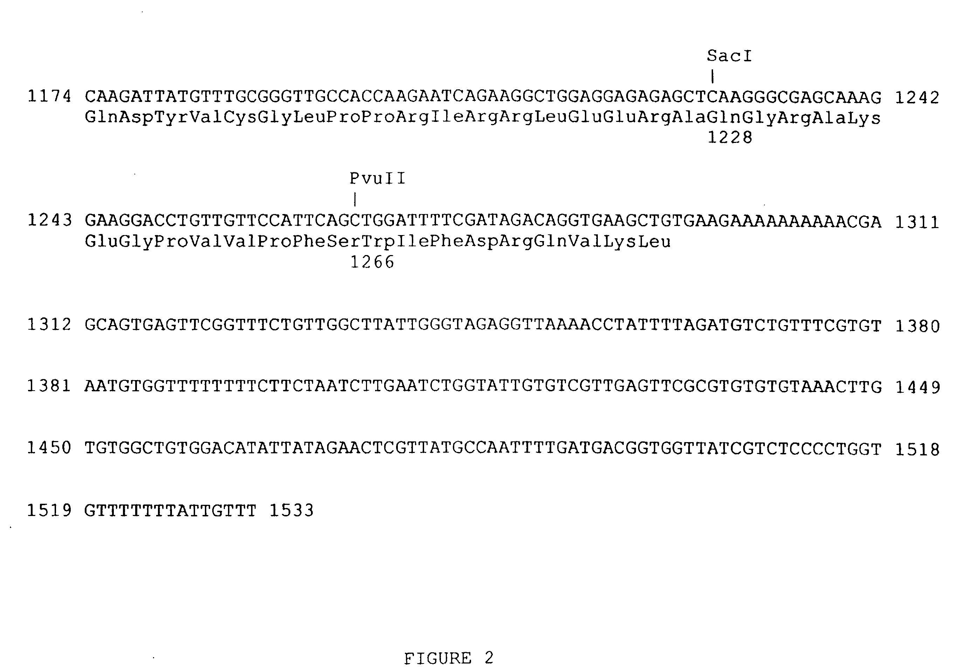

The invention provides an isolated DNA having at least 60% homology to the Δ-9 desaturase DNA coding sequence of Figure 2 and comprising a sequence encoding a Δ-9 desaturase or a portion thereof having Δ-9 desaturase activity; which Δ-9 desaturase is capable of catalysing the insertion of a first double bond into a fatty acyl-ACP moiety, said double bond being inserted at the ninth carbon position.

Preferably, the DNA of the invention is further characterised by comprising a sequence of amino acids as shown in one or more of sequences F1 to F11 in Figure 1.

DNAs of the invention may encode precursor or mature desaturases. They may be obtainable from C.tinctorius, R. communis or B.campestris.

The invention also provides a DNA construct comprising, in the 5' to 3' direction of transcription, a transcriptional regulatory region functional in a host cell and a DNA according to the invention.

Preferably, such a construct further comprises a translational regulatory region immediately 5' to said DNA and a transcriptional/translational termination regulatory region 3' to said DNA, wherein said regulatory regions are functional in a host cell. Preferably, the regulatory regions are functional in a host cell which is a plant cell.

The invention also provides a host cell comprising a construct of the invention. Preferably, The cell is a plant cell. Optionally, the construct is integrated into the genome of the cell.

The invention also provides plants comprising plant cells of the invention.

The invention also provides a method of producing a triglyceride oil from a plant seed comprising growing a plant according to the invention to seed, harvesting the seed and recovering said oil therefrom, which method results in modification of the fatty acid composition in the cells of the plant.

The invention also provides a method of modifying fatty acid composition in a host cell from a given percentage of fatty acid saturation to a different percentage of fatty acid saturation, which method comprises growing a plant cell according to the invention under conditions that promote the activity of the regulatory elements defined at present.

The invention also provides a method of producing a plant desaturase, which method comprises growing a cell according to the invention or a plant or plant seed according to the invention under conditions which permit the production of the plant desaturase.

An aspect of this invention relates to C. tinctorius Δ-9 desaturase. Amino acid sequence of this desaturase is provided in Fig. 2 and as SEQ ID NO: 13.

In a further aspect of this invention DNA constructs comprising a first DNA sequence of the invention and a second DNA sequence which is not naturally found joined to said plant desaturase are provided. This invention also relates to the presence of such constructs in host cells, especially plant host cells. In yet a different aspect, this invention relates to transgenic host cells which have an expressed desaturase therein.

Constructs of this invention may contain, in the 5' to 3' direction of transcription, a transcription initiation control regulatory region capable of promoting transcription in a host cell and a DNA sequence encoding plant desaturase. Transcription initiation control regulatory regions capable of expression in prokatyotic or eukaryotic host cells are provided. Most preferred are transcription initiation control regions capable of expression in plant cells, and more preferred are transcription and translation initiation regions preferentially expressed in plant cells during the period of lipid accumulation. The DNA sequence encoding plant desaturase of this invention may be found in either the sense or anti-sense orientation to the transcription initiation control region.

Specific constructs, expression cassettes having in the 5' to 3' direction of transcription, a transcription and translation initiation control regulatory region comprising sequence immediately 5' to a structural gene preferentially expressed in plant seed during lipid accumulation, a DNA sequence encoding desaturase, and sequence 3' to the structural gene are also provided. The construct may preferably contain DNA sequences encoding plant desaturase obtainable (included obtained) from *Carthamus, Ricinus, Brassica* or *Simmondsia* Δ-9 desaturase genes. Transcription and translation initiation control regulatory regions are preferentially obtained from structural genes preferentially expressed in plant embryo tissue such as napin, seed-ACP or Bce-4.

By this invention, methods and constructs to inhibit the production of endogenous desaturase are also provided.

In yet a different embodiment, this invention is directed to a method of producing plant desaturase in a host cell comprising the steps of growing a host cell comprising an expression cassette, which would contain in the direction of transcription, a) a transcription and translation initiation region functional in said host cell, b) the DNA sequence encoding a plant desaturase in reading frame with said initiation region, and c) and a transcript termination region functional in said host cell, under conditions which will promote the expression of the plant desaturase. Cells containing a plant desaturase as a result of the production of the plant desaturase encoding sequence and also contemplated herein.

By this invention, a method of modifying fatty acid composition in a host plant cell from a given level of fatty acid saturation to a different level of fatty acid saturation is provided by growing a host plant cell having integrated into its genome a recombinant DNA sequence encoding a plant desaturase in either a sense or anti-sense orientation under control of regulatory elements functional in said plant cell during lipid accumulation under conditions which will promote the activity of said regulatory elements. Plant cells having such a modified level of fatty acid saturation are also contemplated hereunder. Oilseeds having such a modified level of fatty acid saturation and oils produced from such oilseeds are further provided.

Constructs of the present invention may comprise a cDNA sequence. The sequence encoding at least a portion of a plant desaturase may be joined to a second nucleic acid sequence which is not naturally joined to said first sequence.

Where the host cell is a plant cell, the transcriptional initiation region may be obtained from a gene preferentially expressed in plant seed tissue during lipid accumulation. The transcriptional initiation region may be selected from the regulatory region 5' upstream to a structural gene of the group consisting of any one of Bce4, seed ACP Bcg 4-4 and napin 1-2. In the constructs, the transcriptional termination region may be a plant desaturase termination region.

Where the Invention provides a plant cell having a modified level of saturated fatty acids, the cell may be a Brassica plant cell, the cell may be in vivo and/or the cell may be an oilseed embryo plant cell.

Where the invention provides a plant seed having a modified level of saturated fatty acids which is produced by growing a plant having integrated into the genome of embryo cells the recombinant DNA sequence as provided, the plant may be Brassica napus. The seed may be an oilseed, the desaturase may be an Δ-9 desaturase.

The present invention also provides a plant seed oil of a plant having an endogenous level of saturated fatty acids comprising a plant seed oil having a modified level of saturated fatty acids. The oil may comprise a Brassica napus oil.

Where the invention provides a host cell comprising a plant desaturase encoding sequence as herein provided, the cell may be a plant cell, the cell may be in vivo and/or the plant cell may be a Brassica plant cell.

The present invention also provides a transgenic host cell comprising an expressed plant desaturase. The host cell may be a plant cell. The plant desaturase may be a Δ-9 desaturase.

In methods of producing a plant desaturase in a host cell or progeny thereof which involve growing a host cell or progeny thereof comprising constructs as herein provided under conditions which will permit the production of the plant desaturase, the host cell may be a plant cell and the construct integrated into the genome of the plant cell. The plant cell may be in vivo. The invention also provides host cells comprising a plant desaturase produced as herein provided. The host cell may be a plant host cell and the construct integrated into the genome of the plant cell.

## DETAILED DESCRIPTION OF THE INVENTION

A desaturase of this invention is encoded by an isolated DNA having at least 60% homology to the Δ-9 desaturase DNA coding sequence of Figure 2 and comprising a sequence encoding a Δ-9 desaturase or a portion thereof having Δ-9 desaturase activity; which Δ-9 desaturase is capable of catalysing the insertion of a first double bond into a fatty acyl-ACP moiety said double bond being inserted at the ninth carbon position. Thus, a desaturase of the invention is a sequence of amino acids, such as a protein, polypeptide, or peptide fragment, preferably obtainable from a plant source, and capable of catalysing the insertion of a first double bond into a fatty acyl-ACP moiety, for example in a plant host cell, i.e., *in vivo,* or in a plant cell-like environment, i.e. *in vitro.* A plant cell-like environment means that any necessary conditions are available in an environment (i.e., such factors as temperatures, pH, lack of inhibiting substances) which will permit the enzyme to function. In particular, this invention relates to enzymes which add such a first double bond at the ninth carbon position in a fatty acyl-ACP chain.

Nucleotide sequences encoding desaturases may be obtained from natural sources or be partially or wholly artificially synthesized. They may directly correspond to a desaturase endogenous to a natural plant source or contain modified amino acid sequences, such as sequences which have been mutated, truncated, increased or the like. Desaturases may be obtained by a variety of methods, including but not limited to, partial or homogenous purification of plant extracts, protein modeling, nucleic acid probes, antibody preparations and sequence comparisons. Typically a plant desaturase will be derived in whole or in part from a natural plant source.

Of special interest are Δ-9 desaturases which are obtainable, including those with are obtained, from *Carthamus, Ricinus, Simmondsia*, or *Brassica*, for example *C. tinctorius, R. communis, S. chinensis* and *B. campestris,* respectively, or from plant desaturases which are obtainable through the use of these sequences. "Obtainable" refers to those desaturases which have sufficiently similar sequences to that of the native sequences provided herein to provide a biologically active desaturase.

Once a DNA sequence which encodes a desaturase is obtained, it may be employed as a gene of interest in a nucleic acid construct or in probes in accordance with this invention. A desaturase may be produced in host cells for harvest or as a means of effecting a contact between the desaturase and its substrate. Constructs may be designed to produce desaturase in either prokaryotic or eukaryotic cells. Plant cells containing recombinant constructs encoding biologically active desaturase sequences, both expression and anti-sense constructs, as well as plants and cells containing modified levels of desaturase proteins are of special interest. For use in a plant cell, constructs may be designed which will effect an increase or a decrease in amount of endogenous desaturase available to a plant cell transformed with such a construct.

Where the target gene encodes an enzyme, such as a plant desaturase, which is already present in the host plant, there are inherent difficulties in analyzing mRNA, engineered protein or enzyme activity, and modified fatty acid composition or oil content in plant cells, especially in developing seeds; each of which can be evidence of biological activity. This is because the levels of the message, enzyme and various fatty acid species are changing rapidly during the stage where measurements are often made, and thus it can be difficult to discriminate between changes brought about by the presence of the foreign gene and those brought about by natural developmental changes in the seed. Where an expressed Δ-9 desaturase DNA sequence is derived from a plant species heterologous to the plant host into which the sequence is introduced and has a distinguishable DNA sequence, it is often possible to specifically probe for expression of the foreign gene with oligonucleotides complimentary to unique sequences of the inserted DNA/RNA. And, if the foreign gene codes for a protein with slightly different protein sequence, it may be possible to obtain antibodies which recognize unique epitopes on the engineered protein. Such antibodies can be obtained by mixing the antiserum to the foreign protein with extract from the host plant, or with extracts containing the host plant enzyme. For example, one can isolate antibodies uniquely specific to a *C. tinctorius* Δ-9 desaturase by mixing antiserum to the desaturase with an extract containing a *Brassica* Δ-9 desaturase. Such an approach will allow the detection of *C. tinctorius* desaturase in *Brassica* plants transformed with the *C. tinctorius* desaturase gene. In plants expressing an endogenous gene in an antisense orientation, the problem is slightly different. In this case, there are no specific reagents to measure expression of a foreign protein. However, one is attempting to measure a decrease in an enzyme activity that normally is increasing during development. This makes detection of expression a simpler matter. In the final seed maturation phase, enzyme activities encoded by genes affecting oil composition usually disappear and cannot be detected in final mature seed. Analysis of the fatty acid content may be preformed by any manner known to those skilled in the art, including gas chromatography, for example.

By increasing the amount of desaturase available in the plant cell, an increased percentage of unsaturated fatty acids may be provided; by decreasing the amount of desaturase, an increased percentage of saturated fatty acids may be provided. (Modifications in the pool of fatty acids available for incorporation into triglycerides may likewise affect the composition of oils in the plant cell.) Thus, an increased expression of desaturase in a plant cell may result in increased proportion of fatty acids, such as one or more of palmitoleate (C16:1), oleate (C18:1), linoleate (C18:2) and linolenate (C18:3) are expected. Of special interest is the production of triglycerides having increased levels of oleate. Of special interest is the production of triglycerides having increased levels of stearate or palmitate and stearate. In addition, the production of a variety of ranges of such saturates is desired. Thus, plant cells having lower and higher levels of stearate fatty acids are contemplated. For example, fatty acid compositions, *including* oils, having a 10% level of stearate as well as compositions designed to have up to a 60% level of stearate or other such modified fatty acid (s) composition are contemplated.

The modification of fatty acid compositions may also affect the fluidity of plant membranes. Different lipid concentrations have been observed in cold-hardened plants, for example. By this invention, one may be capable of introducing traits which will lend to chill tolerance. Constitutive or temperature inducible transcription initiation regulatory control regions may have special applications for such uses.

Other applications for use of cells or plants producing desaturase may also be found. For example, potential herbicidal agents selective for plant desaturase may be obtained through screening to ultimately provide environmentally safe herbicide products. The plant desaturase can also be used in conjunction with chloroplast lysates to enhance the production and/or modify the composition of the fatty acids prepared *in vitro.* The desaturase can also be used for studying the mechanism of fatty acid formation in plants and bacteria. For these applications, constitutive promoters may find the best use.

Constructs which contain elements to provide the transcription and translation of a nucleic acid sequence of interest in a host cell are "expression cassettes". Depending upon the host, the regulatory regions will vary, including regions from structural genes from viruses, plasmid or chromosomal genes, or the like. For expression in prokaryotic or eukaryotic microorganisms, particularly unicellular hosts, a wide variety of constitutive or regulatable promoters may be employed. Among transcriptional initiation regions which have been described are regions from bacterial and yeast hosts, such as *E. coli, B. subtilis, Saccharomyces cerevisiae*, including genes such as β-galactosidase, T7 polymerase, trp E and the like.

A recombinant construct for expression of desaturase in a plant cell ("expression cassette") will include, in the 5' to 3' direction of transcription, a transcription and translation initiation control regulatory region (the transcriptional and translational initiation regions together often also known as a "promoter") functional in a plant cell, a nucleic acid sequence encoding a plant desaturase, and a transcription termination region. Numerous transcription initiation regions are available which provide for a wide variety of constitutive or regulatable, e.g., inducible, transcription of the desaturase structural gene. Among transcriptional initiation regions used for plants are such regions associated with cauliflower mosaic viruses (35S, 19S), and structural genes such as for nopaline synthase or mannopine synthase or napin and ACP promoters, etc. The transcription/translation initiation regions corresponding to such structural genes are found immediately 5' upstream to the respective start codons. Thus, depending upon the intended use, different promoters may be desired.

Of special interest in this invention are the use of promoters which are capable of preferentially expressing the desaturase in seed tissue, in particular, at early stages of seed oil formation. Examples of such seed-specific promoters include the region immediately 5' upstream of napin or seed ACP genes, such as described in co-pending USSN 147,781, and the Bce-4 gene such as described in co-pending USSN 494,722. Alternatively, the use of the 5' regulatory region associated with an endogenous plant desaturase structural gene and/or the transcription termination regions found immediately 3' downstream to the gene, may often be desired.

In addition, for some applications, use of more than one promoter may be desired. For example, one may design a dual promoter expression cassette each promoter having a desaturase sequence under its regulatory control. For example, the combination of an ACP and napin cassette could be useful for increased production of desaturase in a seed-specific fashion over a longer period of time than either individually.

Other manners of decreasing the amount of endogenous plant, desaturase, such as ribozymes or the screening of plant cells transformed with constructs for rare events containing sense sequences which in fact act to decrease desaturase expression, are also contemplated herein. Other analogous methods may be applied by those of ordinary skill in the art.

By careful selection of plants, transformants having particular oils profiles may be obtained. This may in part depend upon the qualities of the transcription initiation region(s) employed or may be a result of culling transformation events to exploit the variabilities of expression observed.

In order to obtain the nucleic acid sequences encoding *C. tinctorius* desaturase, a protein preparation free of a major albumin-type contaminant is required. As demonstrated more fully in the Examples, the protocols of McKeon and Stumpf, *supra*, result in a preparation contaminated with a seed storage protein. Removal of the protein contaminant may be effected by application of a reverse-phase HPLC, or alternatively, by application of a reduction and alkylation step followed by electrophoresis and blotting, for example. Other purification methods may be employed as well, now that the presence of the contaminant is confirmed and various properties thereof described. Once the purified desaturase is obtained it may be used to obtain the corresponding amino acid and/or nucleic acid sequences thereto in accordance with methods familiar to those skilled in the art. Approximately 90% of the total amino acid sequence of the *C. tinctorius* desaturase is provided in Fig. 1 and in SEQ ID NOS: 1-11. The desaturase produced in accordance with the subject invention can be used in preparing antibodies for assays for detecting plant desaturase from other sources.

A nucleic acid sequence of this invention may include genomic or cDNA sequence and mRNA. A cDNA sequence may or may not contain pre-processing sequences, such as transit peptide sequences. Transit peptide sequences facilitate the delivery of the protein to a given organelle and are cleaved from the amino acid moiety upon entry into the organelle, releasing the "mature" sequence.

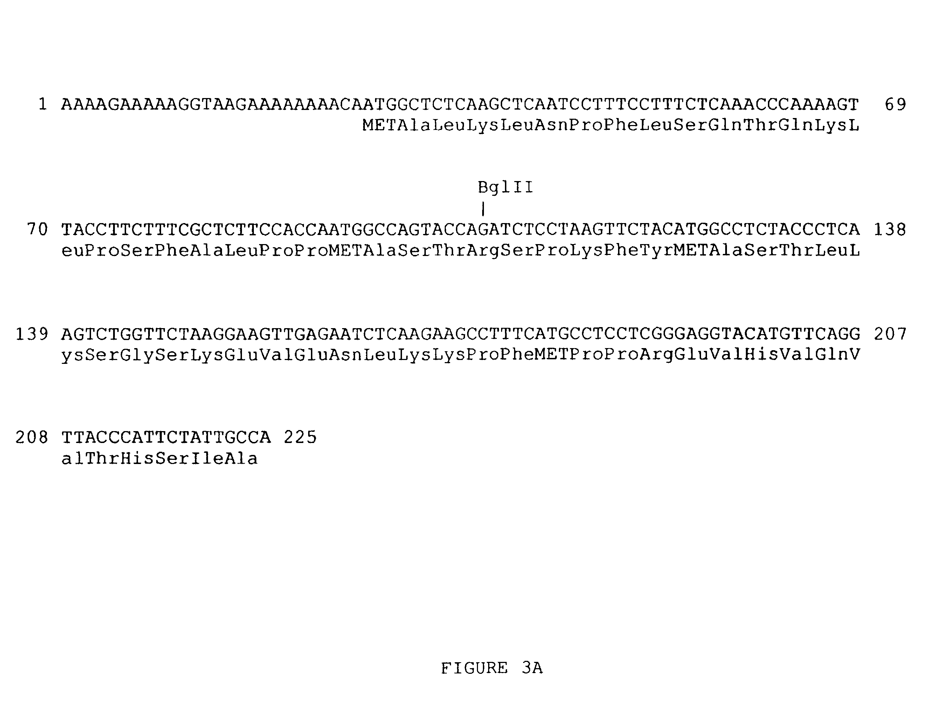

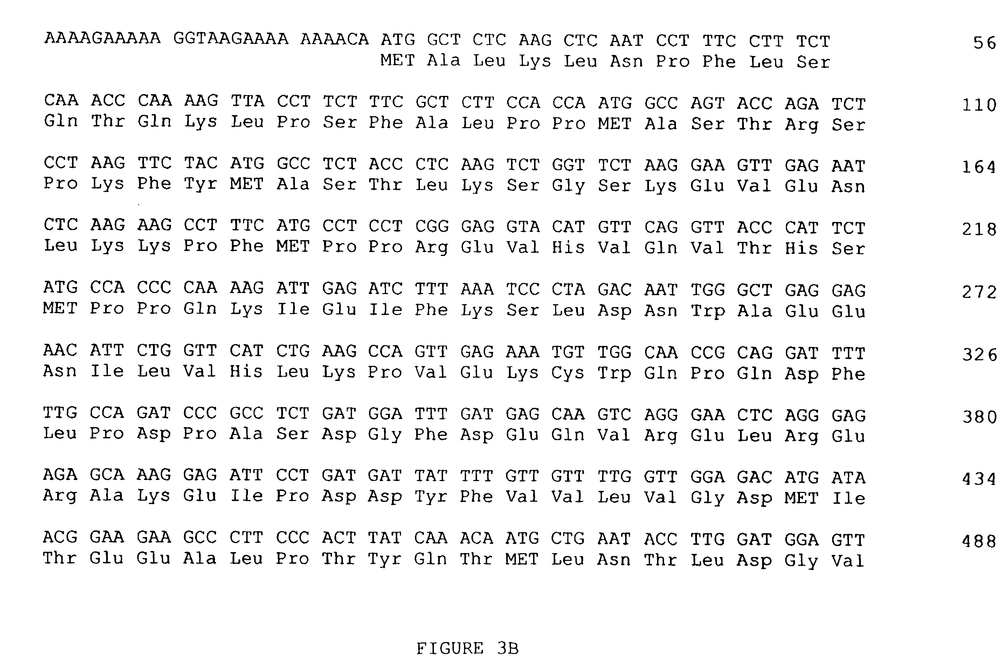

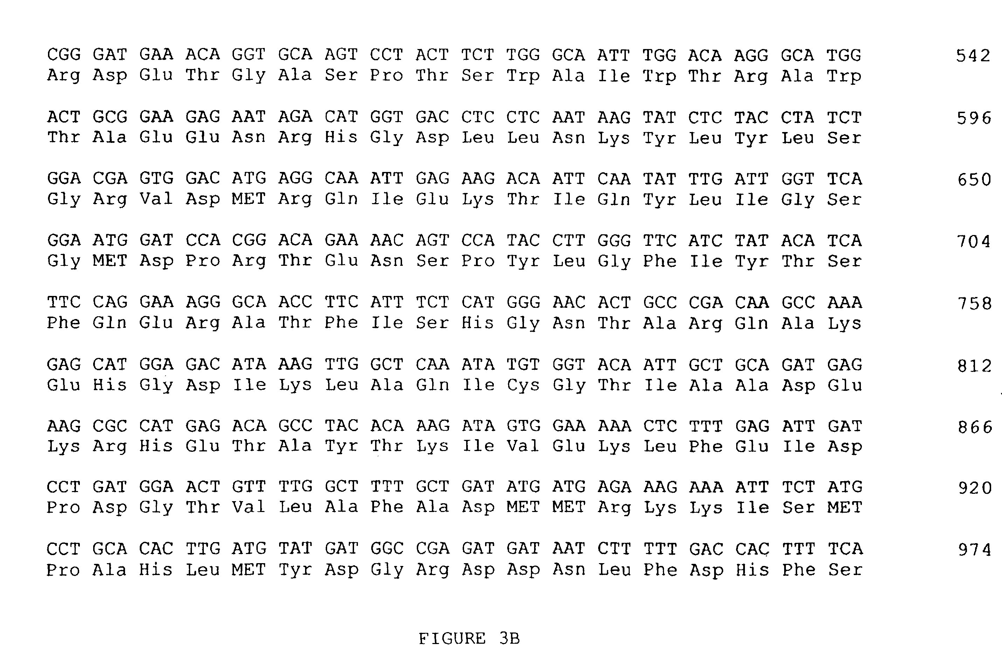

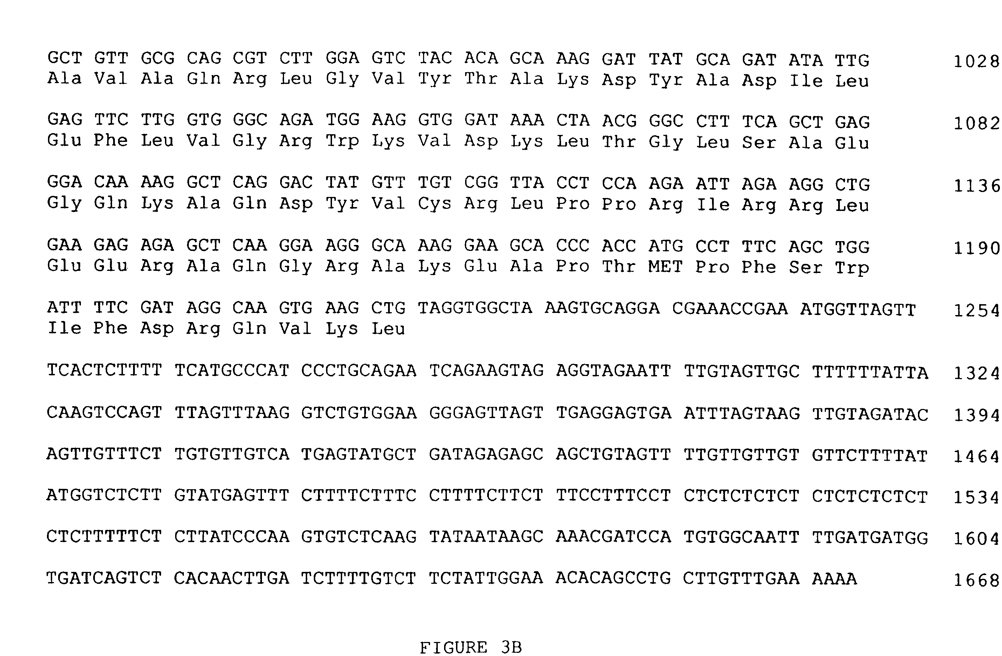

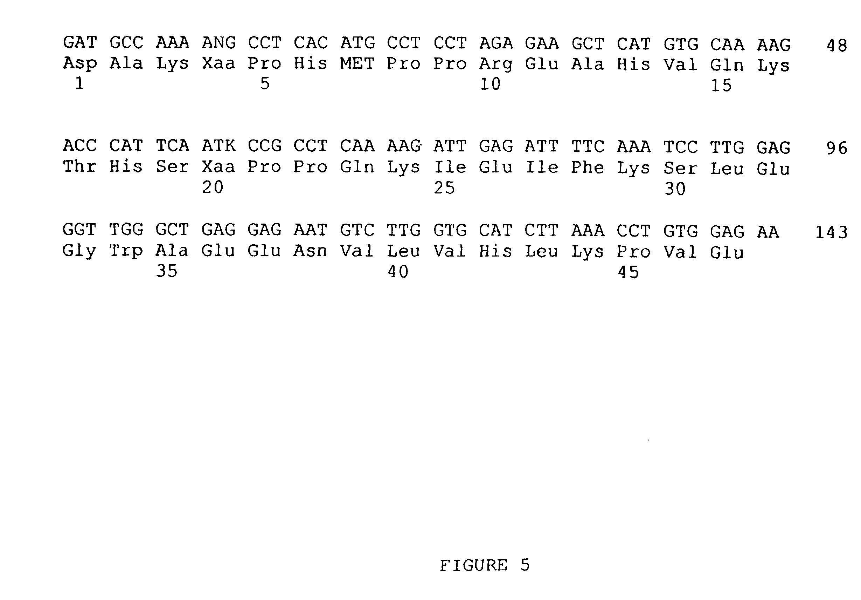

In Fig. 2 and SEQ ID NO: 13, the sequence of the *C. tinctorius* desaturase precursor protein is provided; both the transit peptide and mature protein sequence are shown. Also provided in this invention are cDNA sequences relating to *R. communis* desaturase (Fig.3 and SEQ ID NOS: 14-15), *B. campestris* desaturase (Fig. 4 and SEQ ID NOS: 17-19) and *S. chinesis* (Fig. 5 and SEQ ID NOS: 43).

The use of the precursor cDNA sequence is preferred in desaturase expression cassettes. In addition, desaturase transit peptide sequences may be employed to translocate other proteins of interest to plastid organelles for a variety of uses, including the modulation of other enzymes related to the FAS pathway. See, European Patent Application Publication No. 189, 707.

As described in more detail below, the complete genomic sequence of a desaturase may be obtained by the screening of a genomic library with a desaturase cDNA probe and isolating those sequences which regulate expression in seed tissue. In this manner, the transcription, translation initiation regions and/or transcript termination regions of the desaturase may be obtained for use in a variety of DNA constructs, with or without the respective desaturase structural gene.

Other nucleic acid sequences "homologous" or "related" to DNA sequences encoding other desaturases are also provided. "Homologous" or "related" includes those nucleic acid sequences which are identical or conservatively substituted as compared to the exemplified *C. tinctorius, R. communis, S. chinesis* or *B. campestris* desaturase sequences of this invention or a plant desaturase which has in turn been obtained from a plant desaturase of this invention. By conservatively substituted is meant that codon substitutions encode the same amino acid, as a result of the degeneracy of the DNA code, or that a different amino acid having similar properties to the original amino acid is substituted. One skilled in the art will readily recognize that antibody preparations, nucleic acid probes (DNA and RNA) sequences encoding and the like may be prepared and used to screen and recover desaturase from other plant sources. Typically, nucleic acid probes are labeled to allow detection, preferably with radioactivity although enzymes or other methods may also be used. For immunological screening methods, antibody preparations either monoclonal or polyclonal are utilized. Polyclonal antibodies, although less specific, typically are more useful in gene isolation. For detection, the antibody is labeled using radioactivity or any one of a variety of second antibody/enzyme conjugate systems that are commercially available. Examples of some of the available antibody detection systems are described by Oberfilder (*Focus* (1989) BRL Life Technologies, Inc., *11*:1-5).

A "homologous" or "related" nucleic acid sequence will show at least about 60% homology, and more preferably at least about 70% homology, between the known desaturase sequence and the desired candidate plant desaturase of interest, excluding any deletions which may be present. Homology is determined upon comparison of sequence information, nucleic acid or amino acid, or through hybridization reactions. Amino acid sequences are considered homologous by as little as 25% sequence identity between the two complete mature proteins. (*See generally*, Doolittle, R.F., of URFS and ORFS, University Science Books, CA, 1986.)

Oligonucleotide probes can be considerably shorter than the entire sequence, but should be at least about 10, preferably at least about 15, more preferably at least 20 nucleotides in length. When shorter length regions are used for comparison, a higher degree of sequence identity is required than for longer sequences. Shorter probes are often particularly useful for polymerase chain reactions (PCR), especially when highly conserved sequences can be identified. (*See*, Gould, *et al.*, *PNAS USA* (1989) *86*:1934-1938.) Longer oligonucleotides are also useful, up to the full length of the gene encoding the polypeptide of interest. When longer nucleic acid fragments are employed (>100 bp) as probes, especially when using complete or large cDNA sequences, one would screen with low stringencies (for example 40-50°C below the melting temperature of the probe) in order to obtain signal from the target sample with 20-50% deviation, i.e., homologous sequences. (*See*, Beltz, *et al., Methods in Enzymology* (1983) *100*:266-285.) Both DNA and RNA probes can be used.

A genomic library prepared from the plant source of interest may be probed with conserved sequences from a known desaturase to identify homologously related sequences, Use of the entire cDNA may be employed if shorter probe sequences are not identified. Positive clones are then analyzed by restriction enzyme digestion and/or sequencing. When a genomic library is used, one or more sequences may be identified providing both the coding region, as well as the transcriptional regulatory elements of the desaturase gene from such plant source. In this general manner, one or more sequences may be identified providing both the coding region, as well as the transcriptional regulatory elements of the desaturase gene from such plant source.

In use, probes are typically labeled in a detectable manner (for example with $^{32}P$-labeled or biotinylated nucleotides) and are incubated with single-stranded DNA or RNA from the plant source in which the gene is sought, although unlabeled oligonucleotides are also useful. Hybridization is detected by means of the label after single-stranded and double-stranded (hybridized) DNA or DNA/RNA have been separated, typically using nitrocellulose paper or nylon membranes. Hybridization techniques suitable for use with oligonucleotides are well known to those skilled in the art. Thus, plant desaturase genes may be isolated by various techniques from any convenient plant. Plant desaturase of developing seed obtained from other oilseed plants, such as soybean, coconut, oilseed rape, sunflower, oil palm, peanut, cocoa, cotton, corn and the like are desired as well as from non-traditional oil sources, including but not limited to spinach chloroplast, avocado mesocarp, cuphea, California Bay, cucumber, carrot, meadowfoam, *Oenothera* and *Euglena gracillis.*

Once the desired plant desaturase sequence is obtained, it may be manipulated in a variety of ways. Where the sequence involves non-coding flanking regions, the flanking regions may be subjected to resection, mutagenesis, etc. Thus, transitions, transversions, deletions, and insertions may be performed on the naturally occurring sequence. In addition, all or part of the sequence may be synthesized, where one or more codons may be modified to provide for a modified amino acid sequence, or one or more codon mutations may be introduced to provide for a convenient restriction site or other purpose involved with construction or expression. The structural gene may be further modified by employing synthetic adapters, linkers to introduce one or more convenient restriction sites, or the like.

Recombinant constructs containing a nucleic acid sequence encoding a desaturase of this invention may be combined with other, i.e. "heterologous," DNA sequences in a variety of ways. By heterologous DNA sequences is meant any DNA sequence which is not naturally found joined to the native desaturase, including combinations of DNA sequences from the same plant of the plant desaturase which are not naturally found joined together. In a preferred embodiment, the DNA sequence encoding a plant desaturase is combined in a DNA construct having, in the 5' to 3' direction of transcription, a transcription initiation control region capable of promoting transcription in a host cell, and a DNA sequence encoding a desaturase in either a sense or anti-sense orientation. As described in more detail elsewhere, a variety of regulatory control regions containing transcriptional or transcriptional and translational regions may be employed, including all or part of the non-coding regions of the plant desaturase.

The open reading frame coding for the plant desaturase or functional fragment thereof will be joined at its 5' end to a transcription initiation regulatory control region. In some instances, such as modulation of plant desaturase via a desaturase in an anti-sense orientation, a transcription initiation region or transcription/ translation initiation region may be used. In embodiments wherein the expression of the desaturase protein is desired in a plant host, a transcription/ translation initiation regulatory region, is needed. Additionally, modified promoters, i.e., having transcription initiation regions derived from one gene source and translation initiation regions derived from a different gene source or enhanced promoters, such as double 35S CaMV promoters, may be employed for some applications.

As described above, of particular interest are those 5' upstream non-coding regions which are obtained from genes regulated during seed maturation, particularly those preferentially expressed in plant embryo tissue, such as ACP-and napin-derived transcription initiation control regions. Such regulatory regions are active during lipid accumulation and therefore offer potential for greater control and/or effectiveness to modify the production of plant desaturase and/or modification of the fatty acid composition. Especially of interest are transcription initiation regions which are preferentially expressed in seed tissue, i.e., which are undetectable in other plant parts. For this purpose, the transcript initiation region of acyl carrier protein isolated from *B. campestris* seed and designated as "Bcg 4-4" and an unidentified gene isolated from *B. campestris* seed and designated as "Bce-4" are also of substantial interest.

Briefly, Bce4 is found in immature embryo tissue at least as early as 11 days after anthesis (flowering), peaking about 6 to 8 days later or 17-19 days post-anthesis, and becoming undetectable by 35 days post-anthesis. The timing of expression of the Bce4 gene closely follows that of lipid accumulation in seed tissue. Bce4 is primarily detected in seed embryo tissue and to a lesser extent found in the seed coat. Bce4 has not been detected in other plant tissues tested, root, stem and leaves.

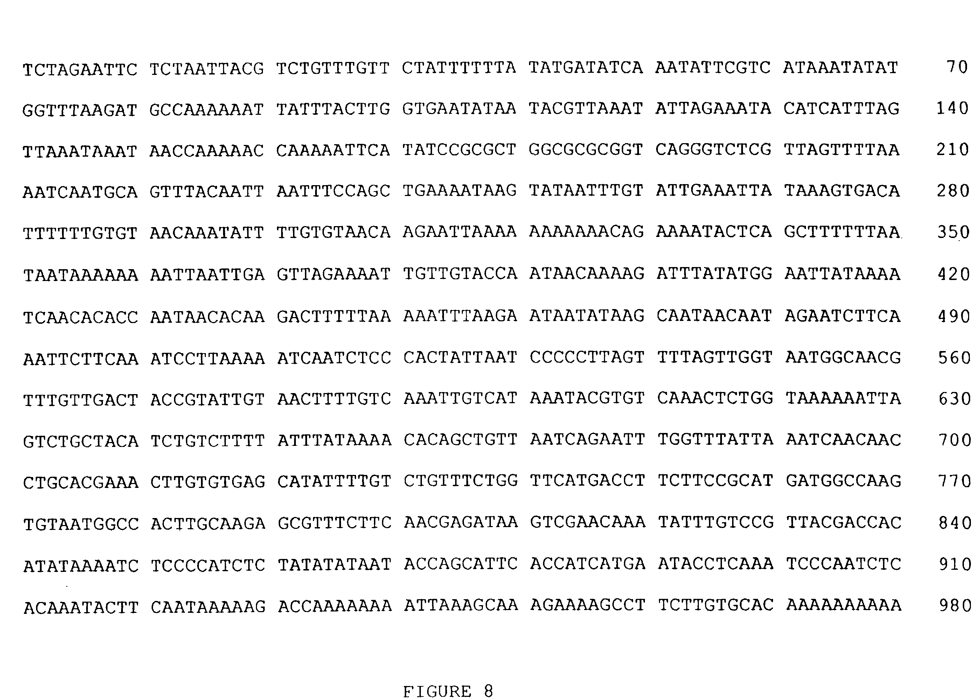

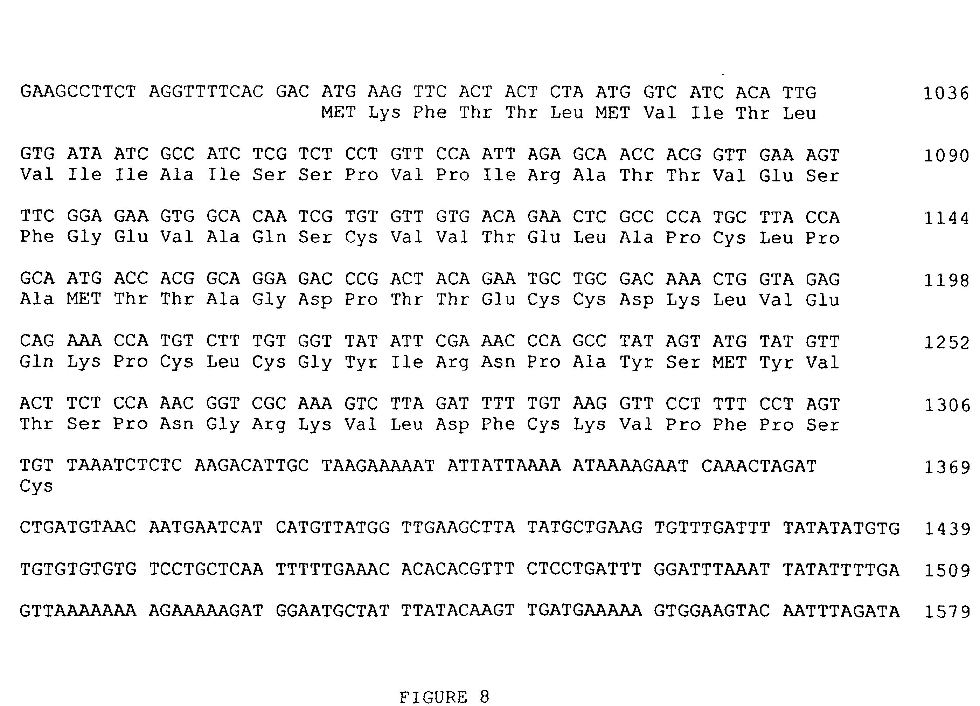

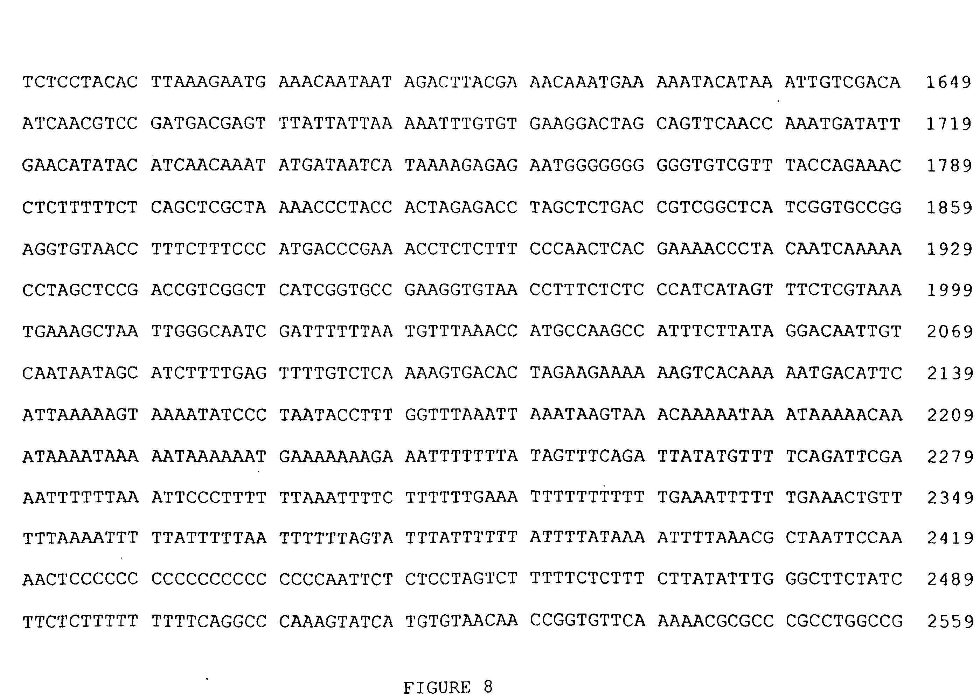

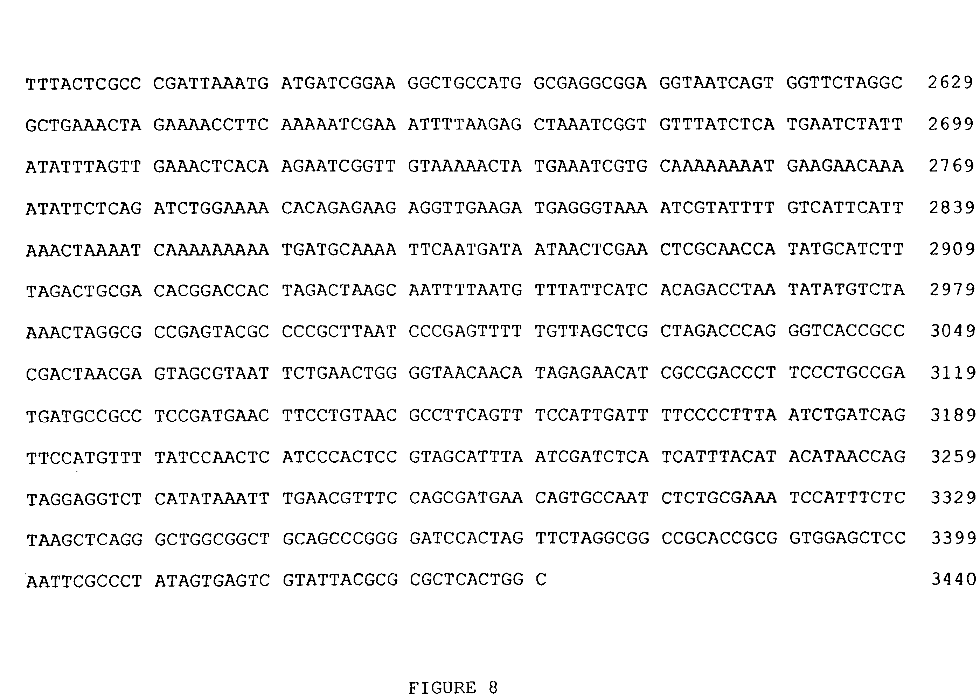

Approximately 3.4 kb genomic sequence of Bce4 is provided in Fig. 8 and as SEQ ID NO: 27, including about 1 kb 5' to the structural gene, about 0.3 kb of the Bce4 coding gene sequence, and about 2.1 kb of the non-coding regulatory 3' sequence. Bce4 transcript initiation regions will contain at least 1 kb and more preferably about 5 to about 7.5 kb of sequence immediately 5' to the Bce4 structural gene.

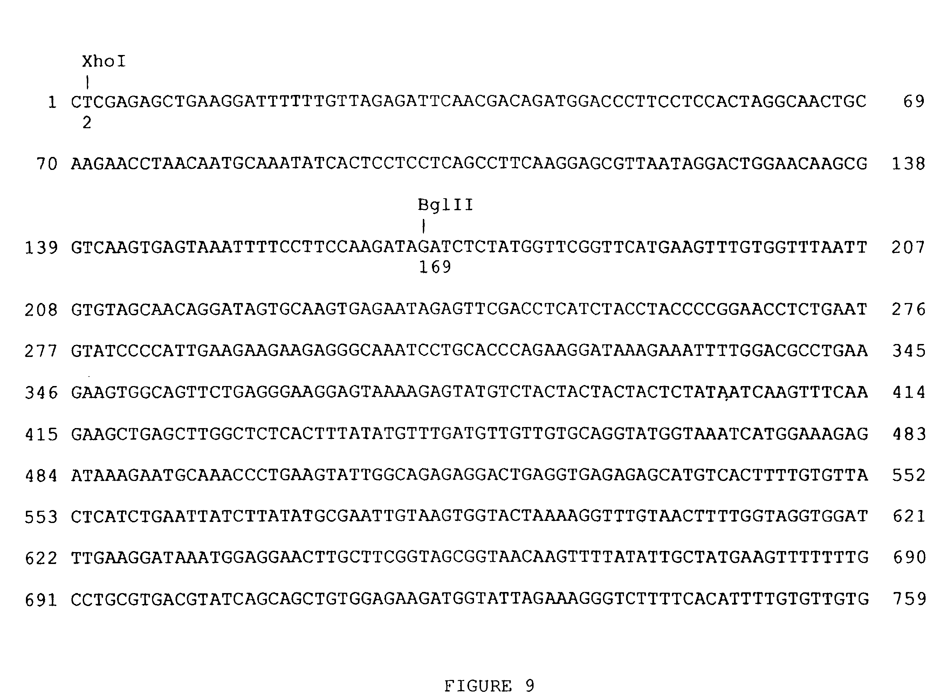

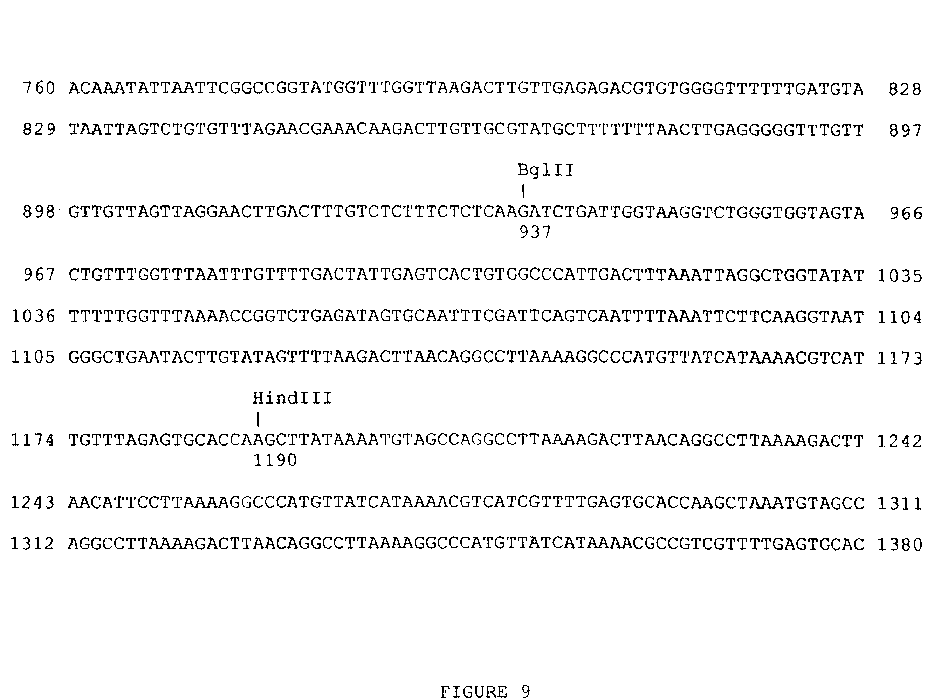

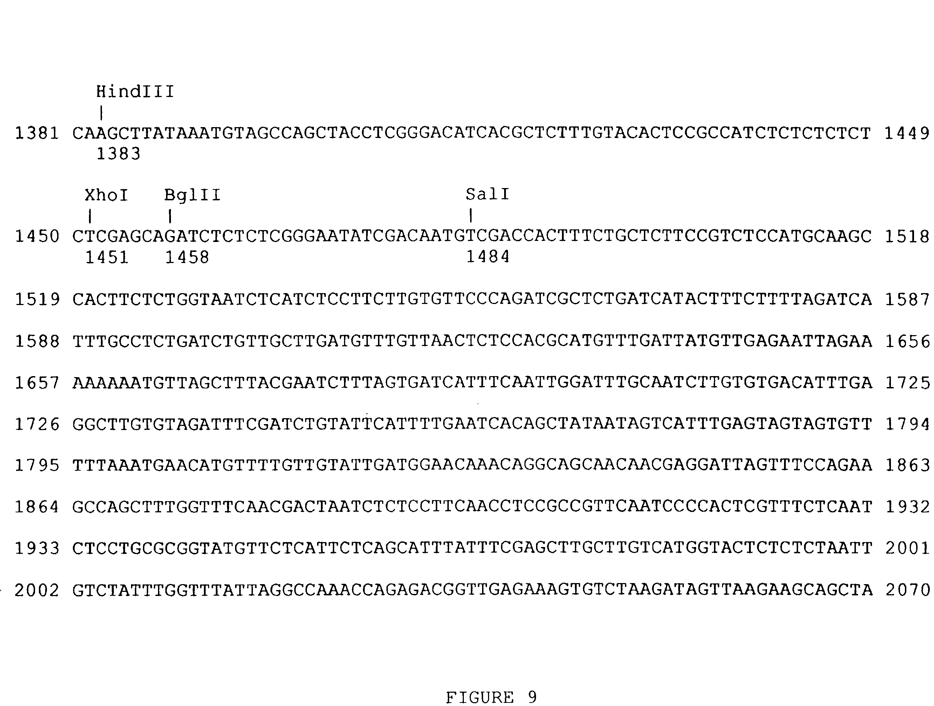

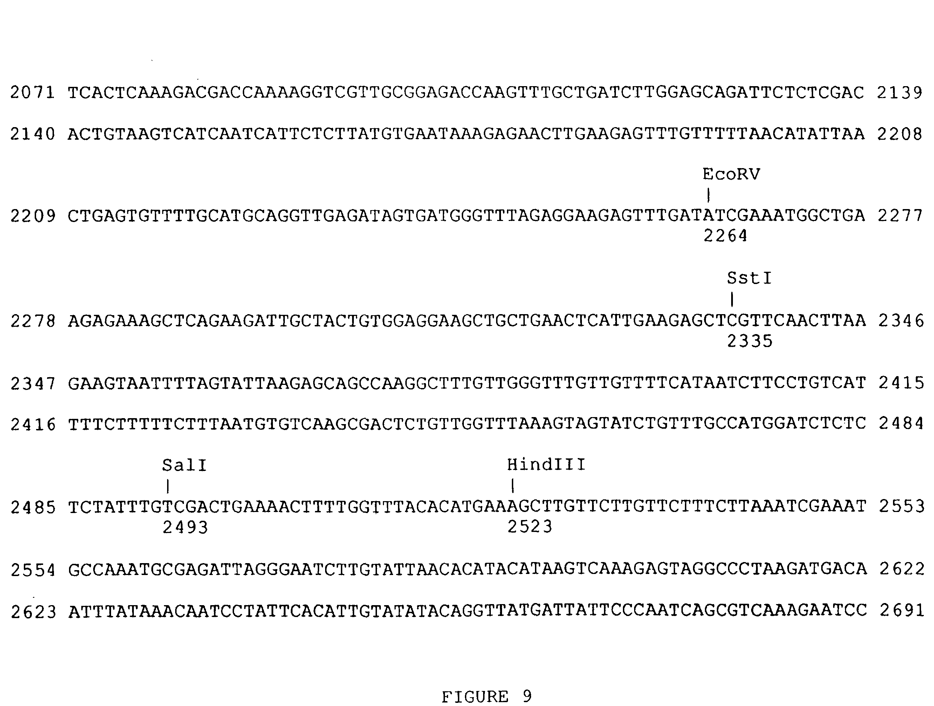

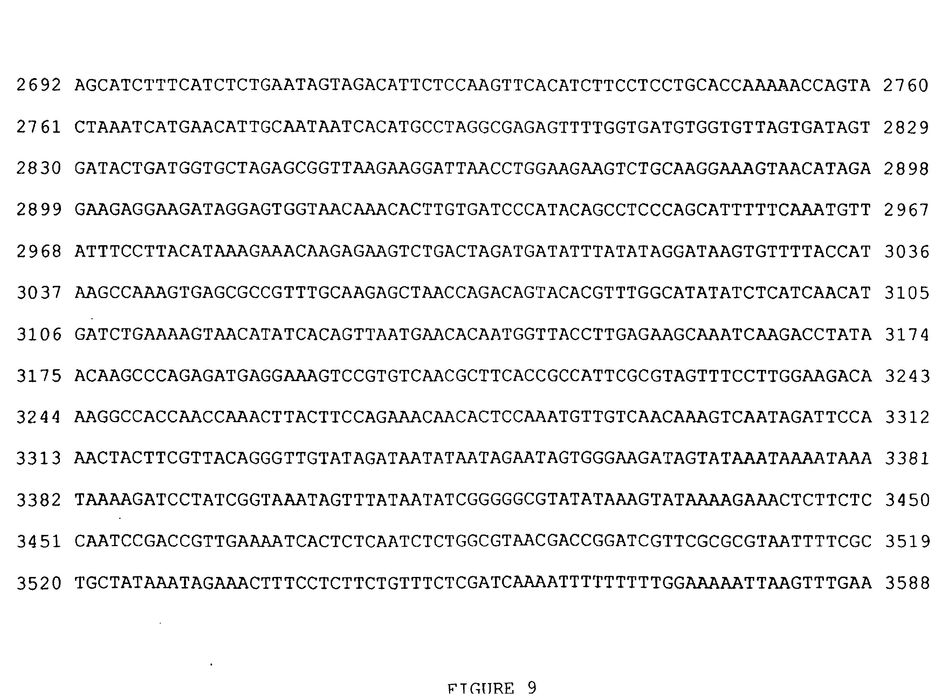

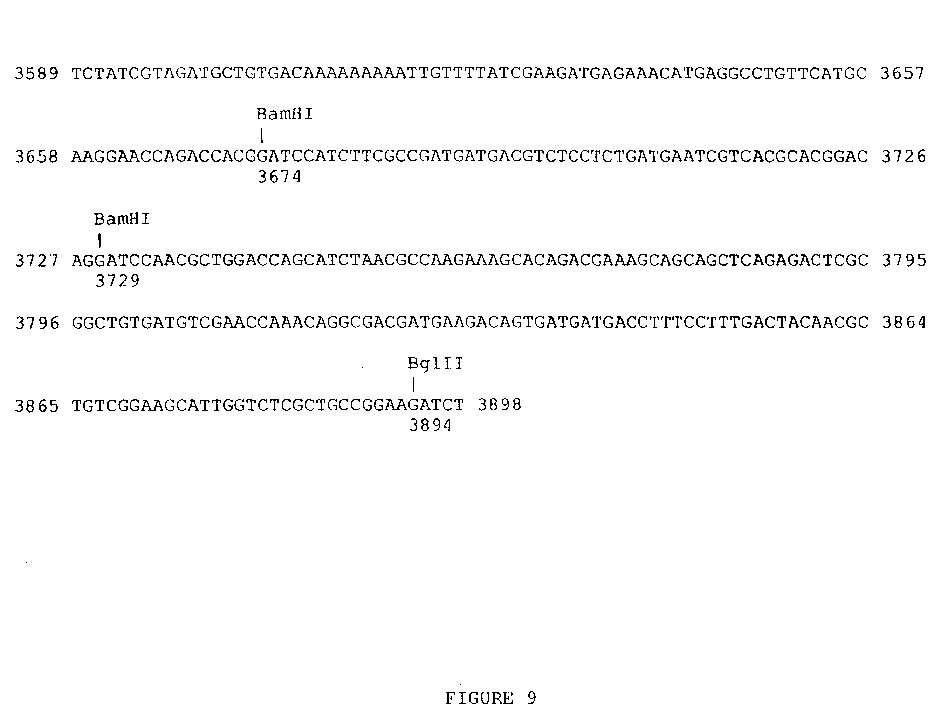

The Bcg 4-4 ACP message presents a similar expression profile to that of Bce4 and, therefore, also corresponds to lipid accumulation in the seed tissue. Bcg 4-4 is not found in the seed coat and may show some differences in expression level, as compared to Bce4, when the Bcg 4-4 5' non-coding sequence is used to regulate transcription or transcription and translation of a plant stearoyl-ACP desaturase of this invention. Genomic sequence of Bcg 4-4 is provided in Fig. 9 and as SEQ ID NO: 28, including about 1.5 kb 5' to the structural gene, about 1.2 kb of the Bcg 4-4 (ACP) structural gene sequence, and about 1.3 kb of the non-coding regulatory 3' sequence.

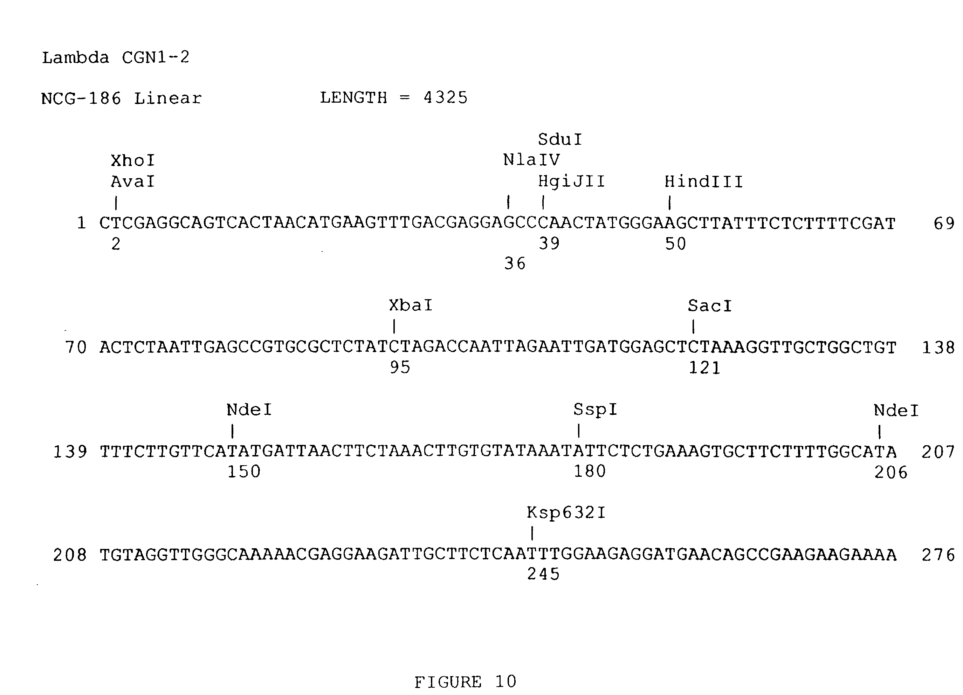

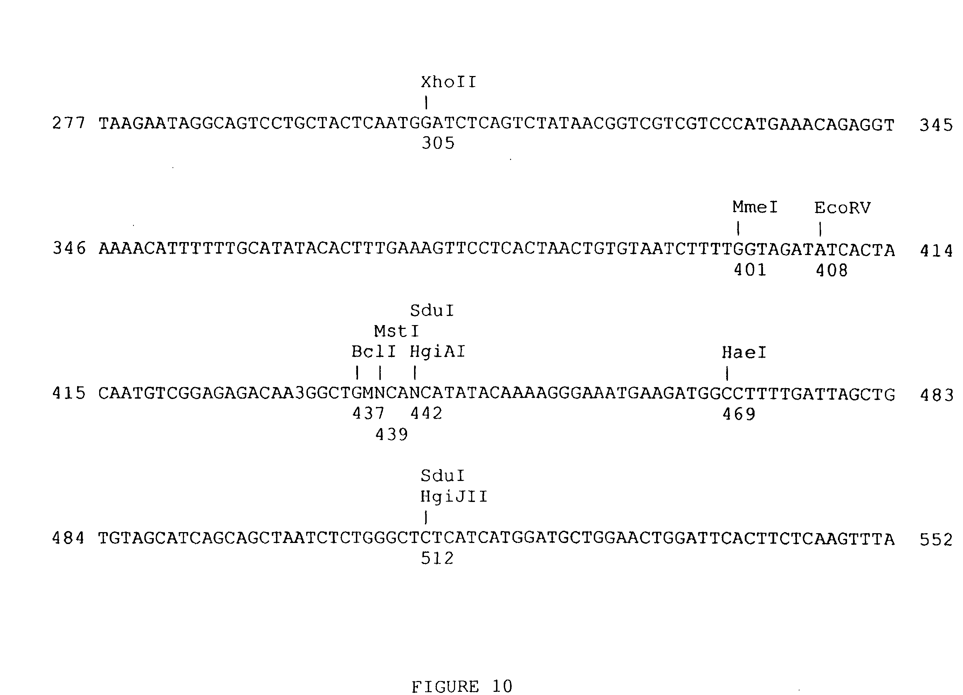

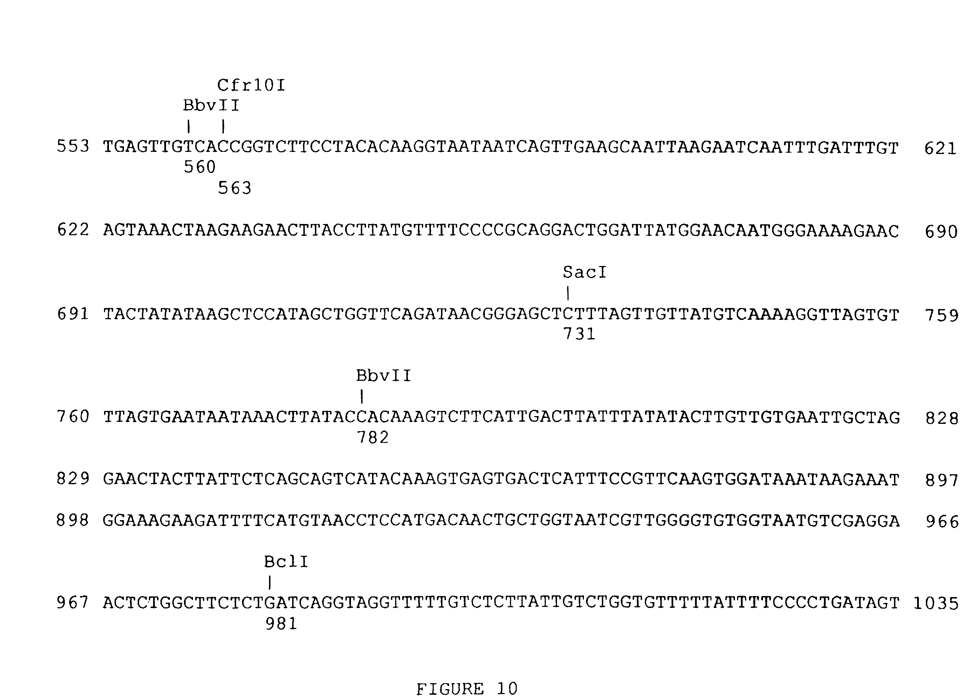

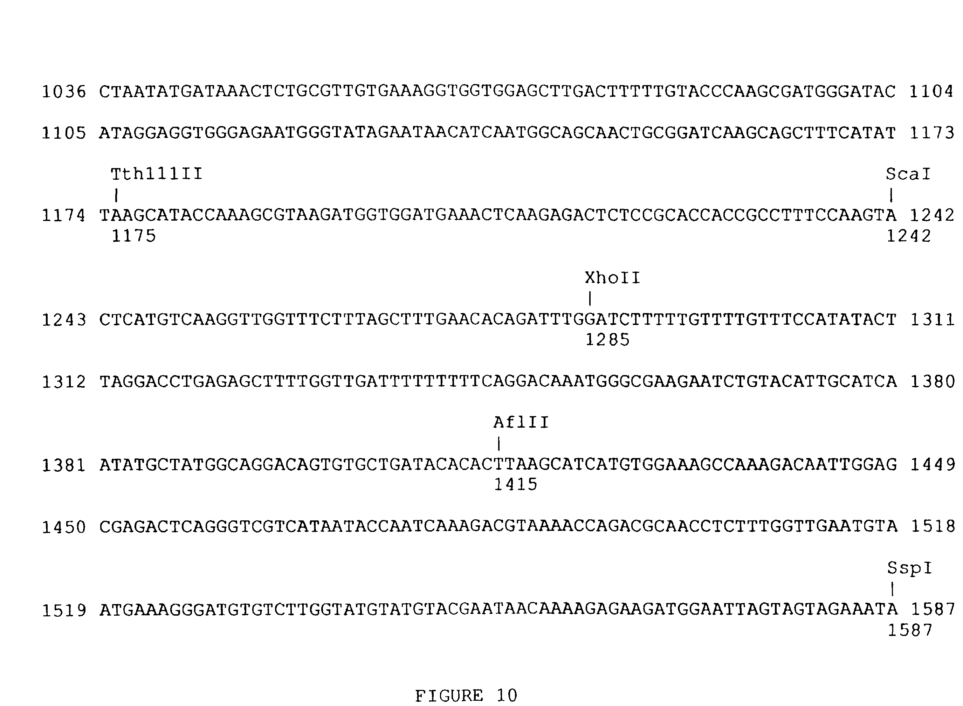

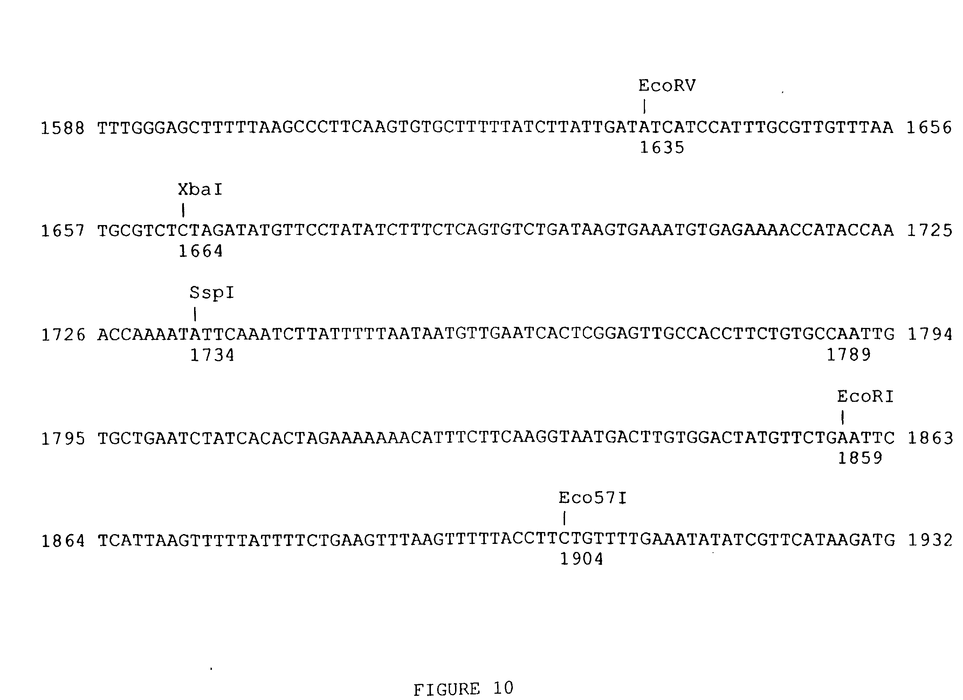

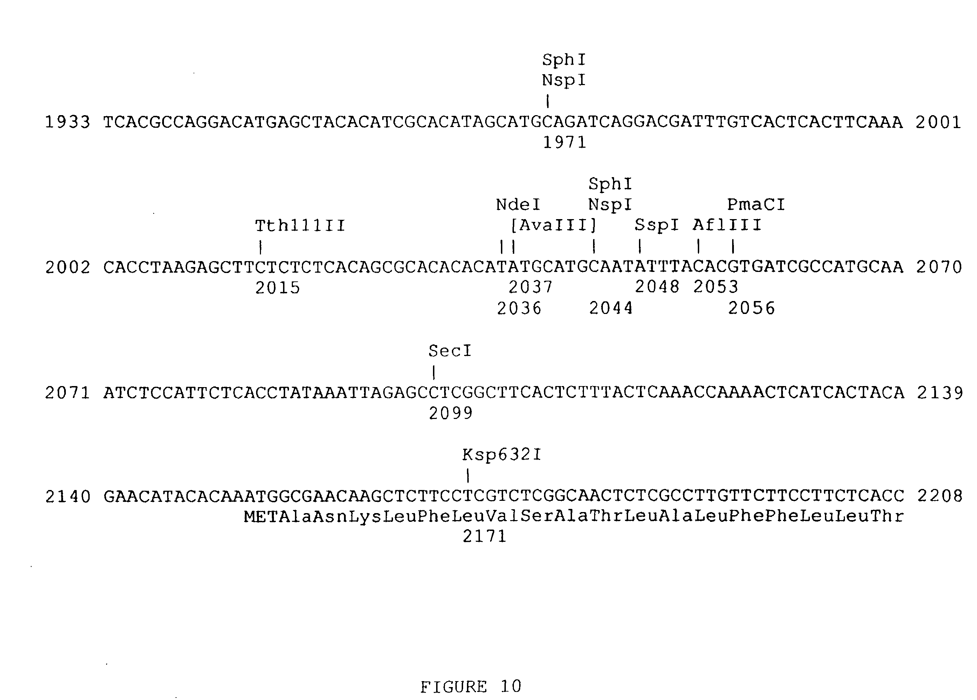

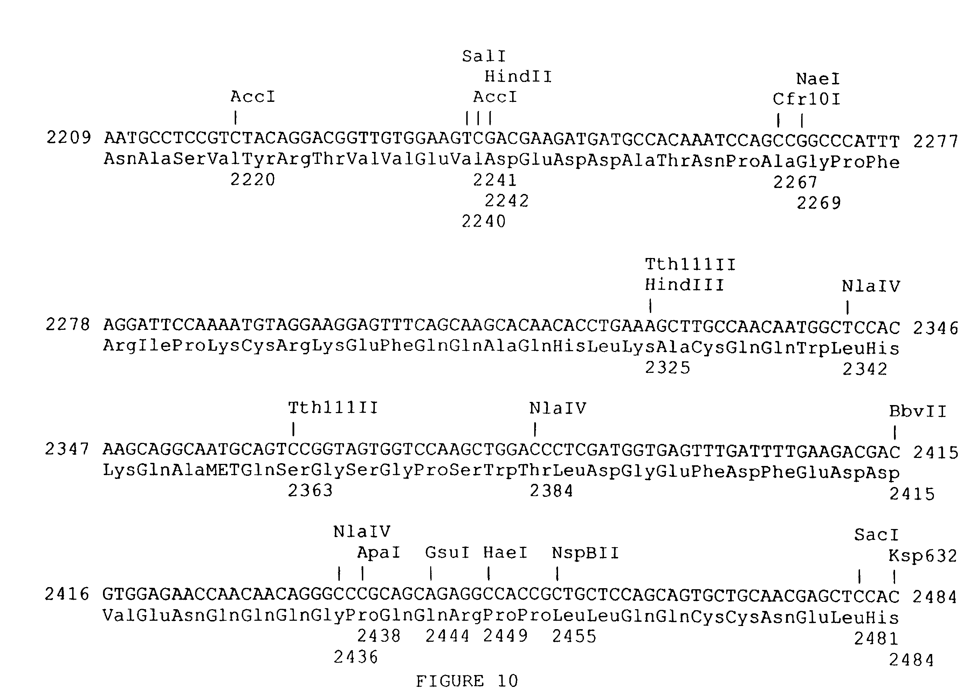

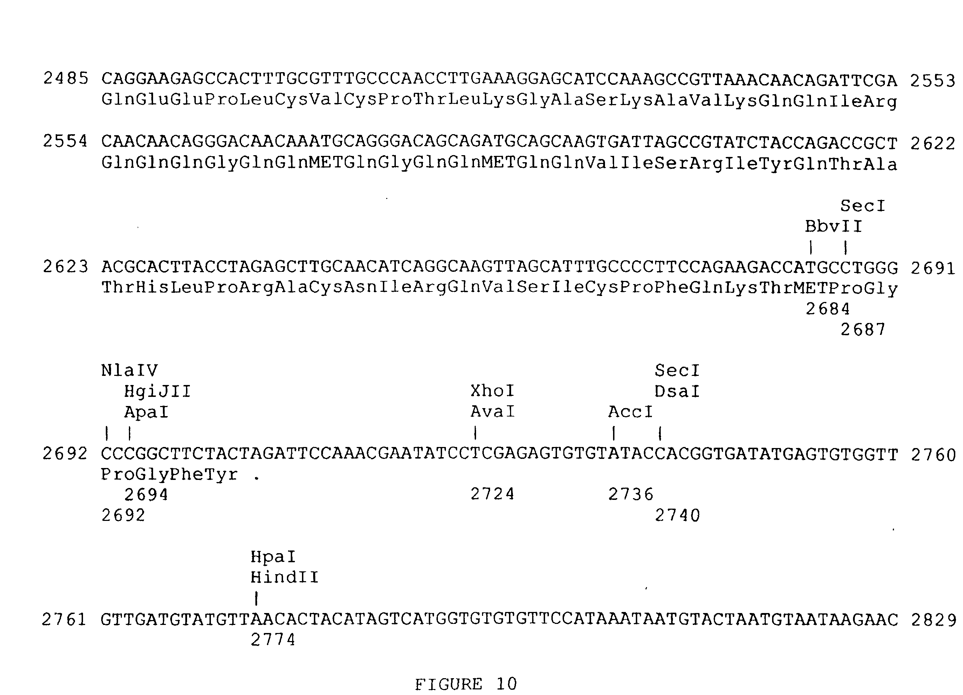

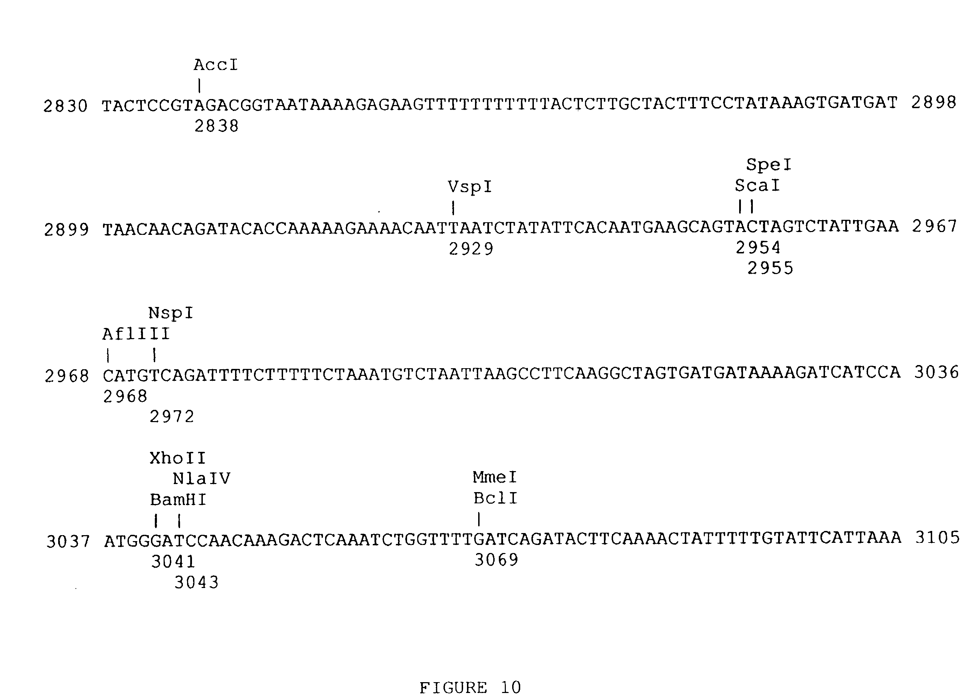

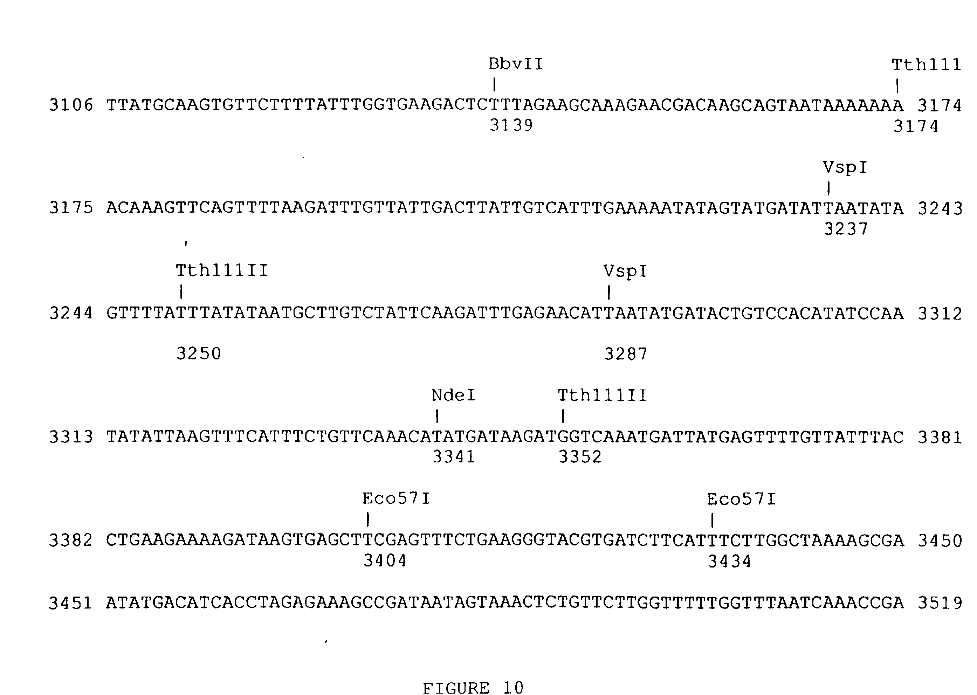

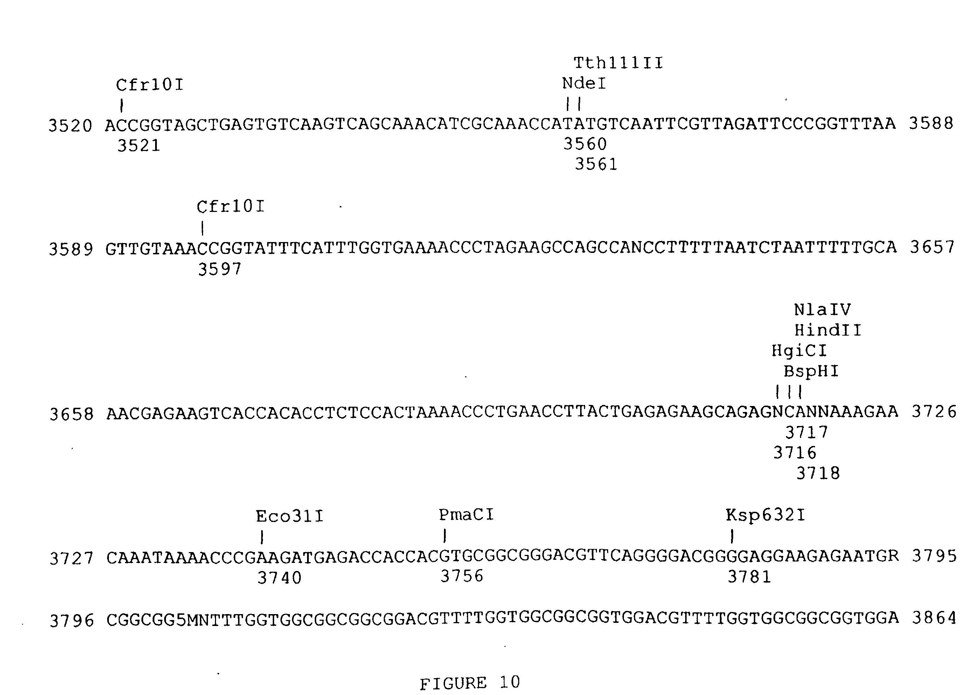

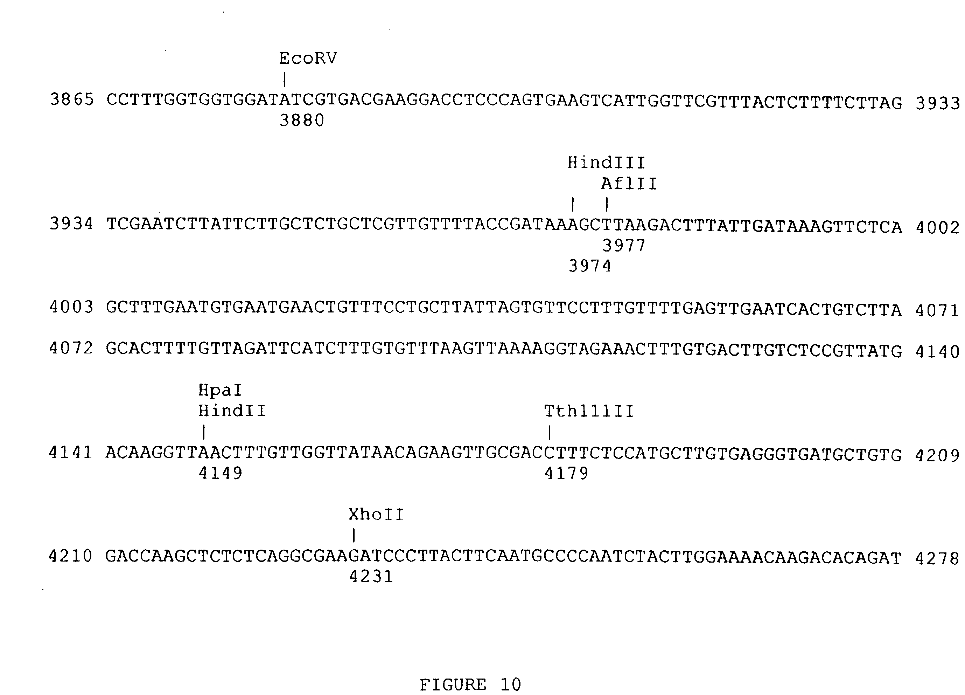

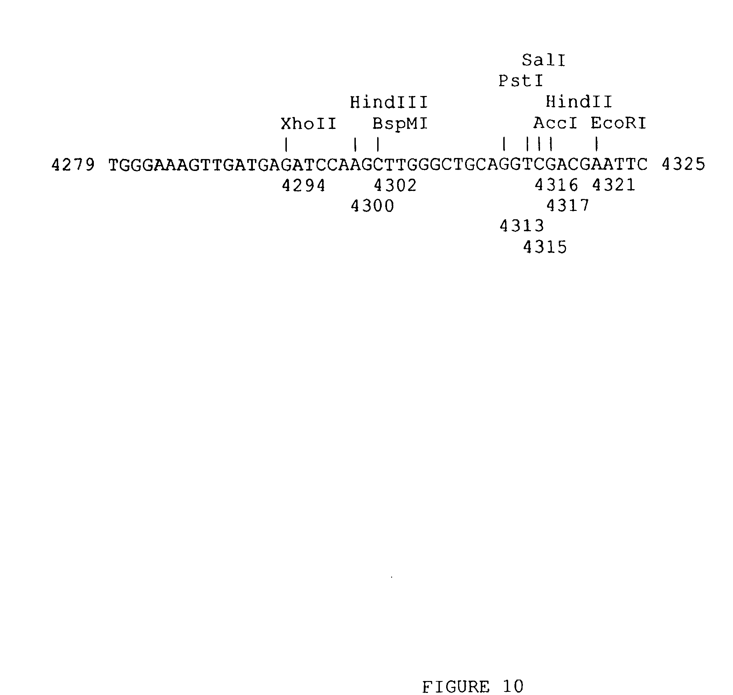

The napin 1-2 message is found in early seed development and thus, also offers regulatory regions which can offer preferential transcriptional regulation of a desired DNA sequence of interest such as the plant desaturase DNA sequence of this invention during lipid accumulation. Napins are one of the two classes of storage proteins synthesized in developing *Brassica* embryos (Bhatty, *et al., Can J. Biochem.* (1968) *46*:1191-1197) and have been used to direct tissue-specific expression when reintroduced into the *Brassica* genome (Radke, *et al., Theor. Appl. Genet.* (1988) *75*:685-694). Genomic sequence of napin 1-2 is provided in Fig. 10 and as SEQ ID NO: 29, including about 1.7 kb 5' to the structural gene and about 1.3 kb of the non-coding regulatory 3' sequence

Regulatory transcript termination regions may be provided in DNA constructs of this invention as well. Transcript termination regions may be provided by the DNA sequence encoding the plant desaturase or a convenient transcription termination region derived from a different gene source, especially the transcript termination region which is naturally associated with the transcript initiation region. The transcript termination region will contain at least about 1 kb, preferably about 3 kb of sequence 3' to the structural gene from which the termination region is derived.

In developing the DNA construct, the various components of the construct or fragments thereof will normally be inserted into a convenient cloning vector which is capable of replication in a bacterial host, e.g., *E. coli*. Numerous vectors exist that have been described in the literature. After each cloning, the plasmid may be isolated and subjected to further manipulation, such as restriction, insertion of new fragments, ligation, deletion, insertion, resection, etc., so as to tailor the components of the desired sequence. Once the construct has been completed, it may then be transferred to an appropriate vector for further manipulation in accordance with the manner of transformation of the host cell.

Normally, included with the DNA construct will be a structural gene having the necessary regulatory regions for expression in a host and providing for selection of transformant cells. The gene may provide for resistance to a cytotoxic agent, e.g. antibiotic, heavy metal, toxin, etc., complementation providing prototrophy to an auxotrophic host, viral immunity or the like. Depending upon the number of different host species into which the expression construct or components thereof are introduced, one or more markers may be employed, where different conditions for selection are used for the different hosts.

The manner in which the DNA construct is introduced into the plant host is not critical to this invention. Any method which provides for efficient transformation may be employed. Various methods for plant cell transformation include the use of Ti- or Ri-plasmids, microinjection, electroporation, liposome fusion, DNA bombardment or the like. In many instances, it will be desirable to have the construct bordered on one or both sides by T-DNA, particularly having the left and right borders, more particularly the right border. This is particularly useful when the construct uses *A. tumefaciens* or *A. rhizogenes* as a mode for transformation, although the T-DNA borders may find use with other modes of transformation.

Where *Agrobacterium* is used for plant cell transformation, a vector may be used which may be introduced into the *Agrobacterium* host for homologous recombination with T-DNA or the Ti- or Ri-plasmid present in the *Agrobacterium* host. The Ti- or Ri-plasmid containing the T-DNA for recombination may be armed (capable of causing gall formation) or disarmed (incapable of causing gall formation), the latter being permissible, so long as the vir genes are present in the transformed *Agrobacterium* host. The armed plasmid can give a mixture of normal plant cell and gall.

A preferred method for the use of *Agrobacterium* as the vehicle for transformation of plant cells employs a vector having a broad host range replication system, at least one T-DNA boundary and the DNA sequence or sequences of interest. Commonly used vectors include pRK2 or derivatives thereof. See, for example, Ditta *et al., PNAS USA*, (1980) *77*:7347-7351 and EPA 0 120 515, which are incorporated herein by reference. Normally, the vector will be free of genes coding for opines, oncogenes and *vir*-genes. Included with the expression construct and the T-DNA will be one or more markers, which allow for selection of transformed *Agrobacterium* and transformed plant cells. A number of markers have been developed for use with plant cells, such as resistance to chloramphenicol,. the aminoglycoside G418, hygromycin, or the like. The particular marker employed is not essential to this invention, one or another marker being preferred depending on the particular host and the manner of construction.

The expression constructs may be employed with a wide variety of plant life, particularly plant life involved in the production of vegetable oils. These plants include, but are not limited to rapeseed, sunflower, *C. tinctorius*, cotton, *Cuphea*, peanut, soybean, oil palm and corn. Anti-sense constructs may be employed in such plants which share complementarity between the endogenous sequence and the anti-sense desaturase. Of special interest is the use of an anti-sense construct having a *B. campestris* desaturase in rapeseed, including *B. campestris* and *B. napus.*

For transformation of plant cells using *Agrobacterium*, explants may be combined and incubated with the transformed *Agrobacterium* for sufficient time for transformation, the bacteria killed, and the plant cells cultured in an appropriate selective medium. Once callus forms, shoot formation can be encouraged by employing the appropriate plant hormones in accordance with known methods and the shoots transferred to rooting medium for regeneration of plants. The plants may then be grown to seed and the seed used to establish repetitive generations and for isolation of vegetable oils compositions. A variety of stable genetic lines having fixed levels of saturation may be obtained and integrated into a traditional breeding program. Hemizygous and heterozygous lines or homozygous lines may demonstrate different useful properties for oil production and/or breeding. For example, saturation levels may be increased up to 2-fold by the development of homozygous plants as compared with heterozygous (including hemizygous) plants.

The invention now being generally described, it will be more readily understood by reference to the following examples which are included for purposes of illustration only and are not intended to limit the present invention.

## EXAMPLES

### MATERIALS

Commercially available biological chemicals and chromatographic materials, including BSA, catalase (twice crystalized from bovine liver), spinach ferredoxin, ferredoxin-$NADP^+$ oxidoreductase (spinach leaf), NADPH, unlabeled fatty acids, DEAE-cellulose (Whatman DE-52) CNBr-activated Sepharose 4B, and octyl-Sepharose, and Reactive Blue Agarose are from Sigma (St. Louis, MO). Triethylamine, trichloroacetic acid, guanidine-HCl, and hydrazine-hydrate are also from Sigma. Proteolytic enzymes, including endoproteinases lysC, gluC, and aspN are sequencing grade enzymes obtained from Boehringer Mannheim (Indianapolis, IN). Organic solvents, including acetone, acetonitrile, methanol, ether and petroleum ether are purchased from J.T. Baker (Phillipsburg, NJ); concentrated acids and sodium sulfate are also from J.T. Baker (Phillipsburg, NJ). HPLC grade acetonitrile and trifluoracetic acid (TFA) are obtained from Burdick and Jackson (Muskegon, MI), and from Applied Biosystems (Foster City, CA), respectively. Radiochemicals, including [9,10(n)-$^3$H] oleic acid (10mCi/μmol) and [$^3$H]-iodoacetic acid (64Ci/mol) are from New England Nuclear (Boston, MA). Phenacyl-8 Reagent (bromoacetophenone with a crown ether catalyst) used to prepare phenacyl esters of the fatty acids for analysis are from Pierce (Rockford, IL). C18 reversed-phase thin-layer chromatography plates are from Whatman (Clifton, NJ).

Acyl carrier protein (ACP) and acyl-ACP synthase are isolated from *E. coli* strain K-12 as described by Rock and Cronan (Rock and Cronan, *Methods in Enzymol* (1981) *71*:341-351 and Rock *et al., Methods in Enzymol.* (1981) *72*:397-403). The *E. coli* is obtainable from Grain Processing (Iowa) as frozen late-logarithmic phase cells.

[9,10(n)-$^3$H, stearic acid is synthesized by reduction of [9,10(n)-$^3$H]oleic acid with hydrazine hydrate essentially as described by Johnson and Gurr (*Lipids* (1971) *6*:78-84). [9,10(n)-$^3$H]oleic acid (2 mCi) supplemented with 5.58mg unlabeled oleic acid to give a final specific radioactivity of 100mCi/mmol, is dissolved in 2ml of acetonitrile, acidified with 40μl of glacial acetic acid, and heated to 55°C. Reduction is initiated with 100μl of 60% (w/w) hydrazine hydrate; oxygen is bubbled through the mixture continuously. After each hour acetonitrile is added to bring the volume back to 2ml and an additional 100μl of hydrazine hydrate is added. At the end of 5 hr. the reaction is stopped by addition of 3ml of 2M HCl. The reaction products are extracted with three 3ml aliquots of petroleum ether and the combined ether extracts are washed with water, dried over sodium sulfate and evaporated to dryness. The dried reaction products are redissolved in 1.0ml acetonitrile and stored at -20°C. The distribution of fatty acid products in a 15μl aliquot is determined by preparation of phenacyl esters, which are then analyzed by thin layer chromatography on C-18 reverse phase plates developed with methanol:water:95:5 (v/v). Usually reduction to [9,10(n)-$^3$H]stearic acid is greater than 90%, a small amount of unreacted oleic acid may remain. The analysis is used to establish fraction of the total radioactivity that is present as stearate, and thereby to determine the exact substrate concentration in the enzyme assay.

Acyl-ACP substrates, including [9,10(n)-$^3$H] stearoyl-ACP are prepared and purified by the enzymatic synthesis procedure of Rock, Garwin, and Cronan (*Methods in Enzymol.* (1981) *72*:397-403).

Acyl carrier protein was covalently bound to Sepharose 4B by reaction of highly purified ACP with CNBr-activated Sepharose 4B as described by McKeon and Stumpf (*J. Biol. Chem.* (1982) *257*:12141-12147).

## Example 1

In this example, an initial purification of *C. tinctorius* (safflower) desaturase, following the method of McKeon and Stumpf (*J. Biol. Chem.* (1982) *257*:12141-12142), is described.

*Assay:* In each of the following steps, the presence of the enzyme is detected radiometrically by measuring enzyme-catalyzed release of tritium from [9,10(n)-$^3$H]stearoyl-ACP. Preparation of this substrate is described in "Materials" above.

The assay is performed by mixing 150μl water, 5ml dithiothreitol (100mM, freshly prepared in water), 10μl bovine serum albumin (10mg/ml in water), 15μl NADPH (25mM, freshly prepared in 0.1M Tricine-HCl, pH 8.2), 25μl spinach ferredoxin (2mg/ml Sigma Type III in water) , 3μl NADPH:ferredoxin oxidoreductase (2.5 units/ml from Sigma), and 1 μl bovine liver catalase (800,000 units/ml from Sigma); after 10 min at room temperature, this mixture is added to a 13x100 mm screw-cap test tube containing 250μl sodium 1,4-piperazinediethanesulfonate (0.1M, pH 6.0). Finally, 10μl of the sample to be assayed is added and the reaction is started by adding 30μl of the substrate, [9,10 (n)-$^3$H] stearoyl-ACP (100μCi/μmol, 10μM in 0.1M sodium 1,4-piperazinediethanesulfonate, pH 5.8). After sealing with a cap, the reaction is allowed to proceed for 10 min. while shaking at 23°C. The reaction is terminated by addition of 1.2ml of 5.8% tricholoracetic acid and the resulting precipitated acyl-ACP's are removed by centrifugation. The tritium released into the aqueous supernatant by the desaturase reaction is measured by liquid scintillation spectrometry. One unit of activity is defined as the amount of enzyme required to convert 1μmol of stearoyl-ACP to oleoyl-ACP, or to release 4μg-atoms of $^3$H per minute.

*Source tissue:* Developing *C. tinctorius* seeds from greenhouse grown plants are harvested between 16 and 18 days after flowering, frozen in liquid nitrogen and stored at -70°C until extracted.

*Acetone Powder:* Approximately 50g of frozen seeds are ground in liquid nitrogen and sieved to remove large seed coat pieces to provide a fine embryo powder. The powder is washed with acetone on a Buchner funnel until all yellow color is absent from the filtrate. The powder is then air dried and further processed as described below, or may be stored frozen for at least a year at -70°C without loss of enzyme activity.

*Acetone Powder Extract:* The dried acetone powder is weighed and triturated with ten times its weight of 20mM potassium phosphate, pH 6.8; the mixture is then centrifuged at 12,000 x g for 20 minutes and decanted through a layer of Miracloth (Calbiochem, La Jolla, CA).

*Ion Exchange Chromatography:* The acetone powder extract is then applied to a DEAE-cellulose column (Whatman DE-52) (1.5 x 12 cm) equilibrated with 20mM potassium phosphate, pH 6.8. The pass-through and a wash with one column-volume (20ml) of buffer are pooled.

*Affinity Chromatography:* An affinity matrix for purification of the desaturase is prepared by reacting highly purified *E. coli* ACP, with CNBr-activated Sepharose 4B (Sigma). ACP (120mg) is reduced by treatment with 1mM dithiothreitol for 30 min on ice, and then desalted on Sephadex G-10 (Pharmacia) equilibrated with 0.1M sodium bicarbonate, pH 6.0. The treated ACP (20 ml, 6 mg/ml) is then mixed with 20ml of CNBr-activated Sepharose 4B swollen in 0.1M sodium bicarbonate, pH 7.0, and the mixture is allowed to stand at 4°C for one day. The gel suspension is then centrifuged, washed once with 0.1M sodium bicarbonate, pH 7.0, and then treated with 40ml 0.1M glycine, pH 8.0, for 4 hours at room temperature to block unreacted sites. The gel is then washed for five cycles with alternating 50ml volumes of 0.5M NaCl in 0.1M sodium acetate, pH 4.0, and 0.5M NaCl in 0.1M sodium bicarbonate, pH 6.5, to remove non-covalently bound ligand. The gel is loaded into a column (1.5 x 11.2 cm) and equilibrated in 20mM potassium phosphate, pH 6.8.

The combined fractions from the DE-52 column are applied to the column, which is subsequently washed with one column volume (20ml) of the equilibration buffer, and then with 2.5 column volumes (50ml) of 300mM potassium phosphate, pH 6.8. Fractions are assayed for protein using the BCA Protein Assay Reagent (Pierce, Rockford, IL) to make sure that all extraneous protein has been eluted. Active Δ-9 desaturase is eluted from the column with 600mM potassium phosphate, pH 6.8. Active fractions are analyzed by polyacrylamide gel electrophoresis in sodium dodecyl sulfate (SDS-PAGE) on 0.75mm thick 8 x 12 cm mini-gels according to the method of Laemmli (*Nature* (1970) *227*: 680). The running gel contains 10% acrylamide in a 30/0.8 ratio of acrylamide to cross-linker bis-acrylamide. Those fractions containing a predominant band at approximately 43 kD are pooled and stored frozen at -70°C until final purification. The yield from 50g of seed tissue is is approximately 60μg of protein as measured by amino acid analysis.

Further purification as described in Example 2 or Example 3 is then applied to the fractions pooled from the ACP-Sepharose column separation.

**Example 2**

In this example, a protocol for the final purification of *C. tinctorius* desaturase is described. Seeds are treated in accordance with Example 1.

*Reverse-Phase HPLC:* Fractions from the ACP-Sepharose column are pooled and applied to a Vydac C4 reverse-phase column (0.45 x 15 cm) equilibrated in 0.1% TFA, 7% acetonitrile. After a 10 min wash with 0.1% TFA, the column is eluted with a gradient of increasing acetonitrile (7%-70% v/v) in 0.1% TFA over a period of 45 min. The flow rate is 0.5ml/min throughout. Eluting components are monitored by absorbance at 214 nm. Δ-9 desaturase elutes at about 42 min. (approximately 50% acetonitrile); the major contaminant protein remaining from ACP-affinity chromatography elutes at about 28 min. (approximately 30% acetonitrile). The substantially homogeneous desaturase, which is no longer active, is identified by SDS-PAGE, in which it exhibits a single band corresponding to a molecular weight of approximately 43 kD. The quantity of desaturase protein in the sample may be determined by amino acid analysis.

**Example 3**

In this example, a protocol for the final purification of *C. tinctorius* desaturase is described. Seeds are treated in accordance with Example 1.

*Reduction and Alkylation:* Protein is precipitated out of the pooled fraction solutions recovered from the ACP-Sepharose column with 10% (w/v) trichloroacetic acid, washed with cold (-20°C) acetone, and resuspended in 1 ml 500mM Tris-HCl, pH 8.6, containing 6M guanidine-HCl, 10mM EDTA, and 3.2 mM dithiothreitol. After 2 hours, 3.52 μmol [$^3$H]-iodoacetic acid (64μCi/μmol, New England Nuclear) is added, and the reaction is allowed to proceed at room temperature in the dark for 2 hours, at which time the reaction is terminated by addition of 1μl (15μmol) β-mercaptoethanol. The sample is then re-precipitated with 10% (w/v) trichloroacetic acid, and the pellet again washed with cold (-20°C) acetone and resuspended in Laemmli's SDS-sample buffer (*Nature* (1970) *227*:680).

*SDS-Polyacrylamide Gel Electrophoresis:* The resulting sample is boiled for 5 min. and then applied to a 1.5 mm thick, 8 x 12 cm, SDS-polyacrylamide mini-gel prepared as described by Laemmli, *supra.* The running gel contains 17.5% acrylamide in a 30:0.13 ratio of acrylamide to cross-linking bis-acrylamide. Separation is achieved by electrophoresis at 15 mA, for 2 hours at 4°C.

*Blotting from SDS-gels to PVDF Membrane:* Proteins are recovered from the gel by electroblotting at 5 mA/cm$^2$ to a four-layer sandwich of polyvinylidenedifluoride (PVDF) membrane for 2 h at 4°C in a buffer containing 10mM CAPS ("3-[cyclohexylamino]-1-propane-sulfonic acid"), pH 11. The membranes must be wetted in 50% methanol, prior to exposure to the blotting buffer. After blotting, the membrane layers are stained for 1-2 min. in 0.02% Coomassie Blue in 50% methanol, and then destained in 50% methanol. The desaturase is identified as a band corresponding to a molecular weight of about 43 kD; the major contaminant runs at or near the dye front of the gel corresponding to a molecular weight less than 20 kD.

The desaturase band on the PVDF membrane may be applied directly to the Edman sequencer (Applied Biosystems Model 477A) for determination of the N-terminal sequence of the intact protein, or for more extensive sequence determination, may be eluted from the membrane in 40% acetonitrile to recover pure desaturase in solution. Acetonitrile is removed from the eluted desaturase by evaporation on a Speed-Vac (Savant; Farmingdale, NY), and the substantially homogeneous Δ-9 desaturase is resuspended in an appropriate buffer for subsequent proteolytic digestion as described in Example 4. The quantity of desaturase protein present may be determined by amino acid analysis.

Alternatively, if the sample is to be digested with trypsin or gluC protease to generate peptides for amino acid sequence analysis, proteins may be electroblotted to nitrocellulose membranes and stained with Ponceau or amido black.

**Example 4**

In this example, a method for the determination of the amino acid sequence of a desaturase is described.

*Reduction and Alkylation:* Substantially homogenous stearoyl-ACP desaturase (See, Example 2) is reduced and alkylated with [$^3$H]-iodacetic acid (See, Example 3), except that the final acetone-washed pellet is resuspended in the appropriate buffer for subsequent proteolysis. Reduction and alkylation assures complete denaturation of the protein so that complete proteolysis can occur. The sample may be alkylated with radiolabeled iodoacetamide or with 4-vinylpyridine instead of [$^3$H]-iodacetic acid in substantially the same manner. Use of iodoacetic acid affords an alkylated sample with greater solubility, which is advantageous in subsequent sample manipulation.

*Proteolysis:* Substantially pure alkylated samples are digested with endoproteinase lysC. The sample is resuspended in 100 μl of 25 mM Tris-HCl, pH 8.8, containing 1 mM EDTA. Endoproteinase lysC is added to the sample in a protease/desaturase ratio of 1/50 (w/w). Digestion is allowed to proceed at room temperature for 8 hours, at which time another equal amount of protease is added. After 18 more hours, 1 µl of concentrated HCl is added to stop proteolysis, and the sample is applied directly to a Vydac C18 reverse-phase column (0.2 x 15 cm) equilibrated in 7% acetonitrile (v/v) in 0.1 mM sodium phosphate, pH 2.2. After washing for 20 min with the equilibration buffer, peptides are eluted with a gradient in acetonitrile (7-70%, v/v) over 120 min. Flow rate is 50 µl/min, throughout. Eluting components are monitored by absorbance at 214 nm, and individual peptide peaks are collected as separate fractions. The peptide fractions are further purified by application to a second Vydac C18 reverse-phase column (0.2 x 15 cm) equilibrated in 7% (v/v) acetonitrile in 0.1% (v/v) trifluoroacetic acid. Again, after a 20 min wash with equilibration buffer, the substantially pure peptides are eluted with a gradient (7-70%, v/v) of acetonitrile in 0.1% trifluoroacetic acid over 120 min. The flow rate is 50 µl/min, throughout. Eluting components are monitored by absorbance at 214 nm, and individual peptide peaks are collected as separate fractions. These substantially pure peptides are applied directly to the Edman sequencer (Applied Biosystems, Model 477A) for amino acid sequence analysis. Alternatively, peptide fraction from the first HPLC purification in phosphate buffer, or from a single chromatography step in trifluoroacetic acid buffer, may be applied directly to the sequencer, but these fractions, in many cases, are not substantially pure and yield mixed or ambiguous sequence information.

Other proteases may be used to digest desaturase, including but not limited to trypsin, gluC, and aspN. While the individual digest buffer conditions may be different, the protocols for digestion, peptide separation, purification, and sequencing are substantially the same as those outlined for the digestion with lysC. Alternatively, desaturase may be digested chemically using cyanogen bromide (Gross *Methods Enzymol* (1967) *11*:238-255 or Gross and Witkop *J. Am. Chem. Soc.* (1961) *83*:1510), hydroxylamine (Bornstein and Balian *Methods Enzymol.* (1977) *47*:132-745), iodosobenzoic acid (Inglis *Methods Enzymol.* (1983) *91*:324-332), or mild acid (Fontana *et al., Methods Enzymol.* (1983) *91*: 311-317), as described in the respective references.

Fragments generated from these digestion steps of *C. tinctorius* desaturase are presented in Fig. 1 and as SEQ ID NOS: 1-11.

## Example 5

In this example, the preparation of a plant embryo cDNA bank, using the methods as described in Alexander, *et al.* (*Methods in Enzymology* (1987) *154*:41-64) and the screening of the bank to obtain a desaturase cDNA clone is described.

*C. tinctorius*: A plant embryo cDNA library may be constructed from poly(A)+ RNA isolated from *C. tinctorius* embryos collected at 14-17 days post-anthesis. Poly(A)+ RNA is isolated from polyribosomes by a method initially described by Jackson and Larkins (*Plant Physiol.* (1976) *57*:5-10) as modified by Goldberg *et al.* (*Developmental Biol.* (1981) *83*:201-217).

The plasmid cloning vector pCGN1703, derived from the commercial cloning vector Bluescribe M13- (Stratagene Cloning Systems; San Diego, CA), is made as follows. The polylinker of Bluescribe M13- is altered by digestion with *Bam*HI, treatment with mung bean endonuclease, and blunt-end ligation to create a *Bam*HI-deleted plasmid, pCGN1700. pCGN1700 is digested with *Eco*RI and *Sst*I (adjacent restriction sites) and annealed with synthetic complementary oligonucleotides having the sequences 5' CGGATCCACTGCAGTCTAGAGGGCCCGGGA 3'(SEQ ID NO: 30) and 5' AATTTCCCGGGCCCTCTAGACTGCAGTGGATCCGAGCT 3' (SEQ ID NO: 31). These sequences are inserted to eliminate the *Eco*RI site, move the *Bam*HI site onto the opposite side of the *Sst*I (also, sometimes referred to as "*Sac*I" herein) site found in Bluescribe, and to include new restriction sites *Pst*I, *Xba*I, *Apa*I, *Sma*I. The resulting plasmid pCGN1702, is digested with *Hin*dIII and blunt-ended with Klenow enzyme; the linear DNA is partially digested with *Pvu*II and ligated with T4 DNA ligase in dilute solution. A transformant having the *lac* promoter region deleted is selected (pCGN1703) and is used as the plasmid cloning vector.

Briefly, the cloning method for cDNA synthesis is as follows. The plasmid cloning vector is digested with *Sst*I and homopolymer T-tails are generated on the resulting 3'-overhang sticky-ends using terminal deoxynucleotidyl transferase. The tailed plasmid is separated from undigested or un-tailed plasmid by oligo(dA)-cellulose chromatography. The resultant vector serves as the primer for synthesis of cDNA first strands covalently attached to either end of the vector plasmid. The cDNA-mRNA-vector complexes are treated with terminal transferase in the presence of deoxyguanosine triphosphate, generating G-tails at the ends of the cDNA strands. The extra cDNA-mRNA complex, adjacent to the *Bam*HI site, is removed by *Bam*HI digestion, leaving a cDNA-mRNA-vector complex with a *Bam*HI sticky-end at one end and a G-tail at the other. This complex is cyclized using the annealed synthetic cyclizing linker, 5'-GATCCGCGGCCGCGAATTCGAGCTCCCCCCCCCC-3' and 3'-GCGCCGGCGCTTAAGCTCGA-5'
which has a *Bam*HI sticky-end and a C-tail end. Following ligation and repair the circular complexes are transformed into *E. coli* strain DH5α (BRL; Gaithersburg, MD) to generate the cDNA library. The *C. tinctorius* embryo cDNA bank contains between $3x10^6$ and $5x10^6$ clones with an average cDNA insert size of approximately 1000 base pairs.

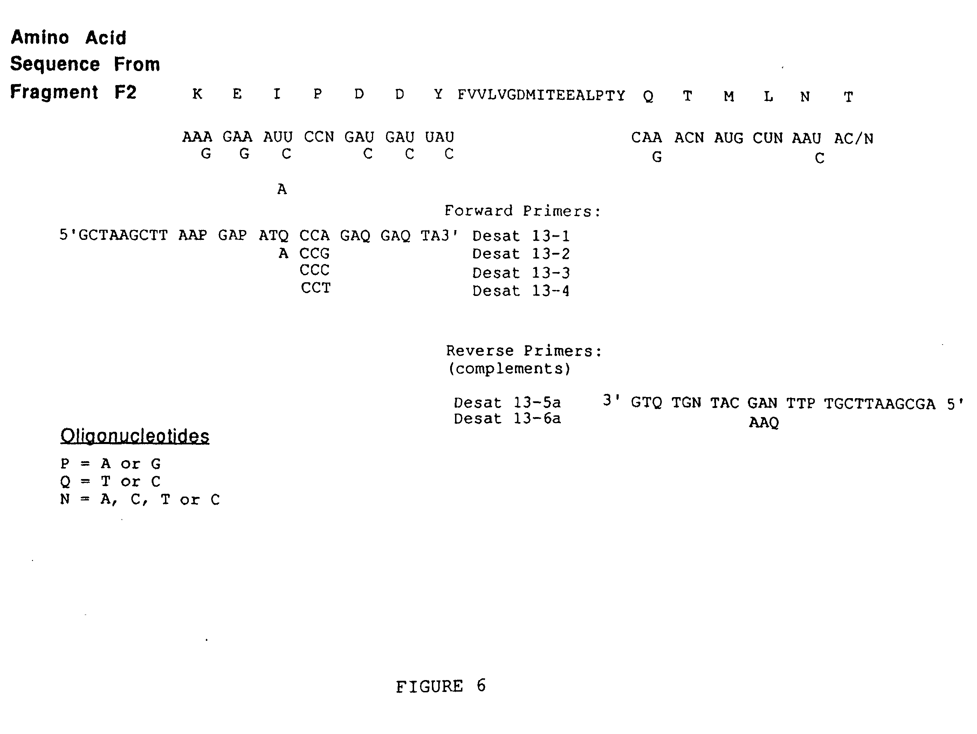

*Probe production Including PCR Reactions*: Two regions of amino acid sequence (Example 4) with low codon degeneracy are chosen from opposite ends of peptide sequence "Fragment F2" (SEQ ID NO:2) for production of a probe for the plant desaturase cDNA. Two sets of mixed oligonucleotides are designed and synthesized for use as forward (SEQ ID NOS: 21-24) and reverse (SEQ ID NOS: 25-26) primers, respectively, in the polymerase chain reaction (Saiki *et al., Science* (1985) *230*:1350-1354; Oste, *Biotechniques* (1988) *6*:162-167). See, Fig. 6. All oligonucleotides are synthesized on an Applied Biosystems 380A DNA synthesizer.

Probes to *C. tinctorius* desaturase may be prepared using the peptide sequence "Fragment 2" (SEQ ID NO: 2) identified in Fig. 1. Four types of forward primers were synthesized and labeled 13-1, 13-2, 13-3, and 13-4 (SEQ ID NOS: 21-24, respectively). Two groups of reverse primers were synthesized and designated 13-5A and 13-6A (SEQ ID NOS: 25-26, respectively). The primer sequences are shown in Fig. 6. These oligonucleotide groups have a redundancy of 64 or less and contain either 20 or 17 bases of coding sequence along with flanking restriction site sequences for *Hin*dIII or *Eco*RI. Based on the intervening amino acid sequence between the primer regions on peptide "Fragment 2" (SEQ ID NO: 2) the PCR product is expected to contain 107 base pairs.

Polymerase chain reaction is performed using the cDNA library DNA as template and the possible eight combinations of the four forward and two reverse oligonucleotides as primers in a Perkin-Elmer/Cetus DNA Thermal Cycler (Norwalk, CT) thermocycle file 1 min. 94°C, 2 min. 42°C, 2 min rise from 42°-72°C for 30 cycles, followed by the step cycle file without step rises, 1 min. 94°C, 2 min. 42°C, 3 min. 72°C with increasing 15 sec extensions of the 72°C step for 10 cycles, and a final 10 min. 72°C extension.

The product of the 13-4 forward primer (SEQ ID NO: 24) and the 13-5A reverse primer (SEQ ID NO: 25) reaction was ethanol precipitated and then digested with *Hin*dIII and *Eco*RI, the resulting fragment was subcloned into pUC8 (Vieira and Messing, *Gene* (1982) *19*:259-268). Minipreparation DNA (Maniatis *et al., Molecular_Cloning: A Laboratory Manual* (1982) Cold Harbor Laboratory, New York) of one clone was sequenced by Sanger dideoxy sequencing (Sanger *et al., Proc. Nat. Acad. Sci. USA* (1977) *74*:5463-5467) using the M13 universal and reverse primers. Translation of the resulting DNA sequence results in a peptide sequence that exactly matches the amino acid sequence in peptide "Fragment F2" (SEQ ID NO: 2).

An exact 50 base oligonucleotide designated DESAT-50 is synthesized to match the sequence of the PCR reaction product from the first valine residue to the last tyrosine residue.

The probe DSAT-50 5'-GTAAGTAGGTAGGGCTTCCTCTGTAATCATATCTCCAACCAAAACAACAA-3' (SEQ ID NO: 32) is used to probe the *C. tinctorius* embryo cDNA library.

*Library screen*

The *C. tinctorius* embryo cDNA bank is moved into the cloning vector lambda gt10 (Stratagene Cloning Systems) by digestion of total cDNA with *Eco*RI and ligation to lambda gt10 DNA digested with *Eco*RI. The titer of the resulting library was ~$5x10^5$/ml. The library is then plated on *E. coli* strain C600 (Huynh, *et al., DNA Cloning Vol. 1* Eds. Glover D.M. IRL Press Limited: Oxford England, pp. 56, 110) at a density of 5000 plaques/150 mm NZY ("NZYM" as defined in Maniatis *et al. supra*) agar plate to provide over 45,000 plaques for screening. Duplicate lifts are taken of the plaques using NEN Colony Plaque Screen filters by laying precut filters over the plates for ~1 minute and then peeling them off. The phage DNA is immobilized by floating the filters on denaturing solution (1.5M NaCl, .05M NaOH) for 1 min., transferring the filters to neutralizing solution (1.5M NaCl, 0.5M Tris-HCl pH 8.0) for 2 min. and then to 2XSSC (1xSSC = 0.15M NaCl; 0.015M Na citrate) for 3 min., followed by air drying. The filters are hybridized with $^{32}P$ end-labeled DSAT-50 oligonucleotide (SEQ ID NO: 32) (BRL 5' DNA Terminus Labeling System) by the method of Devlin et al., (*DNA* (1988) *7*:499-807) at 42° C overnight, and washed for 30 min. at 50°C in 2XSSC, 0.5% SDS and then twice for 20 min. each at 50°C in 0.1XSSC, 0.5% SDS. Filters are exposed to X-ray film at -70°C with a Dupont Cronex intensifying screen for 48 hours.

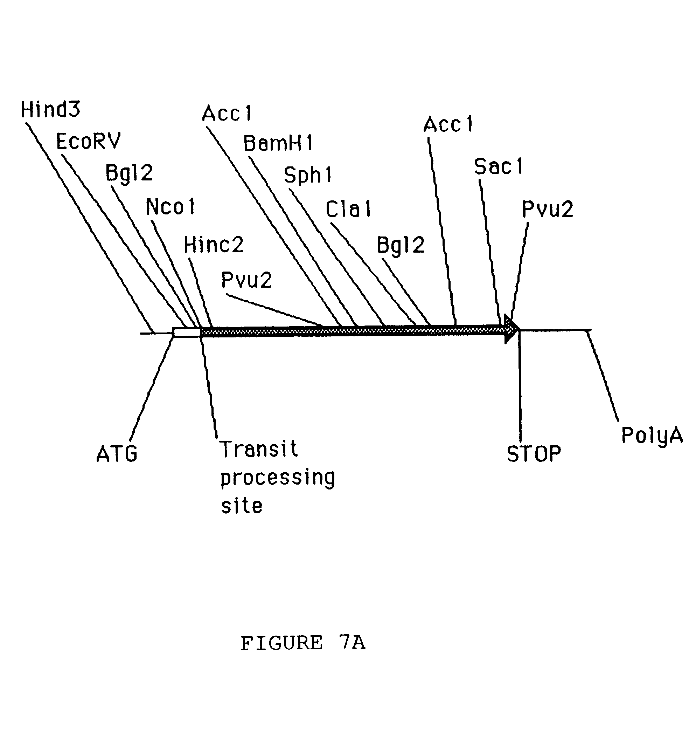

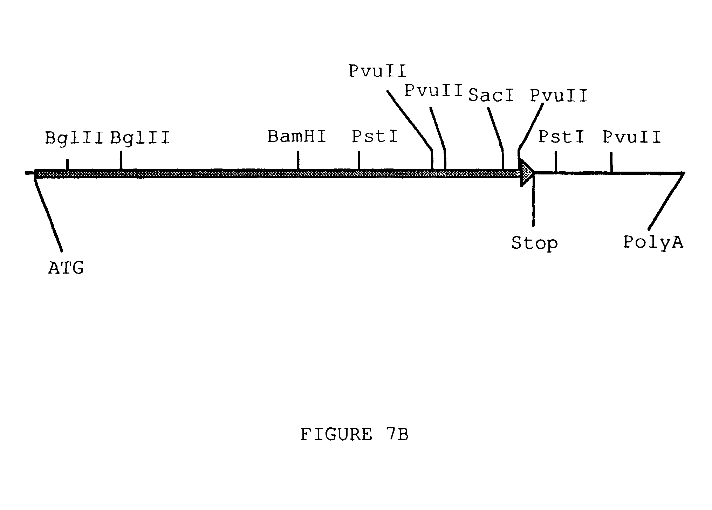

Clones are detected by hybridization with the DSAT-50 oligonucleotide and plaque purified. The complete nucleotide sequence (SEQ ID NO: 12) of the cDNA insert of a clone, pCGN2754, and a partial restriction map thereof are presented in Figures 2 and 7A, respectively. The cDNA insert includes 1533 bases plus a poly(A) track at the 3' end of 100-200 bases. The open reading frame for the desaturase begins at the first ATG (nucleotide 106) from the 5' end and stops at nucleotide 1294. The translated amino acid sequence is presented in Fig. 2 and SEQ ID NO: 13. The open reading frame includes a 33 amino acid transit peptide not found in the amino acid sequence of the mature protein. The N-terminus of the protein begins at the alanine immediately following the *Nco*I site (nucleotide 202) indicating the site of the transit peptide processing.

## Example 6

In this example, expression of a plant desaturase in a prokaryote is described.

*Desaturase expression construct in E. coli*

A plasmid for expression of desaturase activity in *E. coli* is constructed as follows. The desaturase cDNA clone pCGN2754 is digested with *Hin*dIII and *Sa*lI and ligated to pCGN2016 (a chloramphenicol resistant version of Bluescript KS-) digested with *Hin*dIII and *Xho*I. The resulting plasmid is pCGN1894.

pCGN2016 is prepared by digesting pCGN565 with *Hha*I, and the fragment containing the chloramphenicol resistance gene is excised, blunted by use of mung bean nuclease, and inserted into the *Eco*RV site of Bluescript KS- (Stratagene: La Jolla, CA) to create pCGN2008. The choramphenicol resistance gene of pCGN2008 is removed by *Eco*RI/*Hin*dIII digestion. After treatment with Klenow enzyme to blunt the ends, the fragment is ligated to *Dra*I digested Bluescript KS-. A clone that has the *Dra*I fragment containing ampicillin resistance replaced with the chloramphenicol resistance is chosen and named pCGN2016.

pCGN565 is a cloning vector based on pUC12-cm (K. Buckley Ph.D. Thesis, Regulation and expression of the phi X174 lysis gene, University of California, San Diego, 1985), but contains pUC18 linkers (Yanisch-Perron, *et al., Gene* (1985) *53*:103-119).

The fragment containing the mature coding region of the Δ-9 desaturase, 3'-noncoding sequences and poly (A) tails is subcloned from pCGN1894 digested with *Nco*1 and *Asp*718 into pUC120, an *E. coli* expression vector based on pUC118 (Vieira and Messing, *Methods in Enzymology* (1987) *153*:3-11) with the lac region inserted in the opposite orientation and an *Nco*I site at the ATG of the lac peptide (Vieira, J. PhD. Thesis, University of Minnesota, 1988). The *E. coli* desaturase expression plasmid is designated pCGN3201. The desaturase sequences are inserted such that they are aligned with the lac transcription and translation signals.

*Expression of Desaturase in E.coli*

Single colonies of *E. coli* strain 7118 (Maniatis *et al., supra*) containing pUC120 or pCGN3201 are cultured in 80 mls each of ECLB broth, 300 mg/L penicillin. The cells are induced by the addition of 1mM IPTG. Cells are grown overnight (18 hrs) at 37° C.

Eighty mls of overnight cultures of *E. coli* (induced and uninduced) containing pUC120 or pCGN3201 are centrifuged at 14,800 x g for 15 min. The pelleted cells are resuspended in 3 mls 20 mM phosphate buffer, pH 6.8. Resuspended cells were broken in a french press at 16,000 psi. Broken cell mixtures are centrifuged 5000xg for 5 min. 100 μl of each supernatant is applied to a G-25 Sephadex gel filtration centrifugal column (Boehringer Mannheim Biochemicals), equilibrated in 20mM phosphate buffer pH 6.8. Columns are spun for 4 min at 5000xg. Effluent was collected and used as enzyme source in the desaturase assay.

Desaturase activity is assayed as described in Example 1. Both pUC120-containing, IPTG-induced cells and uninduced cells do not express detectable stearoyl-ACP desaturase activity. The pCGN3201 IPTG-induced extract contains 8.22 nmol/min of desaturase activity. pCGN3201 uninduced extracts contains 6.45 nmol/min of activity. The pCGN3201 IPTG-induced extract shows 21.5% more activity than the uninduced pCGN3201 extract.

*Detection of induced protein in E. coli.*

Extracts of overnight cultures of *E. coli* strain 7118 (Maniatis *et al. supra* ) containing pCGN3201 or pUC120 grown in ECLB containing 300 mg/L penicillin induced with 1mM IPTG are prepared as follows. 1.5 ml of overnight culture grown shaking at 37°C are pelleted in Eppendorf tubes for 10 min at 10-13,000 μg. Pellets are resuspended in 150 ul SDS sample buffer (0.05M Tris-HCl, pH6.8, 1% SDS, 5% β-mercaptoethanol, 10% glycerol and 0.005% bromophenol blue) and boiled for 10 min. 25 μl of each sample are electrophoresed on a 10% polyacrylamide gel (Laemmli, *Nature* (1970) *227*:680) at 25 mA for 5 hours. Gels are stained in 0.05% Coomassie Brilliant Blue, 25% isopropanol and 10% acetic acid and destained in 10% acetic acid and 10% isopropanol. A band is detected at a position just below the 43,000 MW protein marker (SDS PAGE standard, Low molecular weight, BioRad, Richmond CA) in the pCGN3201 extracts that is not present in the pUC120 extracts. This is the approximate molecular weight of mature desaturase protein.

*Requirement for Spinach Ferredoxin*

Stearoyl-ACP desaturase can also be expressed in *E. coli* by subcloning into the *E. coli* expression vector pET8c (Studier, *et al., Methods Enzymol.* (1990) *185*:60-89). The mature coding region (plus an extra Met codon) of the desaturase cDNA with accompanying 3'-sequences is inserted as an *Nco*1 - *Sma* 1 fragment into pET8c at the *Nco*1and *Bam*H1 sites (after treatment of the *Bam*H1 site with Klenow fragment of DNA polymerase to create a blunt end) to create pCGN3208. The plasmid pCGN3208 is maintained in *E. coli* strain BL21(DE3) which contains the T7 polymerase gene under the control of the isopropyl-b-D-thiogalactopyranoside (IPTG)-inducible *lac*UV5 promoter (Studier *et al., supra*).

*E. coli* cells containing pCGN3208 are grown at 37°C to an $OD_{595}$ of ~O.7 in NZY broth containing 0.4% (w/v) glucose and 300 mg/liter penicillin, and then induced for 3 hours with 0.4 mM IPTG. Cells are pelleted from 1 ml of culture, dissolved in 125 µl of SDS sample buffer (10) and heated to 100°C for 10 min. Samples are analyzed by SDS polyacrylamide gel electrophoresis at 25 mA for 5 h. Gels are stained in 0.05% Coomassie Brilliant Blue, 25% (v/v) isopropanol and 10% (v/v) acetic acid. A band is detected at a position just below the 43,000 MW protein marker (SDS PAGE standard, Low Molecular Weight, BioRad, Richmond, CA) in the pCGN3208 extract that is not present in the pET8c extracts. This is the approximate molecular weight of mature desaturase protein.

For activity assays, cells are treated as described above and extracts are used as enzyme source in the stearoyl-ACP desaturase assay as described in Example 1. The extract from IPTG-induced pCGN3208 cells contains 8.61 nmol/min/mg protein of desaturase activity. The extract from pCGN3208 uninduced cells contains 1.41 nmol/min/mg protein of desaturase activity. The extract from pCGN3208 induced cells, thus has approximately 6-fold greater activity than the extract from uninduced pCGN3208 cells. Extracts from both induced and uninduced cells of pET8c do not contain detectable stearoyl-ACP desaturase activity.

Samples are also assayed as described in Example 1, but without the addition of spinach ferredoxin, to determine if the *E. coli* ferredoxin is an efficient electron donor for the desaturase reaction. Minimal activity is detected in *E. coli* extracts unless spinach ferredoxin is added exogenously.

## Example 7

In this example, the preparation of an ACP expression cassette containing a plant desaturase in a binary vector suitable for plant transformation is described.

*ACP Expression Cassette*

An expression cassette utilizing 5'-upstream sequences and 3'-downstream sequences obtainable from *B. campestris* ACP gene can be constructed as follows.

A 1.45kb *Xho*I fragment of Bcg 4-4 (Fig. 9 and SEQ ID NO: 28) containing 5'-upstream sequences is subcloned into the cloning/sequencing vector Bluescript+ (Stratagene Cloning Systems, San Diego, CA). The resulting construct, pCGN1941, is digested with *Xho*I and ligated to a chloramphenicol resistant Bluescript M13+ vector, pCGN2015 digested with *Xho*I. pCGN2015 is prepared as described for pCGN2016 (See, Example 6) except that the *Eco*RI/*Hin*dIII "chloramphenicol" fragment isolated from pCGN2008 is ligated with the 2273 bp fragment of Bluescript KS+ (Stratagene; LaJolla, CA) isolated after digestion with *Dra*I. This alters the antibiotic resistance of the plasmid from penicillin resistance to chloramphenicol resistance. The chloramphenicol resistant plasmid is pCGN1953.

3'-sequences of Bcg 4-4 are contained on an *Sst*I/BglII fragment cloned in the *Sst*I/*Bam*HI sites of M13 Bluescript+ vector. This plasmid is named pCGN1940. pCGN1940 is modified by *in vitro* site-directed mutagenesis (Adelman *et al., DNA* (1983) 2:183-193) using the synthetic oligonucleotide 5'-CTTAAGAAGTAACCCGGGCTGCAGTTTTAGTATTAAGAG-3' (SEQ ID NO: 33) to insert *Sma*I *a*nd *Pst*I restriction sites immediately following the stop codon of the reading frame for the ACP gene 18 nucleotides from the *Sst*I site. The 3'-noncoding sequences from this modified plasmid, pCGN1950, are moved as a *PsI*-*Sma*I fragment into pCGN1953 cut with *Pst*I and *Sma*I. The resulting plasmid pCGN1977 comprises the ACP expression cassette with the unique restriction sites *Eco*RV, *Eco*RI and *Pst*I available between the 1.45kb 5' and 1.5 kb of 3'-noncoding sequences (SEQ ID NO: 28) for the cloning of genes to be expressed under regulation of these ACP gene regions.

*Desaturase Expression in Plants*

Desaturase cDNA sequences from pCGN2754 are inserted in the ACP expression cassette, pCGN1977, as follows. pCGN2754 is digested with *Hin*dIII (located 160 nucleotides upstream of the start codon) and *Asp*718 located in the polylinker outside the poly(A) tails. The fragment containing the coding region for desaturase was blunt-ended using DNA polymerase I and ligated to pCGN1977 digested with *Eco*RV. A clone containing the desaturase sequences in the sense orientation with respect to the ACP promoter is selected and called pCGN1895. This expression cassette may be inserted into a binary vector, for example, for *Agrobacterium*-mediated transformation, or employed in other plant transformation techniques.

*Binary Vector and Agrobacterium Transformation*

The fragment containing the pCGN1895 expression sequences ACP 5'/desaturase/ACP 3' is cloned into a binary vector pCGN1557 (described below) for *Agrobacterium* transformation by digestion with Asp718 and XbaI and ligation to pCGN1557 digested with Asp718 and XbaI. The resulting binary vector is called pCGN1898.

pCGN1898 is transformed into *Agrobacterium tumefaciens* strain EHA101 (Hood, *et al., J. Bacteriol.* (1986) *168*:1291-1301) as per the method of Holsters, *et al., Mol. Gen. Genet.* (1978) *163*:181-187.

RNA blot analysis of seeds (T2) from T1 plants show the presence of a mRNA species for the inserted *C. tinctorius* desaturase, but the amount of message is low compared to endogenous levels of mRNA for the *Brassica* desaturase, suggesting that the expression levels were insufficient to significantly increase the amount of desaturase enzyme above that normally present. This is consistent with the negative results from oil, desaturase activity and Western blot analyses.

Construction of pCGN1557

pCGN1557 (McBride and Summerfelt, *Plant Molecular Biology* (1990) *14(2)*:269-276) is a binary plant transformation vector containing the left and right T-DNA borders of *Agrobacterium tumefaciens* octopine Ti-plasmid pTiA6 (Currier and Nester, *supra*, the gentamycin resistance gene of pPH1JI (Hirsch and Beringer, *supra*), an *Agrobacterium rhizogenes* Ri plasmid origin of replication from pLJbB11 (Jouanin *et al., supra*), *a* 35S promoter-kanR-tml3' region capable of conferring kanamycin resistance to transformed plants, a ColE1 origin of replication from pBR322 (Bolivar *et al., supra*), and a *lac*Z' screenable marker gene from pUC18 (Yanish-Perron *et al., supra*).

There are three major intermediate constructs used to generate pCGN1557:

PCGN1532 (see below) contains the pCGN1557 backbone, the pRi plasmid origin of replication, and the ColE1 origin of replication.

pCGN1546 (see below) contains the CaMV35S5'-kan$^R$-tml3' plant selectable marker region.

pCGN1541b (see below) contains the right and left T-DNA borders of the *A. tumefaciens* octopine Ti-plasmid and the *lac*Z' region from pUC19.

To construct pCGN1557 from the above plasmids, pCGN1546 is digested with *Xho*I, and the fragment containing the CaMV 35S5'-kan$^R$-tml3' region is cloned into the *Xho*I site of pCGN1541b to give the plasmid pCGN1553, which contains T-DNA/left border/CaMV 35S5'-kanR-tml3'/*lac*Z'/T-DNA left border. pCGN1553 is digested with *Bgl*II, and the fragment containing the T-DNA/left border/CaMV35S5'-kan$^R$-tml3'/*lac*Z'/T-DNA left border region is ligated into *Bam*HI-digested pCGN1532 to give the complete binary vector, pCGN1557.

pCGN1532

The 3.5kb *Eco*RI-*Pst*I fragment containing the gentamycin resistance gene is removed from pPh1JI (Hirsch and Beringer, *Plasmid* (1984) *12*:139-141) by *Eco*RI-*Pst*I digestion and cloned into *Eco*RI-*Pst*I digested pUC9 (Vieira and Messing, *Gene* (1982) *19*:259-268) to generate pCGN549. *Hin*dIII-*Pst*I digestion of pCGN549 yields a 3.1 kb fragment bearing the gentamycin resistance gene, which is made blunt ended by the Klenow fragment of DNA polymerase I and cloned into *Pvu*II digested pBR322 (Bolivar *et al., Gene* (1977) *2*:95-113) to create pBR322Gm. pBR322Gm is digested with *Dra*I and *Sph*I, treated with Klenow enzyme to create blunt ends, and the 2.8 kb fragment cloned into the Ri origin-containing plasmid pLJbB11 (Jouanin *et al., Mol. Gen. Genet.* (1985) *201*:370-374) which has been digested with *Apa*I and made blunt-ended with Klenow enzyme, creating pLHbB11Gm. The extra ColE1 origin and the kanamycin resistance gene are deleted from pLHbB11Gm by digestion with *Bam*HI followed by self closure to create pGmB11. The *Hind*II site of pGmB11 is deleted by *Hin*dIII digestion followed by treatment with Klenow enzyme and self closure, creating pGmB11-H. The *Pst*I site of pGmB11-H is deleted by *Pst*I digestion followed by treatment with Klenow enzyme and self closure, creating pCGN1532.

Construction of pCGN1546

The 35S promoter-tml3' expression cassette, pCGN986, contains a cauliflower mosaic virus 35S (CaMV35) promoter and a T-DNA tml 3'-region with multiple restriction sites between them. pCGN986 is derived from another cassette, pCGN206, containing a CaMV35S promoter and a different 3' region, the CaMV region VI 3'-end. The CaMV 35S promoter is cloned as an *Alu*I fragment (bp 7144-7734) (Gardner *et. al., Nucl. Acids Res.* (1981) *9*:2871-2888) into the *Hinc*II site of M13mp7 (Messing, *et. al., Nucl. Acids Res.* (1981) *9*:309-321) to create C614. An *Eco*RI digest of C614 produced the *Eco*RI fragment from C614 containing the 35S promoter which is cloned into the *Eco*RI site of pUC8 (Vieira and Messing, *Gene* (1982) *19*:259) to produce pCGN147.

pCGN148a containing a promoter region, selectable marker (KAN with 2 ATG's) and 3' region, is prepared by digesting pCGN528 with *Bgl*II and inserting the *Bam*HI-*Bgl*II promoter fragment from pCGN147. This fragment is cloned into the *Bgl*II site of pCGN528 so that the *Bgl*II site is proximal to the kanamycin gene of pCGN528.

The shuttle vector used for this construct pCGN528, is made as follows: pCGN525 is made by digesting a plasmid containing Tn5 which harbors a kanamycin gene (Jorgenson *et. al., Mol. Gen. Genet.* (1979) *177*:65) with *Hin*dIII-*Bam*HI and inserting the *Hin*dIII-*Bam*HI fragment containing the kanamycin gene into the *Hin*dIII-*Bam*HI sites in the tetracycline gene of pACYC184 (Chang and Cohen, *J. Bacteriol.* (1978) *134*:1141-1156). pCGN526 was made by inserting the *Bam*HI fragment 19 of pTiA6 (Thomashow *et. al., Cell* (1980) *19*:729-739), modified with *Xho*I linkers inserted into the *Sma*I site, into the *Bam*HI site of pCGN525. pCGN528 is obtained by deleting the small *Xho*I fragment from pCGN526 by digesting with *Xho*I and religating.

pCGN149a is made by cloning the *Bam*HI-kanamycin gene fragment from pMB9KanXXI into the *Bam*HI site of pCGN148a. pMB9KanXXI is a pUC4K variant (Vieira and Messing, *Gene* (1982) *19*:259-268) which has the *Xho*I site missing, but contains a functional kanamycin gene from Tn903 to allow for efficient selection in *Agrobacterium.*

pCGN149a is digested with *Hin*dIII and *Bam*HI and ligated to pUC8 digested with *Hin*dIII and *Bam*HI to produce pCGN169. This removes the Tn903 kanamycin marker. pCGN565 (see pCGN2016 description) and pCGN169 are both digested with *Hin*dIII and *Pst*I and ligated to form pCGN203, a plasmid containing the CaMV 35S promoter and part of the 5'-end of the Tn5 kanamycin gene (up to the *Pst*I site, Jorgenson *et. al*., (1979), *supra*). A 3'-regulatory region is added to pCGN203 from pCGN204 (an *Eco*RI fragment of CaMV (bp 408-6105) containing the region VI 3' cloned into pUC18 (Yanisch-Perron, *et al., Gene* (1985) *33*:103-119) by digestion with *Hin*dIII and *Pst*I and ligation. The resulting cassette, pCGN206, is the basis for the construction of pCGN986.

The pTiA6 T-DNA tml 3'-sequences are subcloned from the *Bam*19 T-DNA fragment (Thomashow *et al.*, (1980) *supra*) as a *Bam*HI-*Eco*RI fragment (nucleotides 9062 to 12,823, numbering as in Barker *et al., Plant Mol. Biol.* (1982) *2*:335-350) and combined with the pACYC184 (Chang and Cohen (1978), *supra*) origin of replication as an *Eco*RI-*Hin*dIII fragment and a gentamycin resistance marker (from plasmid pLB41), obtained from D. Figurski) as a *Bam*HI-*Hin*dIII fragment to produce pCGN417.

The unique *Sma*I site of pCGN417 (nucleotide 11,207 of the *Bam*19 fragment) is changed to a *Sac*I site using linkers and the *Bam*HI-*Sac*I fragment is subcloned into pCGN565 to give pCGN971. The *Bam*HI site of pCGN971 is changed to an *Eco*RI site using linkers. The resulting *Eco*RI-*Sac*I fragment containing the tml 3' regulatory sequences is joined to pCGN206 by digestion with *Eco*RI and *Sac*I to give pCGN975. The small part of the Tn5 kanamycin resistance gene is deleted from the 3'-end of the CaMV 35S promoter by digestion with *Sal*I and *Bgl*II, blunting the ends and ligation with *Sal*I linkers. The final expression cassette pCGN986 contains the CaMV 35S promoter followed by two *Sal*I sites, an *Xba*I site, *Bam*HI, *Sma*I, *Kpn*I and the tml 3' region (nucleotides 11207-9023 of the T-DNA).

The 35S promoter-tml 3' expression cassette, pCGN986 is digested with *Hin*dIII. The ends are filled in with Klenow polymerase and *Xho*I linkers added. The resulting plasmid is called pCGN986X. The *Bam*HI-*Sac*I fragment of pBRX25 (see below) containing the nitrilase gene is inserted into *Bam*HI-*Sac*I digested pCGN986X yielding pBRX66.

Construction of pBRX25 is described in U.S. Letters Patent 4,810,648, which is hereby incorporated by reference. Briefly, the method is as follows: The nucleotide sequence of a 1212-bp *Pst*I-*Hinc*II DNA segment encoding the bromoxynil-specific nitrilase contains 65-bp of 5' untranslated nucleotides. To facilitate removal of a portion of these excess nucleotides, plasmid pBRX9 is digested with *Pst*I, and treated with nuclease *Bal*31. *Bam*HI linkers are added to the resulting ends. *Bam*HI-*Hinc*II fragments containing a functional bromoxynil gene are cloned into the *Bam*HI-*Sma*I sites of pCGN565. The resulting plasmid, pBRX25, contains only 11 bp of 5' untranslated bacterial sequence.

pBRX66 is digested with *Pst*I and *Eco*RI, blunt ends generated by treatment with Klenow polymerase, and *Xho*I linkers added. The resulting plasmid pBRX68 now has a tml 3' region that is approximately 1.1kb. pBRX68 is digested with *Sal*I and *Sac*I, blunt ends generated by treatment with Klenow polymerase and *Eco*RI linkers added. The resulting plasmid, pCGN986XE is a 35S promoter - tml 3' expression cassette lacking the nitrilase gene.

The Tn5 kanamycin resistance gene is then inserted into pCGN986XE. The 1.0 kb *Eco*RI fragment of pCGN1536 (see pCGN1547 description) is ligated into pCGN986XE digested with *Eco*RI. A clone with the Tn5 kanamycin resistance gene in the correct orientation for transcription and translation is chosen and called pCGN1537b. The 35S promoter $Kan^R$-tml 3' region is then transferred to a chloramphenical resistant plasmid backbone. pCGN786, (a pUC-CAM based vector with the synthetic oligonucleotide 5' GGAATTCGTCGACAGATCTCTGCAGCTCGAGGGATCCAAGCTT 3' (SEQ ID NO: 34) containing the cloning sites *Eco*RI, *Sal*I, *Bgl*II, *Pst*I, *Xho*I, *Bam*HI, and *Hin*dIII inserted into pCGN566, pCGN566 contains the *Eco*HI-*Hin*dIII linker of pUC18 inserted into the *Eco*KI-*Hin*dIII sites of pUC13-cm (K. Buckler (1985) *supra*)) is digested with *Xho*I and the *Xho*I fragment of pCGN1537b containing the 35S promoter - $Kan^R$-tml 3' region is ligated in. The resulting clone is termed pCGN1546.

**pCGN1541b**

pCGN565RBα2X (see below) is digested with *Bgl*II and *Xho*I, and the 728bp fragment containing the T-DNA right border piece and the *lac*Z' gene is ligated with *Bgl*II-*Xho*I digested pCGN65ΔKX-S+K (see below), replacing the *Bgl*II-*Xho*I right border fragment of pCGN65ΔKX-S+K. The resulting plasmid, pCGN65α2X contains both T-DNA borders and the *lac*Z' gene. The *Cla*I fragment of pCGN65α2X is replaced with an *Xho*I site by digesting with *Cla*I blunting the ends using the Klenow fragment, and ligating with *Xho*I linker DNA, resulting in plasmid pCGN65α2XX. pCGN65α2XX is digested with *Bgl*II and *Eco*RV, treated with the Klenow fragment of DNA polymerase I to create blunt ends, and ligated in the presence of *Bgl*II linker DNA, resulting in pCGN65α2XX'. pCGN65α2XX' is digested with *Bgl*II and ligated with *Bgl*II digested pCGN1538 (see below), resulting in pCGN1541a, which contains both plasmid backbones. pCGN1541a is digested with *Xho*I and religated. Ampicillin resistant, chlormaphenicol sensitive clones are chosen, which lack the pACYC184-derived backbone, creating pCGN1541b.

pCGN1538 is generated by digesting pBR322 with *Eco*RI and *Pvu*II, treating with Klenow to generate blunt ends, and ligating with *Bgl*II linkers. pCGN1538 is ampicillin resistant, tetracycline sensitive.

**pCGN65ΔKX-S+K**

pCGN501 is constructed by cloning a 1.85 kb *Eco*RI-*Xho*I fragment of pTiA6 (Currier and Nester, *J. Bact.* (1976) *126*:157-165) containing bases 13362-15208 (Barker *et al., Plant Mo. Biol.* (1983) *2*:335-350) of the T-DNA (right border), into *Eco*RI-*Sal*I digested M13mp9 (Vieira and Messing, *Gene* (1982) *19*:259-268). pCGN502 is constructed by cloning a 1.6 kb *Hin*dIII-*Sma*I fragment of pTiA6, containing bases 602-2212 of the T-DNA (left border), into *Hin*dIII-*Sma*I digested M13mp9. pCGN501 and pCGN502 are both digested with *Eco*RI and *Hin*dIII and both T-DNA-containing fragments cloned together into *Hin*dIII digested pUC9 (Vieira and Messing, *Gene* (1982) *19*:259-268) to yield pCGN503, containing both T-DNA border fragments. pCGN503 is digested with *Hin*dIII and *Eco*RI and the two resulting *Hin*dIII-*Eco*RI fragments (containing the T-DNA borders) are cloned into *Eco*RI digested pHC79 (Hohn and Collins, *Gene* (1980) *11*:291-298) to generate pCGN518. The 1.6kb *Kpn*I-*Eco*RI fragment from pCGN518, containing the left T-DNA border, is cloned into *Kpn*I-*Eco*RI digested pCGN565 to generate pCGN580. The *Bam*HII-*Bgl*II fragment of pCGN580 is cloned into the *Bam*HI site of pACYC184 (Chang and Cohen, *J. Bacteriol.* (1978) *134*:1141-1156) to create pCGN51. The 1.4 kb *Bam*HI-*Sph*I fragment of pCGN60 containing the T-DNA right border fragment, is cloned into *Bam*HI-*Sph*I digested pCGN51 to create pCGN65, which contains the right and left T-DNA borders.

pCGN65 is digested with *Kpn*I and *Xba*I, treated with Klenow enzyme to create blunt ends, and ligated in the presence of synthetic *Bgl*II linker DNA to create pCGN65ΔKX. pCGN65ΔKX is digested with *Sal*I, treated with Klenow enzyme to create blunt ends, and ligated in the presence of synthetic *Xho*I linker DNA to create pCGN65ΔKX-S+X.

pCGN565RBα2X

pCGN451 (see below) is digested with *Hpa*I and ligated in the presence of synthetic *Sph*I linker DNA to generate pCGN55. The *Xho*I-*Sph*I fragment of pCGN55 (bp13800-15208, including the right border, of *Agrobacterium tumefaciens* T-DNA; (Barker *et al., Gene* (1977) *2*:95-113) is cloned into *Sal*I-*Sph*I digested pUC19 (Yanisch-Perron *et al., Gene* (1985) *53*:103-119) to create pCGN60. The 1.4 kb *Hin*dIII-*Bam*HI fragment of pCGN60 is cloned into *Hin*dIII-*Bam*HI digested pSP64 (Promega, Inc.) to generate pCGN1039. pCGN1039 is digested with *Sma*I and *Nru*I (deleting bp14273-15208; (Barker *et al., Gene* (1977) *2*:95-113) and ligated in the presence of synthetic *Bgl*II linker DNA creating PCGN1039ΔNS. The 0.47 kb *Eco*RI-*Hin*dIII fragment of pCGN1039ΔNS is cloned into *Eco*RI-*Hin*dIII digested pCGN565 to create pCGN565RB. The *Hin*dIII site of pCGN565RB is replaced with an *Xho*I site by digesting with *Hin*dIII, treating with Klenow enzyme, and ligating in the presence of synthetic *Xho*I linker DNA to create pCGN565RB-H+X.

pUC18 (Norrander *et al., Gene* (1983) *26*:101-106) is digested with *Hae*II to release the *lac*Z' fragment, treated with Klenow enzyme to create blunt ends, and the *lac*Z'-containing fragment ligated into pCGN565RB-H+X, which had been digested with *Acc*I and *Sph*I and treated with Klenow enzyme in such a orientation that the *lac*Z' promoter is proximal to the right border fragment; this construct, pCGN565RBα2x is positive for *lac*Z' expression when plated on an appropriate host and contains bp 13990-14273 of the right border fragment (Barker *et al., Plant Mo. Biol.* (1983) *2*: 335-350) having deleted the *Acc*I-*Sph*I fragment (bp 13800-13990).

pCGN451

pCGN451 contains an ocs5'-ocs3' cassette, including the T-DNA right border, cloned into a derivative of pUC8 (Vieira and Messing, *supra*). The modified vector is derived by digesting pUC8 with *Hin*cII and ligating in the presence of synthetic linker DNA, creating pCGN416, and then deleting the *Eco*RI site of pCGN416 by *Eco*RI digestion followed by treatment with Klenow enzyme and self-ligation to create pCGN426.

The ocs5'-ocs3' cassette is created by a series of steps from DNA derived from the octopine Ti-plasmid pTiA6 (Currier and Nester, *supra*). To generate the 5'end, which includes the T-DNA right border, an *Eco*RI fragment of pTiA6 (bp 13362-16202 (the numbering is by Barker, *et al., (Plant Mol. Bio* (1983) *2*:335-350) for the closely related Ti plasmid pTil5955)) is removed from pVK232 (Knauf and Nester, *Plasmid* (1982) *8*:45) by *Eco*RI digestion and cloned into *Eco*RI digested pACYC184 (Chang and Cohen, *supra*) to generate pCGN15.

The 2.4kb *Bam*HI-*Eco*RI fragment (bp 13774-16202) of pCGN15 is cloned into *Eco*RI-*Bam*HI digested pBR322 (Bolivar, *et al., supra*) to yield pCGN429. The 412 bp *Eco*RI-*Bam*HI fragment (bp 13362-13772) of pCGN15 is cloned into *Eco*RI-*Bam*HI digested pBR322 to yield pCGN407. The cut-down promoter fragment is obtained by digesting pCGN407 with *Xmn*I (bp 13512), followed by resection with *Bal*31 exonuclease, ligation of synthetic *Eco*RI linkers, and digestion with *Bam*HI. Resulting fragments of approximately 130 bp are gel purified and cloned into M13mp9 (Vieira and Messing, *supra*) and sequenced. A clone, I-4, in which the *Eco*RI linker has been inserted at bp 1362 between the transcription initiation point and the translation initiation codon is identified by comparison with the sequence of de Greve, *et al., (J. Mol. Appl. Genet.* (1982) *1*:499-512). The *Eco*RI cleavage site is at position 13639, downstream from the mRNA start site. The 141 bp *Eco*RI-*Bam*HI fragment of I-4, containing the cut-down promoter, is cloned into *Eco*RI-*Bam*HI digested pBR322 to create pCGN428. The 141 bp *Eco*RI-*Bam*HI promoter piece from pCGN428, and the 2.5 kb *Eco*RI-*Bam*HI ocs5' piece from pCGN429 are cloned together into *Eco*RI digested pUC19 (Vieira and Messing, *supra*) to generate pCGN442, reconstructing the ocs upstream region with a cut-down promoter section.

To generate the ocs3' end, the *Hin*dIII fragment of pLB41 (D. Figurski, UC San Diego) containing the gentamycin resistance gene is cloned into *Hin*dIII digested pACYC184 (Chang and Cohen, *supra*) to create pCGN413b. The 4.7 kb *Bam*HI fragment of pTiA6 (*supra*), containing the ocs3' region, is cloned into *Bam*HI digested pBR325 (F. Bolivar, *Gene* (1978) *4*:121-136) to create 33c-19. The *Sma*I site at position 11207 (Barker, *supra*) of 33c-19 is converted to an *Xho*I site using a synthetic *Xho*I linker, generating pCCG401.2. The 3.8 kb *Bam*HI-*Eco*RI fragment of pCGN401.2 is cloned into *Bam*HI-*Eco*RI digested pCGN413b to create pCGN419.

The ocs5'-ocs3' cassette is generated by cloning the 2.64 kb *Eco*RI fragment of pCGN442, containing the 5' region, into *Eco*RI digested pCGN419 to create pCNG446. The 3.1kb *Xho*I fragment of pCGN446, having the ocs 5 region (bp 13639-15208) and ocs3' region (bp 11207-12823) is cloned into the *Xho*I site of pCGN426 to create pCGN451.

## Example 8

In this example, the preparation of a Bce-4 expression cassette containing a plant desaturase is described.

The desaturase cDNA clone from pCGN2754 prepared as described in Example 5, is modified by *in vitro* mutagenesis to insert restriction sites immediately upstream of the ATG start codon and downstream of the TGA stop codon. A single-stranded template DNA is prepared for the mutagenesis reaction from pCGN1894 (described in Example 6) as described by Messing, (*Methods in Enzymol.* (1983) *101*:20-79). Synthetic oligonucleotides are synthesized on an Applied Biosystems 380A DNA synthesizer. The oligonucleotides used are 5'-CCATTTTTGATCTTCCTCGAGCCCGGGCTGCAGTTCTTCTTCTTCTTG-3' (SEQ ID NO: 35) for the 5'mutagenesis and 5'-GCTCGTTTTTTTTTTCTCTGCAGCCCGGGCTCGAGTCACAGCTTCACC -3' (SEQ ID NO: 36) for the 3'-mutagenesis; both add *Pst*I, *Sma*I and *Xho*I sites flanking the coding region. Both oligonucleotides are 5'-phosphorylated (BRL 5'-Terminus labelling kit) and used for mutagenesis with the pCGN1894 template by the procedure of Adelman *et al.* (*DNA* (1983) *2*:183-193). Alternatively, the desired restriction sites may be inserted by PCR, using the 3' oligo described above (SEQ ID NO: 36) and another oligo, 5' ACTGACTGCAGCCCGGGCTCGAGGAAGATCAAAAATGGCTCTTC 3' (SEQ ID NO: 37) for the 3' and 5' primers, respectively. The template in this polymerase chain reaction is DNA from pCGN1894. The *Xho*I fragment from the resulting clone can be subcloned into the Bce4 expression cassette, pCGN1870 (described below) at the unique *Xho*I site. This Bce4/desaturase expression cassette can then be inserted in a suitable binary vector, transformed into *Agrobacterium tumefaciens* strain EHA101 and used to transform plants as provided in Example 10.

### Bce-4 Expression Cassette

pCGN1870 is a Bce-4 expression cassette containing 5' and 3' regulatory regions of the Bce-4 gene and may be derived from the Bce-4 sequence found in pCGN1857, which was deposited with the ATCC on March 9, 1990, and assigned accession number 68251, or by methods known to one skilled in the art from the sequence (SEQ ID NO: 27) provided in Fig. 8. The Bce 4 gene may be isolated as follows:

The *Cla*I fragment of pCGN1857, containing the Bce4 gene is ligated into *Cla*I digested Bluescript KS+ (Stratagene; La Jolla, CA), producing pCGN1864. Single stranded DNA is made from pCGN1864 and altered by *in vitro* mutagenesis using the oligonucleotides

BCE45P :

(5'GAGTAGTGAACTTCATGGATCCTCGAGGTCTTGAAAACCTAGA3') (SEQ ID NO: 38)

and

BCE43P :

(5'CAATGTCTTGAGAGATCCCGGGATCCTTAACAACTAGGAAAAGG3') (SEQ ID NO: 39)

as described by Adelman *et al.* (*DNA* (1983) *2*:183-193). The oligonucleotide BSCP2 (5'GTAAGACACGACTTATCGCCACTG3') (SEQ ID NO: 4 , complementary to a portion of Bluescript, is included in the reaction to improve the yield of double-stranded DNA molecules. The resulting plasmid, pCGN1866, contains *Xho*I and *Bam*HI sites (from BCE45P) immediately 5' to the Bce4 start codon and *Bam*HI and *Sma*I sites (from BCE43P) immediately 3' to the Bce4 stop codon. The *Cla*I fragment of pCGN1866, containing the mutagenized sequences, is inserted into the *Cla*I site of pCGN2016 (described in Example 6), producing pCGN1866C. The *Cla*I fragment of pCGN1866C is used to replace the corresponding wild-type *Cla*I fragment of PCGN1867 (described below) to produce pCGN1868. Bce4 coding sequences are removed by digestion of pCGN1868 with *Bam*HI and recircularization of the plasmid to produce pCGN1870. The Bce4 expression cassette, pCGN1870, contains 7.4 kb of 5' regulatory sequence and 1.9 kb of 3' regulatory sequence derived from the Bce4 genomic clone separated by the cloning sites, *Xho*I, *Bam*HI, and *Sma*I. Desaturase sequences in sense or anti-sense orientation may be inserted into the cassette via the cloning sites and the resulting construct may be employed in a plant transformation technique.

<u>pCGN1867</u>

The *Bam*HI and *Sma*I sites of pUC18 are removed by *Bam*HI-*Sma*I digestion and recircularizing of the plasmid, without repair of the ends, to produce pCGN1862 The *Pst*I fragment of pCGN1857, containing the Bce4 gene, is inserted into the *Pst*I site of pCGN1862 to produce pCGN1867.

## <u>Example 9</u>

In this example, the preparation of a napin 1-2 expression cassette containing a plant desaturase is described.

*Preparation of Desaturase Clone*

The desaturase cDNA clone from pCGN2754 is prepared and modified as described in Example 8. The *Xho*I fragment from the resulting clone can be subcloned into the napin 1-2 expression cassette, pCGN1808 (described below) at the unique *Xho*I site. This napin 1-2/desaturase expression cassette can then be inserted into a suitable binary vector, transformed into *A. tumefaciens* strain EHA101 in a like manner as described in Example 7.

Alternatively, the desaturase safflower clone may be prepared such that restriction sites flank the translation start and stop sites, as described in Example 8, with the following modification. PCR was carried out according to manufacturer's instructions except for the initial annealing of the oligonucleotides to the template. The reaction mix was heated to 90°C for 5 min, cooled to 37°C over a one hour period, kept at 37°C for 20 min and then subjected to standard PCR cycles. The PCR product was digested with *Pst*I and ligated to pUC8 (Vieira and Messing (1982) *Gene 19*:2359-268) digested with *Pst*I to produce pCGN3220. The *Nco*I/*Sac*I fragment of pCGN3220 containing the pUC8 vector and the 5' and 3' sequences of the safflower desaturase cDNA was gel purified and ligated to the gel-purified cloned *Nco*I/*Sac*I fragment from pCGN1894 (see Example 6). The resulting plasmid pCGN3222 contains safflower desaturase cDNA sequences partially from the cDNA clone and partially from the PCR. The regions obtained from the PCR were confirmed by DNA sequencing as being identical to the original cloned sequence.

*Expression Cassettes*

Napin 1-2 pCGN1808 Expression Cassette

An expression cassette utilizing 5' upstream sequences and 3' downstream sequences obtainable from *B. campestris* napin gene can be constructed as follows.

A 2.7 kb *Xho*I fragment of napin 1-2 (Fig. 10 and SEQ ID NO: 29) containing 5' upstream sequences is subcloned into pCGN789 (a pUC based vector the same as pUC119 with the normal polylinker replaced by the synthetic linker -5'GGAATTCGTCGACAGATCTCTGCAGCTCGAGGGATCCAAGCTT 3', SEQ ID NO: 41, (which represented the polylinker *Eco*RI, *Sal*I, *Bgl*II, *Pst*I, *Xho*I, *Bam*HI, *Hin*dIII) and results in pCGN940. The majority of the napin coding region of pCGN940 was deleted by digestion with *Sal*I and religation to form pCGN1800. Single-stranded DNA from pCGN1800 was used in an *in vitro* mutagenesis reaction (Adelman *et al., DNA* (1983) *2*:183-193) using the synthetic oligonucleotide 5' GCTTGTTCGCCATGGATATCTTCTGTATGTTC 3', SEQ ID NO: 42. This oligonucleotide inserted an *Eco*RV and an *Nco*I restriction site at the junction of the promoter region and the ATG start codon of the napin gene. An appropriate mutant was identified by hybridization to the oligonucleotide used for the mutagenesis and sequence analysis and named pCGN1801.

A 1.7 kb promoter fragment was subcloned from pCGN1801 by partial digestion with *Eco*RV and ligation to pCGN786 (a pCGN566 chloramphenicol based vector with the synthetic linker described above in place of the normal polylinker) cut with *Eco*RI and blunted by filling in with DNA Polymerase I Klenow fragment to create pCGN1802.

A 2.1 kb *Sal*I fragment of napin 1-2 (Fig. 10 and SEQ ID NO: 29) containing 3' downstream sequences is subcloned into pCGN789 (described above) and results in pCGN941. pCGN941 is digested with *Xho*I and *Hin*dIII and the resulting approximately 1.6 kb of napin 3' sequences are inserted into *Xho*I-*Hin*dIII digested pCGN1802 to result in pCGN1803. In order to remove a 326 nucleotide *Hin*dIII fragment inserted opposite to its natural orientation, as a result of the fact that there are 2 *Hin*dIII sites in pCGN1803, the pCGN1803 is digested with *Hin*dIII and religated. Following religation, a clone is selected which now contains only 1.25 kb of the original 1.6 napin 3' sequence. This clone, pCGN1808 is the napin 1-2 expression cassette and contains 1.725 kb of napin promoter sequences and 1.265 kb of napin 3' sequence with the unique cloning sites *Sal*I, *Bgl*I, *Pst*I and *Xho*I in between.

Napin 1-2 pCGN3223 Expression Cassette

Alternatively, pCGN1808 may be modified to contain flanking restriction sites to allow movement of only the expression sequences and not the antibiotic resistance marker to binary vectors such as pCGN1557 (McBride and Summerfelt, *supra*). Synthetic oligonucleotides containing *Kpn*I, *Not*I and *Hin*dIII restriction sites are annealed and ligated at the unique *Hin*dIII site of pCGN1808, such that only one *Hin*dIII site is recovered. The resulting plasmid, pCGN3200 contains unique *Hin*dIII, *Not*I and *Kpn*I restriction sites at the 3'-end of the napin 3'-regulatory sequences as confirmed by sequence analysis.

The majority of the napin expression cassette is subcloned from pCGN3200 by digestion with *Hin*dIII and *Sac*I and ligation to *Hin*dIII and *Sac*I digested pIC19R (Marsh, *et al.* (1984) *Gene 32*:481-485) to make pCGN3212. The extreme 5'-sequences of the napin promoter region are reconstructed by PCR using pCGN3200 as a template and two primers flanking the *Sac*I site and the junction of the napin 5'-promoter and the pUC backbone of pCGN3200 from the pCGN1808 construct. The forward primer contains *Cla*I, *Hin*dIII, *Not*I, and *Kpn*I restiction sites as well as nucleotides 408-423 of the napin 5'-sequence (from the *Eco*RV site) and the reverse primer contains the complement to napin sequences 718-739 which include the unique *Sac*I site in the 5'-promoter. The PCR was performed using a Perkin Elmer/Cetus thermocycler according to manufacturer's specifications. The PCR fragment is subcloned as a blunt-ended fragment into pUC8 (Vieira and Messing (1982) *Gene 19*:259-268) and digested with *Hin*cII to give pCGN3217. Sequence of pCGN3217 across the napin insert verifies that no improper nucleotides were introduced by PCR. The napin 5-sequences in pCGN3217 are ligated to the remainder of the napin expression cassette by digestion with *Cla*I and *Sac*I and ligation to pCGN3212 digested with *Cla*I and *Sac*I. The resulting expression cassette pCGN3221, is digested with *Hin*dIII and the napin expression sequences are gel purified away and ligated to pIC20H (Marsh, *supra*) digested with *Hin*dIII. The final expression cassette is pCGN3223, which contains in an ampicillin resistant background, essentially identical 1.725 napin 5' and 1.265 3' regulatory sequences as found in pCGN1808. The regulatory regions are flanked with *Hin*dIII, *Not*I and *Kpn*I restriction sites and unique *Sal*I, *Bgl*II, *Pst*I, and *Xho*I cloning sites are located between the 5' and 3' noncoding regions.

Desaturase sequences in sense or anti-sense orientation may be inserted into a napin expression cassette via the cloning sites. The resulting construct may be employed for plant transformation. For example, one of ordinary skill in the art could also use known techniques of gene cloning, mutations, insertion and repair to allow cloning of a napin expression cassette into any suitable binary vector, such as pCGN1557 (described in Example 7) or other similar vectors.

*Desaturase Expression*

The coding region of the safflower desaturase contained in pCGN3222 is cloned into the pCGN3223 napin cassette by digestion with *Xho*I and ligation to pCGN3223 digested with *Xho*I and *Sal*I. The resulting plasmid, pCGN3229 is digested with *Asp*718 and inserted in the binary vector pCGN1578 (McBride and Summerfelt (1990) *Plant Mol. Biol. 14*:269-276) at the unique *Asp*718 site. The resulting binary vector is pCGN3231 and contains the safflower desaturase coding sequences flanked by the napin 5' and 3' regulatory sequences as well as the plant selectable marker construct, 35s/NPTII/tml.

The resulting binary vector, pCGN3231, is transformed into *Agrobacterium* and utilized for plant transformation as described in Example 10. For Northern analysis, total RNA is isolated from day 21 and day 28 post-anthesis developing seed from plants transformed with pCGN3231. Five samples were analyzed at day 21 and two at day 28 post-anthesis. RNA was isolated by the method of Hughes and Galau (*Plant Mol. Biol. Reporter* (1988) *6*: 253-257). Northern blot analysis was performed using a labeled 0.8 kb *Bgl*II fragment of pCGN1894 as a probe. Prehybridization and hybridization was at 42°C in 50% formamide, 10X Denhardt's solution, 5X SSC, 0.1% SDS, 5mM EDTA and 100ug/ml denatured salmon sperm DNA. Filters were washed at 55°C in 0.1 X SSC, 0.1% SDS. Under these conditions, the probe does not hybridize to the endogenous *Brassica* desaturase gene sequences. mRNA complementary to the safflower desaturase was detected in all the transgenic samples examined. More mRNA was present at day 28 than at day 21 post-anthesis and the highest level of RNA was seen in transgenic 3231-8. The total safflower desaturase mRNA level was estimated to be ~0.01% of the message at day 28 post-anthesis.

Western analysis (see below) gives a preliminary indication of increased protein in one transformant, 3231-8. However, the Western analysis is complicated by two factors: 1. The presence of cross-reacting material at the same molecular weight as expected for the safflower desaturase. We believe this material is the endogenous *Brassica* desaturase. 2. The analysis of levels of protein expressed is also complicated by the normal developmental increase in the expression of desaturase protein during this time period. If the seeds examined are not at the precise developmental stage as the control seeds, quantitative differences in the amount of material seen may be simply due to the normal increase in the *Brassica* desaturase over this time period and not due to the expression of the safflower desaturase.

Western Analysis

Soluble protein is extracted from developing seeds of *Brassica* by homogenization with one volume (1ml/gram fresh weight) of buffer containing 20mM potassium phosphate, pH 6.8. The homogenate is clarified by centrifugation at 12,000 x *g* for 10 minutes. A second centrifugation is performed if necessary to provide a non-particulate supernatant.

Protein concentration of the extract is measured by the micromethod of Bradford (*Anal. Biochem.* (1976) *72*: 248-254). Proteins (20-60μg) are separated by denaturing electrophoresis by the method of Laemmli (*supra*), and are transferred to nitrocellulose membrane by the method of Towbin *et al.* (*Proc. Nat. Acad Sci.* (1979) *76*:4350-4354).

The nitrocellulose membrane is blocked by incubation at room temperature for 15 minutes or at 4°C overnight in Tris-buffered saline with Tween 20 (Polyoxyethylenesorbitan monolaurate) and "TTBS-milk", (TTBS = 20mM Tris-HCl, 500mM NaCl, 0.1% Tween 20 (v/v), pH 7.5; "TTBS-milk" = TTBS and 3% skim milk powder). The volume of liquid in all incubations with the nitrocellulose membrane is sufficient to cover the membrane completely. The membrane is then incubated for an additional 5 minutes in TTBS.

The nitrocellulose membrane is incubated for at least one hour with shaking at room temperature with rabbit anti-stearoyl-ACP desaturase antiserum that was diluted 5,000- or 10,000-fold in "TTBS-milk". The rabbit anti-desaturase antiserum was commercially prepared from desaturase protein (purified as described in Example 1) by Berkeley Antibody Co. (Richmond, CA). The membrane is washed twice by shaking with TTBS for 5 minutes and then with deionized $H_2O$ for 30 seconds.

The nitrocellulose membrane is incubated for at least 45 minutes at room temperature in a solution of "TTBS-milk" in which anti-rabbit IgG-alkaline phosphatase conjugate (Promega, Madison, WI) is diluted 7,500-fold. The membrane is washed twice in TTBS followed by deionized $H_2O$, as described above.

The nitrocellulose membrane is equilibrated in buffer containing 100mM Tris-HCl, 100mM NaCl, 50mM $MgCl_2$, pH 9.5, by shaking for 5 minutes. The color reaction is initiated by placing the nitrocellulose membrane into 50ml of the same buffer to which has been added 15mg p-nitroblue tetrazolium chloride and 7.5mg 5-bromo- 4 chloro- 3-indolyl phosphate toluidine salt (BioRad Labs; Richmond, CA). The color reaction is stopped by rinsing the nitrocellulose

membrane with deionized $H_2O$ and drying it between filter papers.

Oil analysis of developing seeds indicated no significant change in oil composition of the transformed plants with respect to the control plants. This result is consistant with the low levels of safflower mRNA observed in transgenic plants as compared to levels of endogenous *Brassica* desaturase (Example 12).

## Example 10

In this example, an Agrobacterium-mediated plant transformation is described. *Brassica napus* is exemplified. The method is also useful for transformation of other *Brassica* species including *Brassica campestris.*

*Plant Material and Transformation*

Seeds of *Brassica napus* cv. Delta are soaked in 95% ethanol for 2 min, surface sterilized in a 1.0% solution of sodium hypochlorite containing a drop of Tween 20 for 45 min., and rinsed three times in sterile, distilled water. Seeds are then plated in Magenta boxes with 1/10th concentration of Murashige minimal organics medium (Gibco) supplemented with pyrodoxine (50 μg/l), nicotinic acid (50 μg/l), glycine (200 μg/l), and 0.6% Phytagar (Gibco) pH 5.8. Seeds are germinated in a culture room at 22°C in a 16 h photoperiod with cool fluorescent and red light of intensity approximately 65 μEinsteins per square meter per second ($\mu Em^{-2}S^{-1}$).

Hypocotyls are excised from 7 day old seedlings, cut into pieces approximately 4 mm in length, and plated on feeder plates (Horsch et al. 1985). Feeder plates are prepared one day before use by plating 1.0 ml of a tobacco suspension culture onto a petri plate (100x25 mm) containing about 30 ml MS salt base (Carolina Biological) 100 mg/l inositol, 1.3 mg/l thiamine-HCl, 200 mg $KH_2PO_4$ with 3% sucrose, 2,4-D (1.0 mg/l), 0.6% Phytagar, and pH adjusted to 5.8 prior to autoclaving (MS0/1/0 medium). A sterile filter paper disc (Whatman 3 mm) is placed on top of the feeder layer prior to use. Tobacco suspension cultures are subcultured weekly by transfer of 10 ml of culture into 100 ml fresh MS medium as described for the feeder plates with 2,4-D (0.2 mg/l), Kinetin (0.1 mg/l). All hypocotyl explants are preincubated on feeder plates for 24 h. at 22°C in continuous light of intensity 30 $\mu Em^{-2}S^{-1}$ to 65 $\mu EM^{-2}S^{-1}$.

Single colonies of *A. tumefaciens* strain EHA101 containing a binary plasmid are transferred to 5 ml MG/L broth and grown overnight at 30°C. Per liter, MG/L broth contains 5g mannitol, 1 g L-glutamic acid or 1.15 g sodium glutamate, 0.25 g $kH_2PO_4$, 0.10 g NaCL, 0.10 g $MGSO_4 \cdot 7H_2O$, 1 mg biotin, 5 g tryptone, and 2.5 g yeast extract, and the broth is adjusted to pH 7.0. Hypocotyl explants are immersed in 7-12 ml MG/L broth with bacteria diluted to $1x10^8$ bacteria/ml and after 10-20 min. are placed onto feeder plates. After 48 h of co-incubation with *Agrobacterium*, the hypocotyl explants are transferred to B5 0/1/0 callus induction medium which contains filter sterilized carbenicillin (500 mg/l, added after autoclaving) and kanamycin sulfate (Boehringer Mannheim) at concentrations of 25 mg/l.

After 3-7 days in culture at 65 $\mu Em^{-2}S^{-1}$ to 75 $\mu Em^{-2}S^{-1}$ continuous light, callus tissue is visible on the cut surface and the hypocotyl explants are transferred to shoot induction medium, B5BZ (B5 salts and vitamins supplemented with 3 mg/l benzylaminopurine, 1 mg/l zeatin, 1% sucrose, 0.6% Phytagar and pH adjusted to 5.8). This medium also contains carbenicillin (500 mg/l) and kanamycin sulfate (25 mg/l). Hypocotyl explants are subcultured onto fresh shoot induction medium every two weeks.

Shoots regenerate from the hypocotyl calli after one to three months. Green shoots at least 1 cm tall are excised from the calli and placed on medium containing B5 salts and vitamins, 1% sucrose, carbenicillin (300 mg/l), kanamycin sulfate (50 mg/l) and 0.6% Phytagar) and placed in a culture room with conditions as described for seed germination. After 2-4 weeks shoots which remain green are cut at the base and transferred to Magenta boxes containing root induction medium (B5 salts and vitamins, 1% sucrose, 2 mg/l indolebutyric acid, 50 mg/l kanamycin sulfate and 0.6% Phytagar). Green rooted shoots are tested for NPT II activity.

## Example 11

In this example, a DNA-bombardment plant transformation is described. Peanut transformation is exemplified.

DNA sequences of interest may be introduced as expression cassettes, comprising at least a promoter region, a gene of interest, and a termination region, into a plant genome via particle bombardment as described in European Patent Application 332 855 and in co-pending application USSN 07/225,332, filed July 27, 1988.

Briefly, tungsten or gold particles of a size ranging from 0.5μM-3μM are coated with DNA of an expression cassette. This DNA may be in the form of an aqueous mixture or a dry DNA/particle precipitate.

Tissue used as the target for bombardment may be from cotyledonary explants, shoot meristems, immature leaflets, or anthers.

The bombardment of the tissue with the DNA-coated particles is carried out using a Biolistics™ particle gun (Dupont; Wilmington, DE). The particles are placed in the barrel at variable distances ranging from 1cm-14cm from the barrel mouth. The tissue to be bombarded is placed beneath the stopping plate; testing is performed on the tissue at distances up to 20 cm. At the moment of discharge, the tissue is protected by a nylon net or a combination of nylon nets with mesh ranging from 10μM to 300μM.

Following bombardment, plants may be regenerated following the method of Atreya, *et al.,* (*Plant Science Letters* (1984) *34*:379-383). Briefly, embryo axis tissue or cotyledon segments are placed on MS medium (Murashige and Skoog, *Physio. Plant.* (1962) *15*:473) (MS plus 2.0 mg.l 6-benzyladenine (BA) for the cotyledon segments) and incubated in the dark for 1 week at 25 ± 2°C and are subsequently transferred to continuous cool white fluorescent light (6.8 $W/m^2$). On the 10th day of culture, the plantlets are transferred to pots containing sterile soil, are kept in the shade for 3-5 days are and finally moved to greenhouse.

The putative transgenic shoots are rooted. Integration of exogenous DNA into the plant genome may be confirmed by various methods known to those skilled in the art.

**Example 12**

This example describes methods to obtain desaturase cDNA clones from other plant species using the DNA from the *C. tinctorius* Δ-9 desaturase clone as the probe.

*Isolation of RNA for Northern Analysis*

Poly(A)+ RNA is isolated from *C. tinctorius* embryos collected at 14-17 days post-anthesis and *Simmondsia chinensis* embryos as described in Example 5.

Total RNA is isolated from days 17-18 days post-anthesis *Brassica campestris* embryos by an RNA minipreparation technique (Scherer and Knauf, *Plant Mol. Biol.* (1987) *9*:127-134). Total RNA is isolated from *R. communis* immature endosperm of about 14-21 days post-anthesis by a method described by Hailing, *et al.* (*Nucl. Acids Res.* (1985) *13*:8019-8033). Total RNA is isolated from 10 g each of young leaves from *B. campestris, B. napus*, and *C. tinctorius*, by extraction of each sample in 5 ml/g tissue of 4 M guanidine thiocyanate buffer as described by Colbert *et al.* (*Proc. Nat. Acac. Sci.* (1983) *80*:2248-2252). Total RNA is also isolated from immature embryos of *Cuphea hookeriana* by extraction as above in 10 ml/g tissue.

Total RNA is isolated from immature embryos of California bay (*Umbellularia californica*) by an adaptation of the method of Lagrimini *et al.* (*Proc. Nat. Acad. Sci.* (1987) *84*:7542-7546). Following homogenization in grinding buffer (2.5 ml/g tissue) as described, RNA is precipitated from the aqueous phase by addition of 1/10 volume 3 M sodium acetate and 2 volumes ethanol, followed by freezing at -80°C for 30 minutes and centrifugation at 13,000 x g for 20 minutes. The pellets are washed with 80% ethanol and centrifugation is repeated as above. The pellets are resuspended in water, two volumes of 4 M LiCl are added, and the samples are placed at -20°C overnight. Samples are centrifuged as above and the pellets washed with 80% ethanol. Ethanol precipitation is repeated as above.

Total RNA is further purified from *B. campestris, B. napus,* and *C. tinctorius* leaves, and from *C. tinctorius, B. campestris*, California bay, and jojoba, and from *R. communis* immature endosperm, by removing polysaccharides on a 0.25 g Sigma Cell 50 cellulose column. The RNA is loaded onto the column in 1 ml of loading buffer (20 mM Tris-HCl pH 7.5, 0.5M NaCl, ImM EDTA, 0.1% SDS), eluted with loading buffer, and collected in 500 μl fractions. Ethanol is added to the samples to precipitate the RNA. The samples are centrifuged, and the pellets resuspended in sterile distilled water, pooled, and again precipitated in ethanol. The sample is centrifuged, and the resulting RNA is subjected to oligo(dT)-cellulose chromatography to enrich for poly(A)+ RNA as described by Maniatis *et al.* (*Molecular Cloning: A Laboratory Manual*, Cold Spring Harbor Laboratory, New York (1982)). Poly(A)+ RNA is also purified from total *Cuphea hookeriana* RNA by oligo(dT)-cellulose chromatography.

*Northern Analysis Using C. tinctorius Desaturase*

*Clone:* 2.5 μg of poly$(A)^+$ RNA from each of the above described poly(A)+ samples from immature embryos of jojoba, *Cuphea hookeriana,* California bay, *Brassica campestris*, and *C. tinctorius*, from immature endosperm of *R. communis*, and from leaves of *C. tinctorius, B. campestris*, and *B. napus* are electrophoresed on formaldehyde/agarose gels (Fourney *et al., Focus* (1988) *10*:5-7) and transferred to a Hybond-C extra (Amersham, Arlington Heights, IL) filter according to manufacturer's specifications. The filter is prehybridized for four hours and hybridized overnight at 42°C in a roller bottle containing 10 ml of hybridization buffer (1 M NaCl, 1% SDS, 50% formamide, 0.1 mg/ml denatured salmon sperm DNA) in a Hybridization Incubator, model 1040-00-1 (Robbins Scientific Corporation, Sunnyvale, CA). The probe used in the hybridization is a gel-isolated *Bgl*II fragment of the Δ-9 desaturase clone that is labeled with $^{32}P$-dCTP using a BRL (Gaithersburg, MD) nick-translation kit, following manufacturer's instructions. The blot is washed three times for 20 minutes each in 2X SSC, 0.5% SDS at 55°C. The blot is exposed at -80°C, with a Dupont Cronex intensifying screen, to X-ray film for four days.

The autoradiograph shows that the *C. tinctorius* desaturase gene is expressed in both immature embryos and leaves of *C. tinctorius*, although the level of expression is considerably higher in embryos than in leaves. The autoradiograph also shows hybridization of the *C. tinctorius* desaturase clone to mRNA bands of a similar size in immature embryos from jojoba and California bay, and immature endosperm from *R. communis.* Hybridization is also detectable in RNA from *B. campestris* embryos upon longer exposure of the filter to X-ray film.

*R. communis cDNA Library Construction*: A plant seed cDNA library may be constructed from poly(A)+ RNA isolated from *R. communis* immature endosperm as described above. The plasmid cloning vector pCGN1703, and cloning method are as described in Example 5. The *R. communis* endosperm cDNA bank contains approximately $2x10^6$ clones with an average cDNA insert size of approximately 1000 base pairs.

The *R. communis* immature endosperm cDNA bank is moved into the cloning vector lambda gt22 (Stratagene Cloning Systems) by digestion of total cDNA with *Not*I and ligation to lambda gt22 DNA digested with *Not*I. The resulting phage are packaged using a commercially available kit and titered using *E. coli* strain LE392 (Stratagene Cloning Systems, La Jolla, CA). The titer of the resulting library was approximately $1.5 \times 10^7$ pfu/ml.

*R. communis cDNA Library Screen*: The library is plated on *E. coli* strain LE392 at a density of approximately 25,000 pfu/150mm NZY plate to provide approximately 50,000 plaques for screening. Phage are lifted in duplicate on to NEN (Boston, MA.) Colony/Plaque Screen filters as described in Example 5. Following prehybridization at 42°C in 25 ml of hybridization buffer (1 M NaCl, 1% SDS, 50% formamide, 0.1 mg/ml denatured salmon sperm DNA) filters are hybridized overnight with a gel-purified 520 base pair *Bgl*II fragment of the C. *tinctorius* desaturase clone (Figure 7A) that is radiolabeled with $^{32}$P-dCTP using a BRL (Gaithersburg, MD) Nick Translation System. Filters are washed three times for 20 minutes each in 2X SSC, 0.5% SDS at 55°C in a shaking water bath. Filters are exposed to X-ray film overnight at -80°C with a Dupont Cronex intensifying screen.

Clones are detected by hybridization on duplicate filters with the *C. tinctorius* desaturase cDNA fragment and plaque purified. During plaque purification, it was observed that larger plaques were obtained when *E. coli* strain Y1090 (Young, R.A. and Davis, R.W., *Proc. Natl. Acad. Sci. USA* (1983) *80*:1194) was used as the host strain. This strain was thus used in subsequent plaque purification steps. Phage DNA is prepared from the purified clones as described by Grossberger (*NAR* (1987) *15*:6737) with the following modification. The proteinase K treatment is replaced by the addition of 10% SDS and a 10 minute incubation at room temperature. Recovered phage DNA is digested with *Eco*RI, religated at low concentration, and transformed into *E. coli* DH5α(BRL; Gaithersburg, MD) cells to recover plasmids containing cDNA inserts in pCGN1703. Minipreparation DNA (Maniatis *et al., supra*) is prepared from the clones and DNA sequence is determined as described above. Partial nucleotide sequence of the cDNA insert of a *R. communis* desaturase clone pCGN3230 is presented in Figure 3A and SEQ ID NO: 14. The complete nucleotide sequence of this clone is presented in Fig. 3B and as SEQ ID NO: 15.

*Northern Analysis Using R. communis Desaturase Clone*: Total RNA for Northern analysis is isolated from tobacco leaves by the method of Ursin *et al.* (*Plant Cell* (1989) *1*:727-736), petunia and tomato leaves by the method of Ecker and Davis (*Proc.Nat.Acad.Sci.* (1987) *84*:5202-5206), and corn leaves by the method of Turpen and Griffith (*Biotechniques* (1986) *4*:11-15). Total RNA samples from tobacco, corn, and tomato leaves are enriched for poly(A)$^+$ RNA by oligo(dT)-cellulose chromatography as described by Maniatis *et al.* (*supra*).

Poly(A)+ RNA samples from tomato leaves (4 μg) and corn and tobacco leaves (1 μg each), and total RNA from petunia leaves (25 μg) are electrophoresed on a formaldehyde/agarose gel as described by Shewmaker *et al.* (*Virology* (1985) *140*:281-288). Also electrophoresed on this gel are poly(A)+ RNA samples isolated from *B. campestris* day 17-19 embryos and *B. campestris* leaves (2 μg each), immature embryos from *C. tinctorius*, bay, and jojoba (1 μg each), and *R. communis* endosperm (1 μg). The isolation of these poly(A)+ RNA samples is described above for the Northern analysis using *C. tinctorius* desaturase cDNA as probe. The RNA is transferred to a nitrocellulose filter as described by Shewmaker *et al.* (*supra*) and prehybridized and hybridized at 42°C in 50% formamide, 10X Denhardt's solution (described in Maniatis *et al*. (supra)), 5X SSC, 0.1% SDS, 5 mM EDTA, 100 ug/ml denatured salmon sperm DNA, and 10% dextran sulfate (in hybridization buffer only). The probe for hybridization is the $^{32}$P-labeled (BRL Nick Translation Kit) 1.7 kb SalI insert of pCGN3230 that has been gel-purified from minipreparation DNA. The filter is washed following hybridization for 30 minutes in 2X SSC, 0.1% SDS at 42°C and at 50°C twice for 15 minutes each. The filter is exposed to X-ray film overnight at -80°C with a Dupont Cronex intensifying screen.

The autoradiograph shows hybridization of the *R. communis* desaturase clone to mRNA bands of a similar size in immature embryos from *B. campestris*, California bay, and *C. tinctorius*, and also in corn leaves and *R. communis* endosperm.

*B. campestris Embryo cDNA Library Construction:* Total RNA is isolated from 5 g of *B. campestris* cv. R500 embryos obtained from seeds harvested at days 17-19 post-anthesis. RNA is extracted in 25 mls of 4 M guanidine thiocyanate buffer as described by Colbert *et al.* (*PNAS* (1983) *80*:2248-2252). Polysaccharides are removed from the RNA sample by resuspending the pellet in 6 ml of 1X TE (10 mM Tris/1 mM EDTA pH 8), adding potassium acetate to a concentration of 0.05M, and adding one half volume of ethanol. The sample is placed on ice for 60 minutes and centrifuged for 10 minutes at 3000 x g. RNA is precipitated from the supernatant by adding sodium acetate to a concentration of 0.3 M followed by the addition of two volumes of ethanol. RNA is recovered from the sample by centrifugation at 12,000 x g for 10 minutes and yield calculated by UV spectrophotometry. Two mg of the total RNA is further purified by removing polysaccharides on a 0.25 g Sigma Cell 50 cellulose column, as described above, and is also enriched for poly(A)+ RNA by oligo(dT)-cellulose chromatography as described above.

A *B. campestris* day 17-19 post anthesis embryo cDNA library is constructed in plasmid vector pCGN1703 as described in Example 5, using 5 ug of the above described poly(A)+ RNA. The library, which consists of approximately 1.5 x $10^5$ transformants, is amplified by plating and scraping colonies, and is stored as frozen *E. coli* cells in 10% DMSO at -80° C. DNA is isolated from a portion of the amplified library by scaling up the alkaline lysis technique of Birnboim and Doly (*Nucleic Acids Res.* (1979) *7*:1513), and purified by CsCl centrifugation. Library DNA is digested with *Eco*RI and is cloned into *Eco*RI-digested bacteriophage lambda gt10 (Stratagene; La Jolla, CA) DNA. The DNA is packaged using Gigapack II Gold *in vitro* packaging extracts (Stratagene; La Jolla, CA) according to manufacturer's specifications. The titer of the phage stock, determined by dilution plating of phage in *E. coli* C600 hfl- cells (Huynh, *et al., DNA Cloning. Volume 1.* Eds. Gover, D.M. (1985) IRL Press Limited: Oxford, England, pp. 56,110), is 6 x $10^6$ pfu per ml.

*B. campestris cDNA Library Screen:* The library is plated on *E. coli* strain C600 hfl- at a density of approximately 30,000 pfu/150mm NZY plate to provide approximately 120,000 plaques for screening. Phage are lifted in duplicate on to NEN (Boston, MA.) Colony/Plaque Screen filters as described in Example 5. Filters are prehybridized and hybridized with the $^{32}$P-labeled fragment of pCGN3230 as described above for the Northern hybridization. Filters are washed for 30 minutes in 2X SSC, 0.1% SDS at 50°C and at 55°C twice for 15 minutes each. Filters are exposed to X-ray film overnight at -80°C with a Dupont Cronex intensifying screen.

Clones are detected by hybridization on duplicate filters to the *R. communis* desaturase cDNA fragment and plaque purified. During plaque purification, the probe used was a gel-purified 1.4 kb SstI fragment of pCGN3230 which lacks the poly(A)+ tail. As described above, phage DNA is isolated from purified lambda clones, digested with EcoRI, ligated, and transformed to *E. coli* DH5α cells. Minipreparation DNA is prepared and partial DNA sequence determined as described above. Partial DNA sequences of two clones, pCGN3235 and pCGN3236, are presented in Figure 4A (SEQ ID NO: 17) and 4B (SEQ ID NO: 18), respectively. Initial DNA sequence analysis of the 3' regions of these clones indicates that pCGN3236 and pCGN3235 are cDNA clones from the same gene. pCGN3236 is a shorter clone than pCGN3235, which appears to contain the entire coding region of the *B. campestris* desaturase gene. The complete nucleotide sequence of pCGN3235 is presented in Figure 4C and SEQ ID NO: 19.

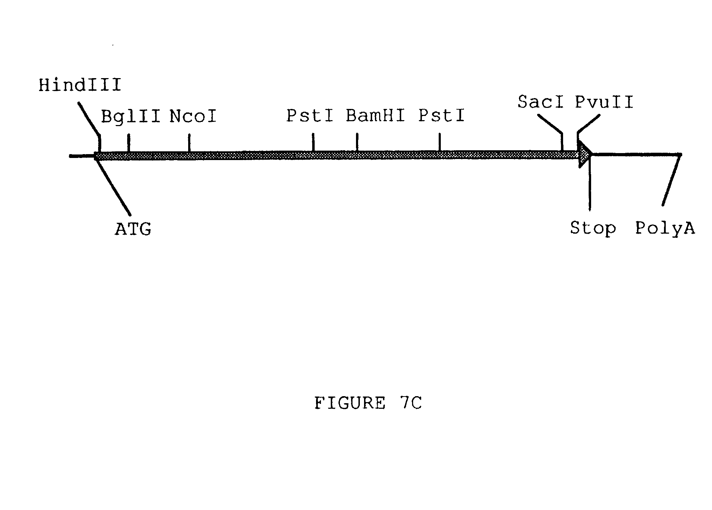

*Desaturase Gene Analysis*: Southern and Northern analyses of Brassica species are conducted to determine the number of genes which encode the Brassica desaturase clone, pCGN3235 in *B. campestris, B. oleracea,* and *B. napus*, and the timing of expression of the gene in *B. campestris* developing seeds. DNA is isolated from leaves of each of the above-named *Brassica* species by the method of Bernatzky and Tanksley (*Theor. Appl. Genet.* (1986) *72*: 314-321). DNA from each of the species is digested with restriction endonucleases EcoRI and XbaI (10 ug/digest), electrophoresed in a 0.7% agarose gel, and transferred to a nitrocellulose filter (Maniatis *et al., supra*). The filter is prehybridized and hybridized at 42°C (as described above for Northern analysis using *R. communis* desaturase clone) with a $^{32}$P-labeled (nick translation) gel-isolated HindIII/PvuII fragment of pCGN3235 (Fig. 7C). The filter is washed following overnight hybridization, for 30 minutes at 55°C in 1X SSC, 0.1% SDS, followed by two 15 minute washed at 55°C in 0.1X SSC, 0.1% SDS.

The autoradiograph indicates that the *Brassica* desaturase is encoded by a small gene family consisting of about two genes in *B. campestris* and *B. oleracea*, and about four genes in *B. napus.*

The timing of expression of the desaturase gene during seed development is determined by Northern analysis. RNA is isolated from immature seeds of *B. campestris* cv. R500 collected at 11, 13, 15, 17, 19, 21, 25, 30, 35, and 40 days post-anthesis. Total RNA is isolated as described by Scherer and Knauf (*Plant Mol. Biol.* (1987) *9*:127-134). Twenty five micrograms of RNA from each time point are electrophoresed through a formaldehyde-containing 1.5% agarose gel as described by Shewmaker, *et al.* (*supra*) and blotted to nitrocellulose (Thomas, *Proc. Nat. Acad. Sci.* (1980) *77*:5201-5205). The blot is pre-hybridized and hybridized at 42°C with the $^{32}$P-labeled HindIII/PvuII fragment of pCGN3235 as described above. The filter is washed following overnight hybridization, for 30 minutes at 55°C in 1X SSC, 0.1% SDS, followed by two 15 minute washed at 55°C in 0.1X SSC, 0.1% SDS.

The autoradiograph indicates that the desaturase gene is expressed in *B. campestris* developing seeds beginning at about day 19 and through about day 30, with maximal expression at day 25. By a similar Northern analysis, the level of desaturase mRNA in developing *Brassica napus* seeds (day 21) was estimated to be approximately 1% of the total mRNA.

*Isolation of Other Desaturase Gene Sequences:* cDNA libraries may be constructed as described above and genomic libraries can be constructed from DNA from various sources using commercially available vectors and published DNA isolation, fractionation, and cloning procedures. For example, a *B. campestris* genomic library can be constructed using DNA isolated according to Scofield and Crouch (J.Biol.Chem. (1987) 262:12202-12208) that is digested with BamHI and fractionated on sucrose gradients (Maniatis *et al., supra*), and cloned into the lambda phage vector LambdaGem-11 (Promega; Madison, WI) using cloning procedures of Maniatis *et al.* (*supra*).

cDNA and genomic libraries can be screened for desaturase cDNA and genomic clones, respectively, using published hybridization techniques. Screening techniques are described above for screening libraries with DNA fragments. Libraries may also be screened with synthetic oligonucleotides, for example using methods described by Berent *et al.* (*BioTechniques* (1985) *3*:208-220). Probes for the library screening can be prepared by PCR, or from the sequences of the desaturase clones provided herein. Oligonucleotides prepared from the desaturase sequences may be used, as well as longer DNA fragments, up to the entire desaturase clone.

For example, jojoba polyadenylated RNA is used to construct a cDNA library in the cloning vector λZAPII/*Eco*RI (Stratagene, San Diego, CA). RNA is isolated from jojoba embryos collected at 80-90 days post-anthesis by isolating polyribosomes using a method initially described by Jackson and Larkins (*Plant Physiol.* (1976) *57*:5-10) and modified by Goldberg *et al.* (*Developmental Biol.* (1981) *83*:201-217). Polysaccharide contaminants in the polyribosomal RNA preparation are removed by running the RNA over a cellulose column (Sigma-cell 50) in high salt buffer (0.5M NaCl, 20mM Tris pH 7.5, 1mM EDTA, 0.1% SDS). The contaminant binds to the column and the RNA is collected in the eluant. The eluant fractions are pooled and the RNA is ethanol precipitated. The precipitated total RNA is then resuspended in a smaller volume and applied to an oligo d(T) cellulose column to isolate the polyadenylated RNA.

The library is constructed using protocols, DNA and bacterial strains as supplied by the manufacturer. Clones are packaged using Gigapack Gold packaging extracts (Stratagene), also according to manufacturer's recommendations. The cDNA library constructed in this manner contins approximately 1 x $10^6$ clones with an average cDNA insert size of approximately 400 base pairs.

The jojoba library is plated on *E. coli* XL1-Blue (Stratagene) at a density of approximately 5000pfu/150mm plate to provide approximately 60,000 plaques for screening. Phage are lifted onto duplicate nylon membrane filters as described previously. Filters are prehybridized at 42°C in a hybridization buffer containing 40% formamide, 10X Denhardt's solution, 5X SSC, 0.1% SDS, 50mM EDTA, and 100μg/ml denatured salmon sperm DNA. Hybridization is at 42°C in the same buffer with added nick translated (BRL Nick Translation System) 520 bp *Bgl*II fragment of the *C. tinctorius* desaturase clone described previously. Filters are washed at 50°C in 2X SSC and exposed to X-ray film overnight.

Desaturase clones are detected by hybridization on duplicate filters with the *C. tinctorius* cDNA fragment and plaque-purified. Positive clones are recovered as plasmids in *E. coli* following manufacturer's directions and materials for *in vivo* excision. Partial, preliminary DNA sequence of a clone, 3-1, is determined and the corresponding amino acid sequence is translated in three frames. In this manner, homology to the *C. tinctorius* desaturase cDNA clone is detected in one reading frame. The preliminary DNA sequence of this jojoba desaturase cDNA fragment is shown in Figure 5 (SEQ ID NO: 43). Also shown is the corresponding translated amino acid sequence in the reading frame having *C. tinctorius* desaturase homology. The jojoba cDNA fragment is approximately 75% homologous at the DNA level and approximately 79% homologous at the amino acid level compared to sequence of the *C. tinctorius* desaturase in this region.

## Example 13

Antisense constructs are described which allow for transcription of a reverse copy of the *B. campestris* desaturase cDNA clone in the 5' to 3' orientation of transcription.

*Preferential Expression of Antisense Constructs in Embryos*

In order to reduce the transcription of a desaturase gene in embryos of *B. napus* or *B. campestris*, constructs may be prepared which allow for production of antisense copies of the desaturase cDNA preferentially in the embryos. Promoter sequences which are desirable to obtain this pattern of expression include, but are not limited to, the ACP, Bce4, and napin 1-2 expression cassettes described in Examples 7, 8, and 9, respectively. It also may be desirable to control the expression of reverse copies of the desaturase cDNA under two different promoters in the same transformed plant to provide for a broader timing of expression of the antisense desaturase DNA. For example, expression from the ACP promoter may begin and end earlier than expression from the napin promoter. Thus, expressing the reverse desaturase from both promoters may result in the production of the antisense strand of DNA over a longer period of embryo development.

An example of expression of an antisense desaturase gene preferentially in the embryos is provided below. Similar constructs containing the same or a different fragment of the desaturase gene and any of the promoters described above, as well as other promoter regions which may be useful, may also be prepared using gene cloning, insertion, mutation and repair techniques well known to those of ordinary skill in the art.

A. Antisense Desaturase Expression from the ACP Promoter

Construction of pCGN3239 is as follows:

pCGN3235 (Example 12) is digested with *Pvu*II and *Hin*dIII and the *Hin*dIII sticky ends are filled in with Klenow in the presence of 200 µM dNTPs. The 1.2 kb *Pvu*II/*Hin*dIII fragment containing the desaturase coding sequence is gel purified and ligated in the antisense orientation into *Eco*RV-digested pCGN1977 (ACP expression cassette; described in Example 7) to create pCGN3238. The 4.2 kb *Xba*I/*Asp*718 fragment of pCGN3238 containing the antisense desaturase in the ACP cassette is transferred into *Xba*I/*Asp*718-digested pCGN1557 (binary transformation vector; described in Example 7) to create pCGN3239.

B. Antisense Desaturase Expression From The Napin Promoter

Construction of pCGN3240 is as follows: pCGN3235 is digested with *Pvu*II and *Hin*dIII, the sticky ends are blunted, and the resulting fragment is inserted in an anti-sense orientation into pCGN3223 which has been digested with *Sal*I and blunted with Klenow enzyme. The resulting plasmid, pCGN3240 will express an anti-sense desaturase RNA from the napin promoter cassette.

C. Antisense Desaturase Expression From a Dual Promoter Cassette

Construction of pCGN3242 is as follows: An *Asp*718 fragment of pCGN3240 containing the napin 5' and 3' regions surrounding the desaturase sequences is inserted into the *Asp*718 site of pCGN3239 (a binary vector containing an ACP promoter, antisense desaturase construct) to create pCGN3242.

*Constitutive Transcription*

A. Binary Vector Construction

1. Construction of pCGP291.

The *Kpn*I, *Bam*HI, and *Xba*I sites of binary vector pCGN1559 (McBride and Summerfelt, *Pl.Mol.Biol.* (1990) *14:* 269-276) are removed by *Asp*718/*Xba*I digestion followed by blunting the ends and recircularization to produce pCGP67. The 1.84 kb *Pst*I/*Hin*dIII fragment of pCGN986 containing the 35S promoter-tml3' cassette is inserted into *Pst*I/*Hin*dIII digested pCGP67 to produce pCGP291.

2. Construction of pCGN986.

The 35S promoter-tml3' expression cassette, pCGN986, contains a cauliflower mosaic virus 35S (CaMV35) promoter and a T-DNA tml 3'-region with multiple restriction sites between them. pCGN986 is derived from another cassette, pCGN206, containing a CaMV35S promoter and a different 3' region, the CaMV region VI 3'-end. The CaMV 35S promoter is cloned as an *Alu*I fragment (bp 7144-7734) (Gardner *et. al., Nucl.Acids Res.* (1981) *9*:2871-2888) into the *Hinc*II site of M13mp7 (Messing, *et. al., Nucl.Acids Res.* (1981) *9*:309-321) to create C614. An *Eco*RI digest of C614 produced the *Eco*RI fragment from C614 containing the 35S promoter which is cloned into the *Eco*RI site of pUC8 (Vieira and Messing, *Gene* (1982) *19*:259) to produce pCGN147. pCGN148a containing a promoter region, selectable marker (KAN with 2 ATG's) and 3' region, is prepared by digesting pCGN528 with *Bgl*II and inserting the *Bam*HI-*Bgl*II promoter fragment from pCGN147. This fragment is cloned into the *Bgl*II site of pCGN528 so that the *Bgl*II site is proximal to the kanamycin gene of pCGN528.

The shuttle vector used for this construct, pCGN528, is made as follows: pCGN525 is made by digesting a plasmid containing Tn5 which harbors a kanamycin gene (Jorgenson *et. al., Mol. Gen. Genet.* (1979) *177*:65) with *Hin*dIII-*Bam*HI and inserting the *Hin*dIII-*Bam*HI fragment containing the kanamycin gene into the *Hind*III-*Bam*HI sites in the tetracycline gene of pACYC184 (Chang and Cohen, *J. Bacteriol.* (1978) *134*:1141-1156). pCGN526 was made by inserting the *Bam*HI fragment 19 of pTiA6 (Thomashow *et. al., Cell* (1980) *19*:729-739), modified with *Xho*I linkers inserted into the *Sma*I site, into the *Bam*HI site of pCGN525. pCGN528 is obtained by deleting the small *Xho*I fragment from pCGN526 by digesting with *Xho*I and religating.

pCGN149a is made by cloning the *Bam*HI-kanamycin gene fragment from pMB9KanXXI into the *Bam*HI site of pCGN148a. pMB9KanXXI is a pUC4K variant (Vieira and Messing, *Gene* (1982) *19*:259-268 which has the *Xho*I site missing, but contains a functional kanamycin gene from Tn903 to allow for efficient selection in *Agrobacterium.*

pCGN149a is digested with *Hin*dIII and *Bam*HI and ligated to pUC8 digested with *Hin*dIII and *Bam*HI to produce pCGN169. This removes the Tn903 kanamycin marker. pCGN565 and pCGN169 are both digested with *Hin*dIII and *Pst*I and ligated to form pCGN203, a plasmid containing the CaMV 35S promoter and part of the 5'-end of the Tn5 kanamycin gene (up to the *Pst*I site, Jorgenson *et. al.*, (1979), *supra*). A 3'-regulatory region is added to pCGN203 from pCGN204, an *Eco*RI fragment of CaMV (bp 408-6105) containing the region VI 3' cloned into pUC18 (Yanisch-Perron, *et al*., *Gene* (1985) *33*:103-119) by digestion with *Hin*dIII and *Pst*I and ligation. The resulting cassette, pCGN206, is the basis for the construction of pCGN986.

The pTiA6 T-DNA tml 3'-sequences are subcloned from the *Bam*19 T-DNA fragment (Thomashow *et al*., (1980) *supra*) as a *Bam*HI-*Eco*RI fragment (nucleotides 9062 to 12,823, numbering as in Barker *et al., Plant Mol. Biol.* (1982) *2*:335-350) and combined with the pACYC184 (Chang and Cohen (1978), *supra*) origin of replication as an *Eco*RI-*Hin*dIII fragment and a gentamycin resistance marker (from plasmid pLB41), obtained from D. Figurski) as a *Bam*HI-*Hin*dIII fragment to produce pCGN417.

The unique *Sma*I site of pCGN417 (nucleotide 11,207 of the *Bam*19 fragment) is changed to a *Sac*I site using linkers and the *Bam*HI-*Sac*I fragment is subcloned into pCGN565 to give pCGN971. The *Bam*HI site of pCGN971 is changed to an *Eco*RI site using linkers. The resulting *Eco*RI-*Sac*I fragment containing the tml 3' regulatory sequences is joined to pCGN206 by digestion with *Eco*RI and *Sac*I to give pCGN975. The small part of the Tn5 kanamycin resistance gene is deleted from the 3'-end of the CaMV 35S promoter by digestion with *Sal*I and *Bgl*II, blunting the ends and ligation with *Sal*I linkers. The final expression cassette pCGN986 contains the CaMV 35S promoter followed by two *Sal*I sites, an *Xba*I site, *Bam*HI, *Sma*I, *Kpn*I and the tml 3' region (nucleotides 11207-9023 of the T-DNA).

B. <u>Insertion of Desaturase Sequence</u>

The 1.6 kb *XbaI* fragment from pCGN3235 containing the desaturase cDNA is inserted in the antisense orientation into the *Xba*I site of pCGP291 to produce pCGN3234.

*Plant Transformation*

The binary vectors containing the expression cassette and the desaturase gene are transformed into *Agrobacterium tumefaciens* strain EHA101 (Hood, *et al., J. Bacteriol.* (1986) *168*:1291-1301) as per the method of Holsters, *et al., Mol. Gen. Genet.* (1978) *163*:181-187. Transformed *B. napus* and/or *Brassica campestris* plants are obtained as described in Example 10.

*Analysis of Transgenic Plants*

A. <u>Analysis of pCGN3242 Transformed *Brassica campestris* cv. Tobin Plants</u>

Due to the self-incompatibility of *Brassica campestris* cv. Tobin, individual transgenic plants are pollinated using non-transformed Tobin pollen. Because of this, the T2 seeds of a transgenic plant containing the antisense desaturase at one locus would be expected to segregate in a 1:1 ratio of transformed to non-transformed seed. The oil composition of ten individual seeds collected at 26 days post-anthesis from several pCGN3242-transformed plants and one non-transformed control was analyzed by gas chromatography according to the method of Browse, *et al., Anal. Biochem.* (1986) *152*:141-145. One transformant, 3242-T-1, exhibits an oil composition that differed distinctly from controls on preliminary analysis. The control Tobin seeds contained an average of 1.8% 18:0 (range 1.5% - 2.0%) and 52.9% 18:1 (range 48.2% - 57.1%). T2 seeds of 3242-T-1 segregated into two distinct classes. Five seeds contained levels of 18:0 ranging from 1.3% to 1.9% and levels of 18:1 ranging from 42.2% to 58.3%. The other five seeds contained from 22.9% to 26.3% 18:0 and from 19.9% to 26.1% 18:1.

B. <u>Analysis of pCGN3234 Transformed Plants</u>

Some abnormalities have been observed in some transgenic *Brassica napus* cv. Delta and Bingo and *Brassica campestris* cv. Tobin plants containing pCGN3234. These effects could be due to the constitutive expression of antisense desaturase RNA from the 35S promoter or could be due to the transformation/tissue culture regime the plants have been subjected to.

The above results demonstrate the ability to obtain plant Δ-9 desaturases, isolate DNA sequences which encode desaturase activity and manipulate them. In this way, the production of transcription cassettes, including expression cassettes can be produced which allow for production, including specially differentiated cell production of the desired product. A purified *C. tinctorius* desaturase is provided and used to obtain nucleic acid sequences of *C. tinctorius* desaturase. Other plant desaturase sequences are provided such as *R. cummunis, B. campestris*, and *S. chinensis.* These sequences as well as desaturase sequences obtained from them may be used to obtain additional desaturease, and so on. And, as described in the application modification of oil composition may be achieved.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claim.

## Claims

1. An isolated DNA having at least 60% homology to the Δ-9 desaturase DNA coding sequence of Figure 2 and comprising a sequence encoding a Δ-9 desaturase or a portion thereof having Δ-9 desaturase activity; which Δ-9 desaturase is capable of catalysing the insertion of a first double bond into a fatty acyl-ACP moiety, said double bond being inserted at the ninth carbon position.

2. A DNA according to claim 1 wherein the Δ-9 desaturase is further **characterised by** comprising a sequence of amino acids as shown in one or more of sequences F1 to F11 in Figure 1.

3. A DNA according to claim 1 or 2 wherein the sequence encodes a precursor desaturase or a mature desaturase.

4. A DNA according to claim 1 or 2 which is obtainable from C. tinctorius, R. communis or B. campestris.

5. A DNA construct comprising, in the 5' to 3' direction of transcription, a transcriptional regulatory region functional in a host cell and a DNA according to claim 1 or 2.

6. A construct according to claim 5 further comprising a translational regulatory region immediately 5' to said DNA and a transcriptional/translational termination regulatory region 3' to said DNA, wherein said regulatory regions are functional in a host cell.

7. A construct according to claim 6 wherein the DNA is joined to a nucleic acid sequence which is not naturally joined to the DNA.

8. A construct according to any one of claims 5 to 7 wherein the DNA is a sense sequence.

9. A construct according to any one of claims 5 to 8 wherein the host cell is a plant cell.

10. A construct according to any one of claims 5 to 9 wherein the regulatory elements function preferentially in a plant seed.

11. A construct according to any one of claims 7 to 10 wherein the regulatory elements function in a seed during lipid accumulation.

12. A construct according to claim 7 wherein the host cell is a plant cell.

13. A construct according to claim 7 or 12 wherein the regulatory elements function preferentially in a plant seed.

14. A construct according to claim 7, 12 or 13 wherein the regulatory elements function in a seed during lipid accumulation.

15. A host cell comprising a construct as defined in any one of claims 7 or 12 to 14.

16. A cell according to claim 15 which is a plant cell.

17. A cell according to claim 15 or 16 having, integrated into its genome, a construct as defined in any one of claims 7 or 12 to 14.

**18.** A plant comprising a cell according to claim 16 or 17.

**19.** A plant seed comprising a cell according to claim 16 or 17.

**20.** A method of producing a triglyceride oil from a plant seed comprising growing a plant according to claim 18 to seed, harvesting the seed and recovering said oil therefrom, which method results in modification of the fatty acid composition in the cells of the plant.

**21.** A method of producing a triglyceride oil from a plant seed comprising regenerating a plant cell according to claim 16 or 17, growing the thus obtained plant to seed and recovering said oil therefrom, which method results in modification of the fatty acid composition in the cells of the plant.

**22.** A method according to claim 20 or 21 wherein overexpression of the plant desaturase is obtained.

**23.** A method according to claim 20 or 21 wherein a decrease in endogenous plant desaturase is obtained.

**24.** A method according to any one of claim 20 or 21 wherein the percentage of long chain unsaturated fatty acids is increased.

**25.** A method according to any one of claims 20 to 24 wherein the plant is rapeseed, sunflower, C. tinctorius, Cuphea, peanut, soybean, oil palm or corn.

**26.** A method according to claim 25 wherein the oil comprises at least 10% stearate.

**27.** A method according to claim 26 wherein the oil comprises at least 25% stearate.

**28.** A method according to any one of claims 20 to 27 wherein the DNA sequence encoding the plant desaturase is integrated into the genome of the embryo cells of the plant.

**29.** A method according to any one of claims 20 to 28 wherein the plant comprises a construct according to claim 13 or 14.

**30.** A method of modifying fatty acid composition in a host cell from a given percentage of fatty acid saturation to a different percentage of fatty acid saturation, which method comprises growing a plant cell according to claim 16 or 17 under conditions that promote the activity of the regulatory elements defined in any one of claims 4 to 7.

**31.** A method of producing a plant desaturase, which method comprises growing a cell according to any one of claims 15 to 17 or a plant or plant seed according to claim 18 or 19 under conditions which permit the production of the plant desaturase.

**Patentansprüche**

**1.** Isolierte DNA mit mindestens 60%-iger Homologie bezüglich der Δ-9 Desaturase DNA-kodierenden Sequenz der Figur 2, und umfassend eine Sequenz, die für eine Δ-9 Desaturase oder einen Teil hiervon mit einer Δ-9 Desaturaseaktivität kodiert; wobei die Δ-9 Desaturase fähig ist, die Insertion einer ersten Doppelbindung in eine Fettacyl-ACP-Einheit zu katalysieren, wobei die Doppelbindung an der Position des neunten Kohlenstoffs insertiert wird.

**2.** DNA nach Anspruch 1, wobei die Δ-9 Desaturase weiterhin **dadurch gekennzeichnet ist. dass** sie eine Sequenz von Aminosäuren umfaßt, wie in einer oder mehreren der Sequenzen F1 bis F11 in Figur 1 gezeigt.

**3.** DNA nach Anspruch 1 oder 2, wobei die Sequenz für eine Vorläuferdesaturase oder eine Ausreifungsdesaturase kodiert.

**4.** DNA nach Anspruch 1 oder 2, welche erhältlich ist aus C. tinctorius, R. communis oder B. campestris.

**5.** DNA-Konstrukt, umfassend in der 5'- bis 3'-Richtung der Transkription eine transkriptionale, regulatorische Region, welche in einer Wirtszelle funktional ist, und eine DNA nach Anspruch 1 oder 2.

6. Konstrukt nach Anspruch 5, umfassend weiterhin eine translatorische, regulatorische Region unmittelbar 5' zu der DNA und eine transkriptionale/translatorische terminationsregulatorische Region 3' zu der DNA, wobei die regulatorischen Regionen in einer Wirtszelle funktional sind.

7. Konstrukt nach Anspruch 6, wobei die DNA an eine Nukleinsäuresequenz gekoppelt ist, welche nicht von Natur aus an die DNA gekoppelt ist.

8. Konstrukt nach mindestens einem der Ansprüche 5 bis 7, wobei die DNA eine Orientierungssequenz ist.

9. Konstrukt nach mindestens einem der Ansprüche 5 bis 8, wobei die Wirtszelle eine Pflanzenzelle ist.

10. Konstrukt nach mindestens einem der Ansprüche 5 bis 9, wobei die regulatorischen Elemente bevorzugt in einem Pflanzensamen wirken.

11. Konstrukt nach mindestens einem der Ansprüche 7 bis 10, wobei die regulatorischen Elemente in einem Samen während der Lipidakkumulation wirken.

12. Konstrukt nach Anspruch 7, wobei die Wirtszelle eine Pflanzenzelle ist.

13. Konstrukt nach Anspruch 7 oder 12, wobei die regulatorischen Elemente vorzugsweise in einem Pflanzensamen wirken.

14. Konstrukt nach Anspruch 7, 12 oder 13, wobei die regulatorischen Elemente in einem Samen während der Lipidakkumulation wirken.

15. Wirtszelle, umfassend ein Konstrukt nach mindestens einem der Ansprüche 7 oder 12 bis 14.

16. Zelle nach Anspruch 15, welche eine Pflanzenzelle ist.

17. Zelle nach Anspruch 15 oder 16, welche integriert in ihrem Genom ein Konstrukt nach mindestens einem der Ansprüche 7 oder 12 bis 14 aufweist.

18. Pflanze, umfassend eine Zelle nach Anspruch 16 oder 17.

19. Pflanzensamen, umfassend eine Zelle nach Anspruch 16 oder 17.

20. Verfahren zur Herstellung eines Triglyceridöls aus einem Pflanzensamen, umfassend das Züchten einer Pflanze nach Anspruch 18 bis zum Samen, Ernten des Samens und Rückgewinnen des Öls hieraus, welches Verfahren in einer Modifizierung der Fettsäurezusammensetzung in den Zellen der Pflanze resultiert.

21. Verfahren zur Herstellung eines Triglyceridöls aus einem Pflanzensamen, umfassend das Regenerieren einer Pflanzenzelle nach Anspruch 16 oder 17, Züchten der so erhaltenen Pflanze bis zum Samen und Gewinnen des Öls hieraus, welches Verfahren in einer Modifizierung der Fettsäurezusammensetzung in den Zellen der Pflanze resultiert.

22. Verfahren nach Anspruch 20 oder 21, wobei eine Überexpression der Pflanzendesaturase erhalten wird.

23. Verfahren nach Anspruch 20 oder 21, wobei eine Abnahme der endogenen Pflanzendesaturase erhalten wird.

24. Verfahren nach Anspruch 20 und/oder 21, wobei der Prozentgehalt an langkettigen, ungesättigten Fettsäuren erhöht wird.

25. Verfahren nach mindestens einem der Ansprüche 20 bis 24, wobei die Pflanze Rapssamen, Sonnenblume, C. tinctorius, Cuphea, Erdnuss, Sojabohne, Ölpalme oder Mais ist.

26. Verfahren nach Anspruch 25, wobei das Öl mindestens 10% Stearat umfaßt.

27. Verfahren nach Anspruch 26, wobei das Öl mindestens 25% Stearat umfaßt.

**28.** Verfahren nach mindestens einem der Ansprüche 20 bis 27, wobei die DNA-Sequenz, welche für die Pflanzendesaturase kodiert, in das Genom der Embryozellen der Pflanze integriert ist.

**29.** Verfahren nach mindestens einem der Ansprüche 20 bis 28, wobei die pflanze ein Konstrukt nach Anspruch 13 oder 14 umfaßt.

**30.** Verfahren zum Modifizieren einer Fettsäurezusammensetzung in einer Wirtszelle von einem gegebenen Prozentgehalt an Fettsäuresättigung zu einem verschiedenen Prozentgehalt an Fettsäuresättigung, welches Verfahren das Züchten einer Pflanzenzelle nach Anspruch 16 oder 17 unter Bedingungen umfaßt, welche die Aktivität der regulatorischen Elemente, wie in mindestens einem der Ansprüche 4 bis 7 definiert, fördern.

**31.** Verfahren zur Herstellung einer Pflanzendesaturase, welches Verfahren das Züchten einer Pflanze nach mindestens einem der Ansprüche 15 bis 17 oder einer Pflanze oder eines Pflanzensamens nach Anspruch 18 oder 19 unter Bedingungen umfaßt, welche die Produktion der Pflanzendesaturase ermöglichen.

## Revendications

**1.** ADN isolé présentant au moins 60 % d'homologie par rapport à la séquence codant pour l'ADN de la désaturase Δ-9 de la figure 2 et comprenant une séquence codant pour la désaturase Δ-9 ou une partie de celle-ci présentant une activité de désaturase Δ-9, laquelle désaturase Δ-9 est capable de catalyser l'insertion d'une première double liaison dans une fraction acyle gras-protéine ACP, ladite double liaison étant insérée au niveau de la neuvième position de carbone.

**2.** ADN selon la revendication 1, dans lequel la désaturase Δ-9 est en outre **caractérisée en ce qu'**elle comprend une séquence d'acides animés, comme indiqué dans une ou plusieurs des séquences F1 à F11 sur la figure 1.

**3.** ADN selon la revendication 1 ou 2, dans lequel la séquence code pour une désaturase précurseur ou une désaturase mature.

**4.** ADN selon la revendication 1 ou 2, lequel peut être obtenu à partir de C. tinctorius, R. communis ou B. campestris.

**5.** Produit de synthèse d'ADN comprenant, dans la direction 5' vers 3' de la transcription, une région régulatrice transcriptionelle fonctionnelle dans une cellule hôte et un ADN selon la revendication 1 ou 2.

**6.** Produit de synthèse selon la revendication 5, comprenant en outre une région régulatrice translationnelle immédiatement en 5' par rapport audit ADN et une région régulatrice de terminaison transcriptionelle/translationnelle 3' par rapport audit ADN, dans lequel lesdites régions régulatrices sont fonctionnelles dans une cellule hôte.

**7.** Produit de synthèse selon la revendication 6, dans lequel l'ADN est réuni à une séquence d'acides nucléiques qui n'est pas naturellement réunie à l'ADN.

**8.** Produit de synthèse selon l'une quelconque des revendications 5 à 7, dans lequel l'ADN est une séquence sens.

**9.** Produit de synthèse selon l'une quelconque des revendications 5 à 8, dans lequel la cellule hôte est une cellule végétale.

**10.** Produit de synthèse selon l'une quelconque des revendications 5 à 9, dans lequel les éléments régulateurs agissent de préférence dans une graine de plante.

**11.** Produit de synthèse selon l'une quelconque des revendications 7 à 10, dans lequel les éléments régulateurs agissent dans une graine durant l'accumulation des lipides.

**12.** Produit de synthèse selon la revendication 7, dans lequel la cellule hôte est une cellule végétale.

**13.** Produit de synthèse selon la revendication 7 ou 12, dans lequel les éléments régulateurs agissent de préférence dans une graine de plante.

**14.** Produit de synthèse selon la revendication 7, 12 ou 13, dans lequel les éléments régulateurs agissent dans une graine durant l'accumulation des lipides.

**15.** Cellule hôte comprenant un produit de synthèse tel que défini dans l'une quelconque des revendications 7 ou 12 à 14.

**16.** Cellule selon la revendication 15, laquelle est une cellule végétale.

**17.** Cellule selon la revendication 15 ou 16 comportant, intégrée dans son génome, un produit de synthèse tel que défini dans l'une quelconque des revendications 7 ou 12 à 14.

**18.** Plante comprenant une cellule selon la revendication 16 ou 17.

**19.** Graine de plante comprenant une cellule selon la revendication 16 ou 17.

**20.** Procédé de production d'une huile de triglycérides à partir d'une graine de plante comprenant la croissance d'une plante selon la revendication 18 jusqu'à la graine, la récolte de la graine et la récupération de ladite huile depuis celle-ci, lequel procédé résulte en une modification de la composition en acides gras dans les cellules de la plante.

**21.** Procédé de production d'une huile de triglycérides à partir d'une graine de plante comprenant la régénération d'une cellule végétale selon la revendication 16 ou 17, la croissance de la plante ainsi obtenue jusqu'à la graine et la récupération de ladite huile à partir de celle-ci, lequel procédé résulte en une modification de la composition en acides gras dans les cellules de la plante.

**22.** Procédé selon la revendication 20 ou 21, dans lequel une surexpression de la désaturase végétale est obtenue.

**23.** Procédé selon la revendication 20 ou 21, dans lequel une diminution de la désaturase végétale endogène est obtenue.

**24.** Procédé selon l'une quelconque des revendications 20 ou 21, dans lequel le pourcentage d'acides gras insaturés à longue chaîne est augmenté.

**25.** Procédé selon l'une quelconque des revendications 20 à 24, dans lequel la plante est le colza, le tournesol, C. tinctorius, Cuphea, l'arachide, le soja, le palmier à huile ou le maïs.

**26.** Procédé selon la revendication 25, dans lequel l'huile comprend au moins 10 % de stéarate.

**27.** Procédé selon la revendication 26, dans lequel l'huile comprend au moins 25 % de stéarate.

**28.** Procédé selon l'une quelconque des revendications 20 à 27, dans lequel la séquence d'ADN codant pour la désaturase végétale est intégrée dans le génome des cellules embryonnaires de la plante.

**29.** Procédé selon l'une quelconque des revendications 20 à 28, dans lequel la plante comprend un produit de synthèse selon la revendication 13 ou 14.

**30.** Procédé de modification de la composition en acides gras dans une cellule hôte d'un pourcentage donné de saturation des acides gras à un pourcentage différent de saturation des acides gras, lequel procédé comprend la croissance d'une cellule végétale selon la revendication 16 ou 17 dans des conditions qui favorisent l'activité des éléments régulateurs définis dans l'une quelconque des revendications 4 à 7.

**31.** Procédé de production d'une désaturase végétale, lequel procédé comprend la croissance d'une cellule selon l'une quelconque des revendications 15 à 17 ou d'une plante ou d'une graine de plante selon la revendication 18 ou 19 dans des conditions qui permettent la production de la désaturase végétale.

F1: ASTLGSSTPKVDNAKKPFQPPREVHVQVTH$^{S}_{X}$MPPQKIEIFKSIEG$^{W}_{R}$AEQNILV$^{H}_{F}$LKPVEKCWQ

F2: DFLPDPA$^{S}_{T}$EGFDEQVKELRARAKEIPDDYFVVLVGDMITEEALPTYQTMLNTLDGV

F3: DETGASLTPWAVWT

F4: DLLHTYLYLSGRV

F5: DMRQIQKTIQYLI

F6: TENSPYLGFIYTSFQER

F7: DV$^{K}_{F}$LAQI$^{C}_{Q}$GTIASDEKRHETAYTKIVEKLFEIDPDGTVLAFADMMRKKI$^{S}_{T}$MPAHLMY

F8: DNLF

F9: dvFlAV$^{A}_{I}$QRL$^{G}_{I}$VYTAK

F10: DYADILEFLVGRWK

F11: VADLTGLSGEGRKA$^{Q}_{G}$DYVCGLPPRIRRLEERAQGRAKEGPVVPFSWIFDRQVKL

FIGURE 1

```
                                             HindIII
                                             |
  1 GCTCACTTGTGTGGTGGAGGAGAAAAACAGAACTCACAAAAAGCTTTGCGACTGCCAAGAACAACAACA   69
                                             42

 70 ACAACAAGATCAAGAAGAAGAAGAAGAAGATCAAAAATGGCTCTTCGAATCACTCCAGTGACCTTGCAA  138
                                        METAlaLeuArgIleThrProValThrLeuGln

              EcoRV                                   BglII            NcoI
              |                                       |                |
139 TCGGAGAGATATCGTTCGTTTTCGTTTCCTAAGAAGGCTAATCTCAGATCTCCCAAATTCGCCATGGCC  207
    SerGluArgTyrArgSerPheSerPheProLysLysAlaAsnLeuArgSerProLysPheAlaMETAla
              149                                     185              201

                                  HindII
                                  |
208 TCCACCCTCGGATCATCCACACCGAAGGTTGACAATGCCAAGAAGCCTTTTCAACCTCCACGAGAGGTT  276
    SerThrLeuGlySerSerThrProLysValAspAsnAlaLysLysProPheGlnProProArgGluVal
                                  238

277 CATGTTCAGGTGACGCACTCCATGCCACCACAGAAGATAGAGATTTTCAAATCCATCGAGGGTTGGGCT  345
    HisValGlnValThrHisSerMETProProGlnLysIleGluIlePheLysSerIleGluGlyTrpAla

346 GAGCAGAACATATTGGTTCACCTAAAGCCAGTGGAGAAATGTTGGCAAGCACAGGATTTCTTGCCGGAC  414
    GluGlnAsnIleLeuValHisLeuLysProValGluLysCysTrpGlnAlaGlnAspPheLeuProAsp
```

FIGURE 2

```
415 CCTGCATCTGAAGGATTTGATGAACAAGTCAAGGAACTAAGGGCAAGAGCAAAGGAGATTCCTGATGAT  483
    ProAlaSerGluGlyPheAspGluGlnValLysGluLeuArgAlaArgAlaLysGluIleProAspAsp

484 TACTTTGTTGTTTTGGTTGGAGATATGATTACAGAGGAAGCCCTACCTACTTACCAAACAATGCTTAAT  552
    TyrPheValValLeuValGlyAspMETIleThrGluGluAlaLeuProThrTyrGlnThrMETLeuAsn

553 ACCCTAGATGGTGTACGTGATGAGACTGGGGCTAGCCTTACGCCTTGGGCTGTCTGGACTAGGGCTTGG  621
    ThrLeuAspGlyValArgAspGluThrGlyAlaSerLeuThrProTrpAlaValTrpThrArgAlaTrp

         PvuII                                                        AccI
         |                                                            |
622 ACAGCTGAAGAGAACAGGCATGGCGATCTTCTCCACACCTATCTCTACCTTTCTGGGCGGGTAGACATG  690
    ThrAlaGluGluAsnArgHisGlyAspLeuLeuHisThrTyrLeuTyrLeuSerGlyArgValAspMET
         626                                                          684

                                                     BamHI
                                                     |
691 AGGCAGATACAGAAGACAATTCAGTATCTCATTGGGTCAGGAATGGATCCTCGTACCGAAAACAGCCCC  759
    ArgGlnIleGlnLysThrIleGlnTyrLeuIleGlySerGlyMETAspProArgThrGluAsnSerPro
                                                     736

760 TACCTTGGGTTCATCTACACATCGTTTCAAGAGCGTGCCACATTTGTTTCTCACGGAAACACCGCCAGG  828
    TyrLeuGlyPheIleTyrThrSerPheGlnGluArgAlaThrPheValSerHisGlyAsnThrAlaArg
```

FIGURE 2

```
     SphI
     |
829 CATGCAAAGGATCATGGGGACGTGAAACTGGCGCAAATTTGTGGTACAATCGCGTCTGACGAAAAGCGT  897
    HisAlaLysAspHisGlyAspValLysLeuAlaGlnIleCysGlyThrIleAlaSerAspGluLysArg
     833

                                                          ClaI
                                                          |
898 CACGAGACCGCTTATACAAAGATAGTCGAAAAGCTATTCGAGATCGATCCTGATGGCACCGTTCTTGCT  966
    HisGluThrAlaTyrThrLysIleValGluLysLeuPheGluIleAspProAspGlyThrValLeuAla
                                                          942

                              BglII
                              |
967 TTTGCCGACATGATGAGGAAAAAGATCTCGATGCCCGCACACTTGATGTACGATGGGCGTGATGACAAC 1035
    PheAlaAspMETMETArgLysLysIleSerMETProAlaHisLeuMETTyrAspGlyArgAspAspAsn
                              990

                                                      AccI
                                                      |
1036 CTCTTCGAACATTTCTCGGCGGTTGCCCAAAGACTCGGCGTCTACACCGCCAAAGACTACGCCGACATA 1104
     LeuPheGluHisPheSerAlaValAlaGlnArgLeuGlyValTyrThrAlaLysAspTyrAlaAspIle
                                                      1077

1105 CTGGAATTTCTGGTCGGGCGGTGGAAAGTGGCGGATTTGACCGGCCTATCTGGTGAAGGGCGTAAAGCG 1173
     LeuGluPheLeuValGlyArgTrpLysValAlaAspLeuThrGlyLeuSerGlyGluGlyArgLysAla
```

FIGURE 2

```
                                                                SacI
                                                                |
1174 CAAGATTATGTTTGCGGGTTGCCACCAAGAATCAGAAGGCTGGAGGAGAGAGCTCAAGGGCGAGCAAAG 1242
     GlnAspTyrValCysGlyLeuProProArgIleArgArgLeuGluGluArgAlaGlnGlyArgAlaLys
                                                                1228

                            PvuII
                            |
1243 GAAGGACCTGTTGTTCCATTCAGCTGGATTTTCGATAGACAGGTGAAGCTGTGAAGAAAAAAAAAACGA 1311
     GluGlyProValValProPheSerTrpIlePheAspArgGlnValLysLeu
                            1266

1312 GCAGTGAGTTCGGTTTCTGTTGGCTTATTGGGTAGAGGTTAAAACCTATTTTAGATGTCTGTTTCGTGT 1380

1381 AATGTGGTTTTTTTTCTTCTAATCTTGAATCTGGTATTGTGTCGTTGAGTTCGCGTGTGTGTAAACTTG 1449

1450 TGTGGCTGTGGACATATTATAGAACTCGTTATGCCAATTTTGATGACGGTGGTTATCGTCTCCCCTGGT 1518

1519 GTTTTTTTATTGTTT 1533
```

FIGURE 2

```
  1 AAAAGAAAAAGGTAAGAAAAAAAACAATGGCTCTCAAGCTCAATCCTTTCCTTTCTCAAACCCAAAAGT  69
                                            METAlaLeuLysLeuAsnProPheLeuSerGlnThrGlnLysL

                                                        BglII
                                                          |
 70 TACCTTCTTTCGCTCTTCCACCAATGGCCAGTACCAGATCTCCTAAGTTCTACATGGCCTCTACCCTCA 138
    euProSerPheAlaLeuProProMETAlaSerThrArgSerProLysPheTyrMETAlaSerThrLeuL

139 AGTCTGGTTCTAAGGAAGTTGAGAATCTCAAGAAGCCTTTCATGCCTCCTCGGGAGGTACATGTTCAGG 207
    ysSerGlySerLysGluValGluAsnLeuLysLysProPheMETProProArgGluValHisValGlnV

208 TTACCCATTCTATTGCCA 225
    alThrHisSerIleAla
```

FIGURE 3A

```
AAAAGAAAAA GGTAAGAAAA AAAACA ATG GCT CTC AAG CTC AAT CCT TTC CTT TCT       56
                             MET Ala Leu Lys Leu Asn Pro Phe Leu Ser

CAA ACC CAA AAG TTA CCT TCT TTC GCT CTT CCA CCA ATG GCC AGT ACC AGA TCT   110
Gln Thr Gln Lys Leu Pro Ser Phe Ala Leu Pro Pro MET Ala Ser Thr Arg Ser

CCT AAG TTC TAC ATG GCC TCT ACC CTC AAG TCT GGT TCT AAG GAA GTT GAG AAT   164
Pro Lys Phe Tyr MET Ala Ser Thr Leu Lys Ser Gly Ser Lys Glu Val Glu Asn

CTC AAG AAG CCT TTC ATG CCT CCT CGG GAG GTA CAT GTT CAG GTT ACC CAT TCT   218
Leu Lys Lys Pro Phe MET Pro Pro Arg Glu Val His Val Gln Val Thr His Ser

ATG CCA CCC CAA AAG ATT GAG ATC TTT AAA TCC CTA GAC AAT TGG GCT GAG GAG   272
MET Pro Pro Gln Lys Ile Glu Ile Phe Lys Ser Leu Asp Asn Trp Ala Glu Glu

AAC ATT CTG GTT CAT CTG AAG CCA GTT GAG AAA TGT TGG CAA CCG CAG GAT TTT   326
Asn Ile Leu Val His Leu Lys Pro Val Glu Lys Cys Trp Gln Pro Gln Asp Phe

TTG CCA GAT CCC GCC TCT GAT GGA TTT GAT GAG CAA GTC AGG GAA CTC AGG GAG   380
Leu Pro Asp Pro Ala Ser Asp Gly Phe Asp Glu Gln Val Arg Glu Leu Arg Glu

AGA GCA AAG GAG ATT CCT GAT GAT TAT TTT GTT GTT TTG GTT GGA GAC ATG ATA   434
Arg Ala Lys Glu Ile Pro Asp Asp Tyr Phe Val Val Leu Val Gly Asp MET Ile

ACG GAA GAA GCC CTT CCC ACT TAT CAA ACA ATG CTG AAT ACC TTG GAT GGA GTT   488
Thr Glu Glu Ala Leu Pro Thr Tyr Gln Thr MET Leu Asn Thr Leu Asp Gly Val
```

FIGURE 3B

```
CGG GAT GAA ACA GGT GCA AGT CCT ACT TCT TGG GCA ATT TGG ACA AGG GCA TGG     542
Arg Asp Glu Thr Gly Ala Ser Pro Thr Ser Trp Ala Ile Trp Thr Arg Ala Trp

ACT GCG GAA GAG AAT AGA CAT GGT GAC CTC CTC AAT AAG TAT CTC TAC CTA TCT     596
Thr Ala Glu Glu Asn Arg His Gly Asp Leu Leu Asn Lys Tyr Leu Tyr Leu Ser

GGA CGA GTG GAC ATG AGG CAA ATT GAG AAG ACA ATT CAA TAT TTG ATT GGT TCA     650
Gly Arg Val Asp MET Arg Gln Ile Glu Lys Thr Ile Gln Tyr Leu Ile Gly Ser

GGA ATG GAT CCA CGG ACA GAA AAC AGT CCA TAC CTT GGG TTC ATC TAT ACA TCA     704
Gly MET Asp Pro Arg Thr Glu Asn Ser Pro Tyr Leu Gly Phe Ile Tyr Thr Ser

TTC CAG GAA AGG GCA ACC TTC ATT TCT CAT GGG AAC ACT GCC CGA CAA GCC AAA     758
Phe Gln Glu Arg Ala Thr Phe Ile Ser His Gly Asn Thr Ala Arg Gln Ala Lys

GAG CAT GGA GAC ATA AAG TTG GCT CAA ATA TGT GGT ACA ATT GCT GCA GAT GAG     812
Glu His Gly Asp Ile Lys Leu Ala Gln Ile Cys Gly Thr Ile Ala Ala Asp Glu

AAG CGC CAT GAG ACA GCC TAC ACA AAG ATA GTG GAA AAA CTC TTT GAG ATT GAT     866
Lys Arg His Glu Thr Ala Tyr Thr Lys Ile Val Glu Lys Leu Phe Glu Ile Asp

CCT GAT GGA ACT GTT TTG GCT TTT GCT GAT ATG ATG AGA AAG AAA ATT TCT ATG     920
Pro Asp Gly Thr Val Leu Ala Phe Ala Asp MET MET Arg Lys Lys Ile Ser MET

CCT GCA CAC TTG ATG TAT GAT GGC CGA GAT GAT AAT CTT TTT GAC CAC TTT TCA     974
Pro Ala His Leu MET Tyr Asp Gly Arg Asp Asp Asn Leu Phe Asp His Phe Ser
```

FIGURE 3B

```
GCT GTT GCG CAG CGT CTT GGA GTC TAC ACA GCA AAG GAT TAT GCA GAT ATA TTG        1028
Ala Val Ala Gln Arg Leu Gly Val Tyr Thr Ala Lys Asp Tyr Ala Asp Ile Leu

GAG TTC TTG GTG GGC AGA TGG AAG GTG GAT AAA CTA ACG GGC CTT TCA GCT GAG        1082
Glu Phe Leu Val Gly Arg Trp Lys Val Asp Lys Leu Thr Gly Leu Ser Ala Glu

GGA CAA AAG GCT CAG GAC TAT GTT TGT CGG TTA CCT CCA AGA ATT AGA AGG CTG        1136
Gly Gln Lys Ala Gln Asp Tyr Val Cys Arg Leu Pro Pro Arg Ile Arg Arg Leu

GAA GAG AGA GCT CAA GGA AGG GCA AAG GAA GCA CCC ACC ATG CCT TTC AGC TGG        1190
Glu Glu Arg Ala Gln Gly Arg Ala Lys Glu Ala Pro Thr MET Pro Phe Ser Trp

ATT TTC GAT AGG CAA GTG AAG CTG TAGGTGGCTA AAGTGCAGGA CGAAACCGAA ATGGTTAGTT  1254
Ile Phe Asp Arg Gln Val Lys Leu

TCACTCTTTT TCATGCCCAT CCCTGCAGAA TCAGAAGTAG AGGTAGAATT TTGTAGTTGC TTTTTTATTA  1324

CAAGTCCAGT TTAGTTTAAG GTCTGTGGAA GGGAGTTAGT TGAGGAGTGA ATTTAGTAAG TTGTAGATAC  1394

AGTTGTTTCT TGTGTTGTCA TGAGTATGCT GATAGAGAGC AGCTGTAGTT TTGTTGTTGT GTTCTTTTAT  1464

ATGGTCTCTT GTATGAGTTT CTTTTCTTTC CTTTTCTTCT TTCCTTTCCT CTCTCTCTCT CTCTCTCTCT  1534

CTCTTTTTCT CTTATCCCAA GTGTCTCAAG TATAATAAGC AAACGATCCA TGTGGCAATT TTGATGATGG  1604

TGATCAGTCT CACAACTTGA TCTTTTGTCT TCTATTGGAA ACACAGCCTG CTTGTTTGAA AAAA        1668
```

FIGURE 3B

**pCGN3235**

```
 1 TGAGAGATAGTGTGAGAGCATTAGCCTTAGAGAGAGAGAGAGAGAGCTTGTGTCTGAAAGAATCCACAA   69

              HindIII
              |
70 ATGGCATTGAAGCTTAACCCTTTGGCATCTCAGCCTTACAACTTCCCT 117
   METAlaLeuLysLeuAsnProLeuAlaSerGlnProTyrAsnPhePro
```

FIGURE 4A

**pCGN3236**

```
                                          PstI
                                          |
  1 ACTTCATGGGCTATTTGGACAAGAGCTTGGACTGCAGAAGAGAACCGACACGGTGATCTTCTCAATAAG  69
    ThrSerTrpAlaIleTrpThrArgAlaTrpThrAlaGluGluAsnArgHisGlyAspLeuLeuAsnLys

 70 TATCTTTACTTGTCTGGACGTGTTGACATGAGGCAGATTGAAAAGACCATTCAGTACTTGATTGGTTCT 138
    TyrLeuTyrLeuSerGlyArgValAspMETArgGlnIleGluLysThrIleGlnTyrLeuIleGlySer

         BamHI
         |
139 GGAATGGATCCTAGAACAGAGAACAATCCTTACCTCGG 176
    GlyMETAspProArgThrGluAsnAsnProTyrLeuAla
```

FIGURE 4B

**pCGN3235**

```
TGAGAGATAG TGTGAGAGCA TTAGCCTTAG AGAGAGAGAG AGAGAGCTTG TGTCTGAAAG AATCCACAA

ATG GCA TTG AAG CTT AAC CCT TTG GCA TCT CAG CCT TAC AAC TTC CCT TCC TCG
MET Ala Leu Lys Leu Asn Pro Leu Ala Ser Gln Pro Tyr Asn Phe Pro Ser Ser

GCT CGT CCG CCA ATC TCT ACT TTC AGA TCT CCC AAG TTC CTC TGC CTC GCT TCT
Ala Arg Pro Pro Ile Ser Thr Phe Arg Ser Pro Lys Phe Leu Cys Leu Ala Ser

TCT TCT CCC GCT CTC AGC TCC AAG GAG GTT GAG AGT TTG AAG AAG CCA TTC ACA
Ser Ser Pro Ala Leu Ser Ser Lys Glu Val Glu Ser Leu Lys Lys Pro Phe Thr

CCA CCT AAG GAA GTG CAC GTT CAA GTC CTG CAT TCC ATG CCA CCC CAG AAG ATC
Pro Pro Lys Glu Val His Val Gln Val Leu His Ser MET Pro Pro Gln Lys Ile

GAG ATC TTC AAA TCC ATG GAA GAC TGG GCC GAG CAG AAC CTT CTA ACT CAG CTC
Glu Ile Phe Lys Ser MET Glu Asp Trp Ala Glu Gln Asn Leu Leu Thr Gln Leu

AAA GAC GTG GAG AAG TCG TGG CAG CCC CAG GAC TTC TTA CCC GAC CCT GCA TCC
Lys Asp Val Glu Lys Ser Trp Gln Pro Gln Asp Phe Leu Pro Asp Pro Ala Ser

GAT GGG TTC GAA GAT CAG GTT AGA GAG CTA AGA GAG AGG GCA AGA GAG CTC CCT
Asp Gly Phe Glu Asp Gln Val Arg Glu Leu Arg Glu Arg Ala Arg Glu Leu Pro

GAT GAT TAC TTC GTT GTT CTG GTG GGA GAC ATG ATC ACG GAA GAG GCG CTT CCG
Asp Asp Tyr Phe Val Val Leu Val Gly Asp MET Ile Thr Glu Glu Ala Leu Pro
```

FIGURE 4C

ACC TAT CAA ACC ATG TTG AAC ACT TTG GAT GGA GTG AGG GAT GAA ACT GGC GCT
Thr Tyr Gln Thr MET Leu Asn Thr Leu Asp Gly Val Arg Asp Glu Thr Gly Ala

AGC CCC ACT TCA TGG GCT ATT TGG ACA AGA GCT TGG ACT GCA GAA GAG AAC CGA
Ser Pro Thr Ser Trp Ala Ile Trp Thr Arg Ala Trp Thr Ala Glu Glu Asn Arg

CAC GGT GAT CTT CTC AAT AAG TAT CTT TAC TTG TCT GGA CGT GTT GAC ATG AGG
His Gly Asp Leu Leu Asn Lys Tyr Leu Tyr Leu Ser Gly Arg Val Asp MET Arg

CAG ATT GAA AAG ACC ATT CAG TAC TTG ATT GGT TCT GGA ATG GAT CCT AGA ACA
Gln Ile Glu Lys Thr Ile Gln Tyr Leu Ile Gly Ser Gly MET Asp Pro Arg Thr

GAG AAC AAT CCT TAC CTC GGC TTC ATC TAC ACT TCA TTC CAA GAA AGA GCC ACC
Glu Asn Asn Pro Tyr Leu Gly Phe Ile Tyr Thr Ser Phe Gln Glu Arg Ala Thr

TTC ATC TCT CAC GGA AAC ACA GCT CGC CAA GCC AAA GAG CAC GGA GAC CTC AAG
Phe Ile Ser His Gly Asn Thr Ala Arg Gln Ala Lys Glu His Gly Asp Leu Lys

CTA GCC CAA ATC TGC GGC ACA ATA GCT GCA GAC GAG AAG CGT CAT GAG ACA GCT
Leu Ala Gln Ile Cys Gly Thr Ile Ala Ala Asp Glu Lys Arg His Glu Thr Ala

TAC ACC AAG ATA GTT GAG AAG CTC TTT GAG ATT GAT CCT GAT GGT ACT GTG ATG
Tyr Thr Lys Ile Val Glu Lys Leu Phe Glu Ile Asp Pro Asp Gly Thr Val MET

GCG TTT GCA GAC ATG ATG AGG AAG AAA ATC TCG ATG CCT GCT CAC TTG ATG TAC
Ala Phe Ala Asp MET MET Arg Lys Lys Ile Ser MET Pro Ala His Leu MET Tyr

FIGURE 4C

```
GAT GGG CGG GAT GAA AGC CTC TTT GAC AAC TTC TCT TCT GTT GCT CAG AGG CTC
Asp Gly Arg Asp Glu Ser Leu Phe Asp Asn Phe Ser Ser Val Ala Gln Arg Leu

GGT GTT TAC ACT GCC AAA GAC TAT GCG GAC ATT CTT GAG TTT TTG GTT GGG AGG
Gly Val Tyr Thr Ala Lys Asp Tyr Ala Asp Ile Leu Glu Phe Leu Val Gly Arg

TGG AAG ATT GAG AGC TTG ACC GGG CTT TCA GGT GAA GGA AAC AAA GCG CAA GAG
Trp Lys Ile Glu Ser Leu Thr Gly Leu Ser Gly Glu Gly Asn Lys Ala Gln Glu

TAC TTG TGT GGG TTG ACT CCA AGA ATC AGG AGG TTG GAT GAG AGA GCT CAA GCA
Tyr Leu Cys Gly Leu Thr Pro Arg Ile Arg Arg Leu Asp Glu Arg Ala Gln Ala

AGA GCC AAG AAA GGA CCC AAG GTT CCT TTC AGC TGG ATA CAT GAC AGA GAA GTG
Arg Ala Lys Lys Gly Pro Lys Val Pro Phe Ser Trp Ile His Asp Arg Glu Val

CAG CTC TAA AAAGGAA CAAAGCTATG AAACCTTTTC ACTCTCCGTC GTCCCTCATT TGATCTATCT
Gln Leu  *

GCTCTTGAAA TTGGTGTAGA TTACTATGGT TTGTGATATT GTTCGTGGGT CTAGTTACAA AGTTGAGAAG

CAGTGATTTA GTAGCTTTGT TGTTTCCAGT CTTTAAATGT TTTTGTGTTT GGTCCTTTTA GTAAACTTGT

TGTAGTTAAA TCAGTTGAAC TGTTTGGTCT GT
```

FIGURE 4C

```
GAT GCC AAA ANG CCT CAC ATG CCT CCT AGA GAA GCT CAT GTG CAA AAG    48
Asp Ala Lys Xaa Pro His MET Pro Pro Arg Glu Ala His Val Gln Lys
 1               5                  10                  15

ACC CAT TCA ATK CCG CCT CAA AAG ATT GAG ATT TTC AAA TCC TTG GAG    96
Thr His Ser Xaa Pro Pro Gln Lys Ile Glu Ile Phe Lys Ser Leu Glu
            20                  25                  30

GGT TGG GCT GAG GAG AAT GTC TTG GTG CAT CTT AAA CCT GTG GAG AA    143
Gly Trp Ala Glu Glu Asn Val Leu Val His Leu Lys Pro Val Glu
        35                  40                  45
```

FIGURE 5

```
Amino Acid
Sequence From
Fragment F2    K   E   I   P   D   D   Y  FVVLVGDMITEEALPTY  Q   T   M   L   N   T

               AAA GAA AUU CCN GAU GAU UAU                    CAA ACN AUG CUN AAU AC/N
                 G   G   C       C   C   C                      G               C

                       A
                                        Forward Primers:
    5'GCTAAGCTT AAP GAP ATQ CCA GAQ GAQ TA3'  Desat 13-1
                          A CCG               Desat 13-2
                            CCC               Desat 13-3
                            CCT               Desat 13-4


                                        Reverse Primers:
                                        (complements)

                                         Desat 13-5a   3' GTQ TGN TAC GAN TTP TGCTTAAGCGA 5'
                                         Desat 13-6a                  AAQ
```

<u>Oligonucleotides</u>

P = A or G
Q = T or C
N = A, C, T or C

FIGURE 6

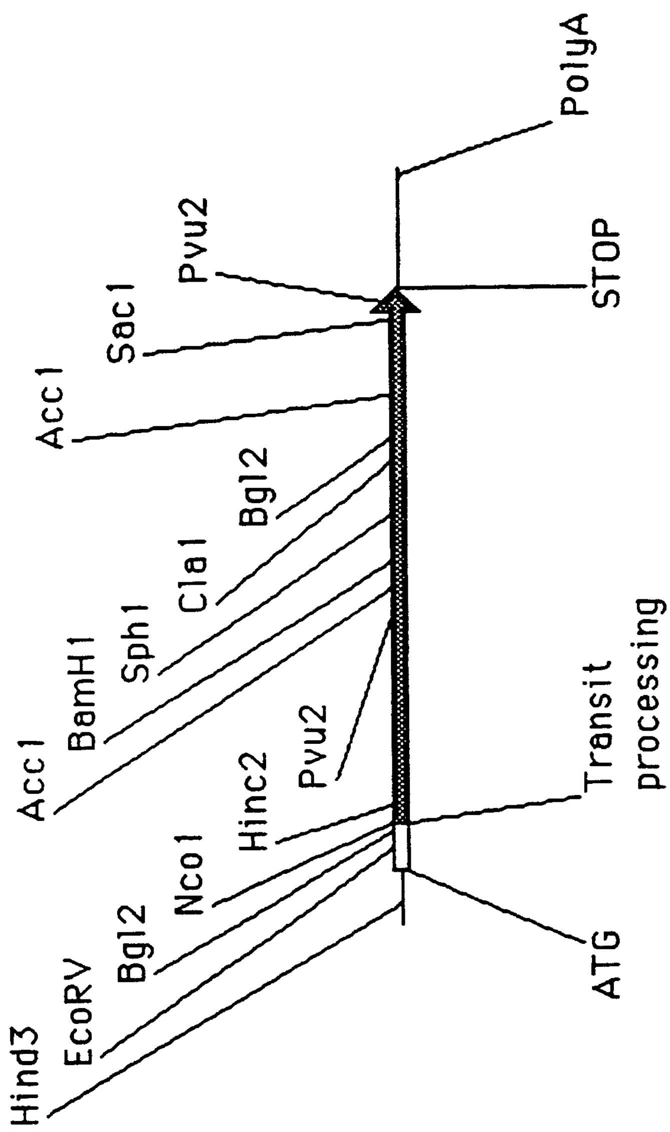
Hind3
EcoRV
Bgl2
Nco1
Hinc2
Pvu2
Acc1
BamH1
Sph1
Cla1
Bgl2
Acc1
Sac1
Pvu2
PolyA
STOP
Transit processing site
ATG

FIGURE 7A

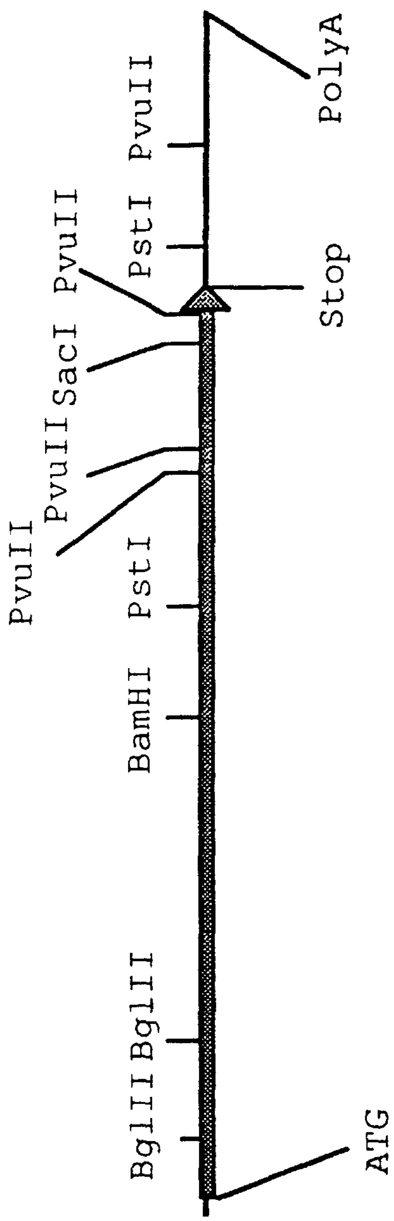
ATG
BglII
BglII
BamHI
PstI
PvuII
PvuII
SacI
PvuII
Stop
PstI
PvuII
PolyA

FIGURE 7B

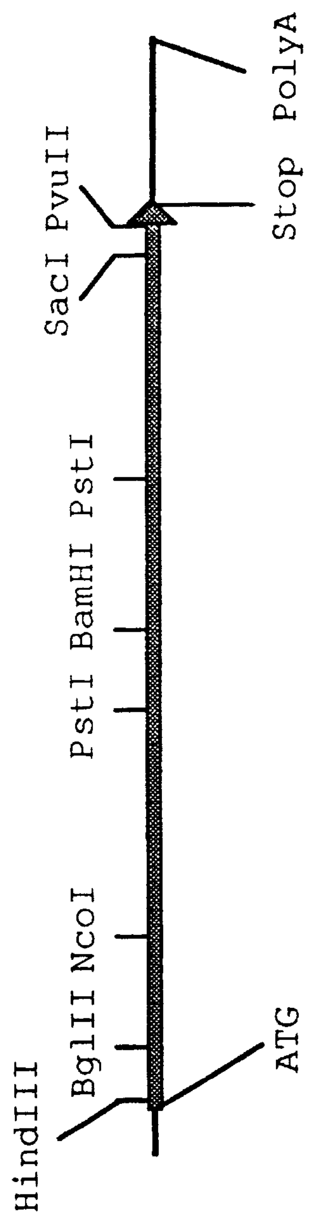
HindIII
BglII
NcoI
PstI
BamHI
PstI
SacI
PvuII
ATG
Stop
PolyA

FIGURE 7C

TCTAGAATTC TCTAATTACG TCTGTTTGTT CTATTTTTTA TATGATATCA AATATTCGTC ATAAATATAT 70

GGTTTAAGAT GCCAAAAAAT TATTTACTTG GTGAATATAA TACGTTAAAT ATTAGAAATA CATCATTTAG 140

TTAAATAAAT AACCAAAAAC CAAAAATTCA TATCCGCGCT GGCGCGCGGT CAGGGTCTCG TTAGTTTTAA 210

AATCAATGCA GTTTACAATT AATTTCCAGC TGAAAATAAG TATAATTTGT ATTGAAATTA TAAAGTGACA 280

TTTTTTGTGT AACAAATATT TTGTGTAACA AGAATTAAAA AAAAAAACAG AAAATACTCA GCTTTTTTAA 350

TAATAAAAAA AATTAATTGA GTTAGAAAAT TGTTGTACCA ATAACAAAAG ATTTATATGG AATTATAAAA 420

TCAACACACC AATAACACAA GACTTTTTAA AAATTTAAGA ATAATATAAG CAATAACAAT AGAATCTTCA 490

AATTCTTCAA ATCCTTAAAA ATCAATCTCC CACTATTAAT CCCCCTTAGT TTTAGTTGGT AATGGCAACG 560

TTTGTTGACT ACCGTATTGT AACTTTTGTC AAATTGTCAT AAATACGTGT CAAACTCTGG TAAAAAATTA 630

GTCTGCTACA TCTGTCTTTT ATTTATAAAA CACAGCTGTT AATCAGAATT TGGTTTATTA AATCAACAAC 700

CTGCACGAAA CTTGTGTGAG CATATTTTGT CTGTTTCTGG TTCATGACCT TCTTCCGCAT GATGGCCAAG 770

TGTAATGGCC ACTTGCAAGA GCGTTTCTTC AACGAGATAA GTCGAACAAA TATTTGTCCG TTACGACCAC 840

ATATAAAATC TCCCCATCTC TATATATAAT ACCAGCATTC ACCATCATGA ATACCTCAAA TCCCAATCTC 910

ACAAATACTT CAATAAAAAG ACCAAAAAAA ATTAAAGCAA AGAAAAGCCT TCTTGTGCAC AAAAAAAAAA 980

FIGURE 8

```
GAAGCCTTCT AGGTTTTCAC GAC ATG AAG TTC ACT ACT CTA ATG GTC ATC ACA TTG       1036
                          MET Lys Phe Thr Thr Leu MET Val Ile Thr Leu

GTG ATA ATC GCC ATC TCG TCT CCT GTT CCA ATT AGA GCA ACC ACG GTT GAA AGT     1090
Val Ile Ile Ala Ile Ser Ser Pro Val Pro Ile Arg Ala Thr Thr Val Glu Ser

TTC GGA GAA GTG GCA CAA TCG TGT GTT GTG ACA GAA CTC GCC CCA TGC TTA CCA     1144
Phe Gly Glu Val Ala Gln Ser Cys Val Val Thr Glu Leu Ala Pro Cys Leu Pro

GCA ATG ACC ACG GCA GGA GAC CCG ACT ACA GAA TGC TGC GAC AAA CTG GTA GAG     1198
Ala MET Thr Thr Ala Gly Asp Pro Thr Thr Glu Cys Cys Asp Lys Leu Val Glu

CAG AAA CCA TGT CTT TGT GGT TAT ATT CGA AAC CCA GCC TAT AGT ATG TAT GTT     1252
Gln Lys Pro Cys Leu Cys Gly Tyr Ile Arg Asn Pro Ala Tyr Ser MET Tyr Val

ACT TCT CCA AAC GGT CGC AAA GTC TTA GAT TTT TGT AAG GTT CCT TTT CCT AGT     1306
Thr Ser Pro Asn Gly Arg Lys Val Leu Asp Phe Cys Lys Val Pro Phe Pro Ser

TGT TAAATCTCTC AAGACATTGC TAAGAAAAAT ATTATTAAAA ATAAAAGAAT CAAACTAGAT       1369
Cys

CTGATGTAAC AATGAATCAT CATGTTATGG TTGAAGCTTA TATGCTGAAG TGTTTGATTT TATATATGTG  1439

TGTGTGTGTG TCCTGCTCAA TTTTTGAAAC ACACACGTTT CTCCTGATTT GGATTTAAAT TATATTTTGA  1509

GTTAAAAAAA AGAAAAAGAT GGAATGCTAT TTATACAAGT TGATGAAAAA GTGGAAGTAC AATTTAGATA  1579
```

FIGURE 8

```
TCTCCTACAC TTAAAGAATG AAACAATAAT AGACTTACGA AACAAATGAA AAATACATAA ATTGTCGACA  1649
ATCAACGTCC GATGACGAGT TTATTATTAA AAATTTGTGT GAAGGACTAG CAGTTCAACC AAATGATATT  1719
GAACATATAC ATCAACAAAT ATGATAATCA TAAAAGAGAG AATGGGGGGG GGGTGTCGTT TACCAGAAAC  1789
CTCTTTTTCT CAGCTCGCTA AAACCCTACC ACTAGAGACC TAGCTCTGAC CGTCGGCTCA TCGGTGCCGG  1859
AGGTGTAACC TTTCTTTCCC ATGACCCGAA ACCTCTCTTT CCCAACTCAC GAAAACCCTA CAATCAAAAA  1929
CCTAGCTCCG ACCGTCGGCT CATCGGTGCC GAAGGTGTAA CCTTTCTCTC CCATCATAGT TTCTCGTAAA  1999
TGAAAGCTAA TTGGGCAATC GATTTTTTAA TGTTTAAACC ATGCCAAGCC ATTTCTTATA GGACAATTGT  2069
CAATAATAGC ATCTTTTGAG TTTTGTCTCA AAAGTGACAC TAGAAGAAAA AAGTCACAAA AATGACATTC  2139
ATTAAAAAGT AAAATATCCC TAATACCTTT GGTTTAAATT AAATAAGTAA ACAAAAATAA ATAAAAACAA  2209
ATAAAATAAA AATAAAAAAT GAAAAAAAGA AATTTTTTTA TAGTTTCAGA TTATATGTTT TCAGATTCGA  2279
AATTTTTTAA ATTCCCTTTT TTAAATTTTC TTTTTTGAAA TTTTTTTTTT TGAAATTTTT TGAAACTGTT  2349
TTTAAAATTT TTATTTTTAA TTTTTTAGTA TTTATTTTTT ATTTTATAAA ATTTTAAACG CTAATTCCAA  2419
AACTCCCCCC CCCCCCCCCC CCCCAATTCT CTCCTAGTCT TTTTCTCTTT CTTATATTTG GGCTTCTATC  2489
TTCTCTTTTT TTTTCAGGCC CAAAGTATCA TGTGTAACAA CCGGTGTTCA AAAACGCGCC CGCCTGGCCG  2559
```

FIGURE 8

```
TTTACTCGCC CGATTAAATG ATGATCGGAA GGCTGCCATG GCGAGGCGGA GGTAATCAGT GGTTCTAGGC  2629
GCTGAAACTA GAAAACCTTC AAAAATCGAA ATTTTAAGAG CTAAATCGGT GTTTATCTCA TGAATCTATT  2699
ATATTTAGTT GAAACTCACA AGAATCGGTT GTAAAAACTA TGAAATCGTG CAAAAAAAAT GAAGAACAAA  2769
ATATTCTCAG ATCTGGAAAA CACAGAGAAG AGGTTGAAGA TGAGGGTAAA ATCGTATTTT GTCATTCATT  2839
AAACTAAAAT CAAAAAAAAA TGATGCAAAA TTCAATGATA ATAACTCGAA CTCGCAACCA TATGCATCTT  2909
TAGACTGCGA CACGGACCAC TAGACTAAGC AATTTTAATG TTTATTCATC ACAGACCTAA TATATGTCTA  2979
AAACTAGGCG CCGAGTACGC CCCGCTTAAT CCCGAGTTTT TGTTAGCTCG CTAGACCCAG GGTCACCGCC  3049
CGACTAACGA GTAGCGTAAT TCTGAACTGG GGTAACAACA TAGAGAACAT CGCCGACCCT TCCCTGCCGA  3119
TGATGCCGCC TCCGATGAAC TTCCTGTAAC GCCTTCAGTT TCCATTGATT TTCCCCTTTA ATCTGATCAG  3189
TTCCATGTTT TATCCAACTC ATCCCACTCC GTAGCATTTA ATCGATCTCA TCATTTACAT ACATAACCAG  3259
TAGGAGGTCT CATATAAATT TGAACGTTTC CAGCGATGAA CAGTGCCAAT CTCTGCGAAA TCCATTTCTC  3329
TAAGCTCAGG GCTGGCGGCT GCAGCCCGGG GATCCACTAG TTCTAGGCGG CCGCACCGCG GTGGAGCTCC  3399
AATTCGCCCT ATAGTGAGTC GTATTACGCG CGCTCACTGG C                                 3440
```

FIGURE 8

```
      XhoI
      |
  1 CTCGAGAGCTGAAGGATTTTTTGTTAGAGATTCAACGACAGATGGACCCTTCCTCCACTAGGCAACTGC  69
      2

 70 AAGAACCTAACAATGCAAATATCACTCCTCCTCAGCCTTCAAGGAGCGTTAATAGGACTGGAACAAGCG 138

                                 BglII
                                 |
139 GTCAAGTGAGTAAATTTTCCTTCCAAGATAGATCTCTATGGTTCGGTTCATGAAGTTTGTGGTTTAATT 207
                                 169

208 GTGTAGCAACAGGATAGTGCAAGTGAGAATAGAGTTCGACCTCATCTACCTACCCCGGAACCTCTGAAT 276

277 GTATCCCCATTGAAGAAGAAGAGGGCAAATCCTGCACCCAGAAGGATAAAGAAATTTTGGACGCCTGAA 345

346 GAAGTGGCAGTTCTGAGGGAAGGAGTAAAAGAGTATGTCTACTACTACTACTCTATAATCAAGTTTCAA 414

415 GAAGCTGAGCTTGGCTCTCACTTTATATGTTTGATGTTGTTGTGCAGGTATGGTAAATCATGGAAAGAG 483

484 ATAAAGAATGCAAACCCTGAAGTATTGGCAGAGAGGACTGAGGTGAGAGAGCATGTCACTTTTGTGTTA 552

553 CTCATCTGAATTATCTTATATGCGAATTGTAAGTGGTACTAAAAGGTTTGTAACTTTTGGTAGGTGGAT 621

622 TTGAAGGATAAATGGAGGAACTTGCTTCGGTAGCGGTAACAAGTTTTATATTGCTATGAAGTTTTTTTG 690

691 CCTGCGTGACGTATCAGCAGCTGTGGAGAAGATGGTATTAGAAAGGGTCTTTTCACATTTTGTGTTGTG 759
```

FIGURE 9

```
                                          BglII
                                          |
760  ACAAATATTAATTCGGCCGGTATGGTTTGGTTAAGACTTGTTGAGAGACGTGTGGGGTTTTTTGATGTA  828

829  TAATTAGTCTGTGTTTAGAACGAAACAAGACTTGTTGCGTATGCTTTTTTTAACTTGAGGGGGTTTGTT  897

                                               BglII
                                               |
898  GTTGTTAGTTAGGAACTTGACTTTGTCTCTTTCTCTCAAGATCTGATTGGTAAGGTCTGGGTGGTAGTA  966
                                               937

967  CTGTTTGGTTTAATTTGTTTTGACTATTGAGTCACTGTGGCCCATTGACTTTAAATTAGGCTGGTATAT 1035

1036 TTTTTGGTTTAAAACCGGTCTGAGATAGTGCAATTTCGATTCAGTCAATTTTAAATTCTTCAAGGTAAT 1104

1105 GGGCTGAATACTTGTATAGTTTTAAGACTTAACAGGCCTTAAAAGGCCCATGTTATCATAAAACGTCAT 1173

                     HindIII
                     |
1174 TGTTTAGAGTGCACCAAGCTTATAAAATGTAGCCAGGCCTTAAAAGACTTAACAGGCCTTAAAAGACTT 1242
                     1190

1243 AACATTCCTTAAAAGGCCCATGTTATCATAAAACGTCATCGTTTTGAGTGCACCAAGCTAAATGTAGCC 1311

1312 AGGCCTTAAAAGACTTAACAGGCCTTAAAAGGCCCATGTTATCATAAAACGCCGTCGTTTTGAGTGCAC 1380
```

FIGURE 9

```
     HindIII
     |
1381 CAAGCTTATAAATGTAGCCAGCTACCTCGGGACATCACGCTCTTTGTACACTCCGCCATCTCTCTCTCT 1449
     1383

      XhoI   BglII                       SalI
      |      |                           |
1450 CTCGAGCAGATCTCTCTCGGGAATATCGACAATGTCGACCACTTTCTGCTCTTCCGTCTCCATGCAAGC 1518
      1451   1458                        1484

1519 CACTTCTCTGGTAATCTCATCTCCTTCTTGTGTTCCCAGATCGCTCTGATCATACTTTCTTTTAGATCA 1587

1588 TTTGCCTCTGATCTGTTGCTTGATGTTTGTTAACTCTCCACGCATGTTTGATTATGTTGAGAATTAGAA 1656

1657 AAAAAATGTTAGCTTTACGAATCTTTAGTGATCATTTCAATTGGATTTGCAATCTTGTGTGACATTTGA 1725

1726 GGCTTGTGTAGATTTCGATCTGTATTCATTTTGAATCACAGCTATAATAGTCATTTGAGTAGTAGTGTT 1794

1795 TTTAAATGAACATGTTTTGTTGTATTGATGGAACAAACAGGCAGCAACAACGAGGATTAGTTTCCAGAA 1863

1864 GCCAGCTTTGGTTTCAACGACTAATCTCTCCTTCAACCTCCGCCGTTCAATCCCCACTCGTTTCTCAAT 1932

1933 CTCCTGCGCGGTATGTTCTCATTCTCAGCATTTATTTCGAGCTTGCTTGTCATGGTACTCTCTCTAATT 2001

2002 GTCTATTTGGTTTATTAGGCCAAACCAGAGACGGTTGAGAAAGTGTCTAAGATAGTTAAGAAGCAGCTA 2070
```

FIGURE 9

```
                                                                        EcoRV
                                                                        |
2071 TCACTCAAAGACGACCAAAAGGTCGTTGCGGAGACCAAGTTTGCTGATCTTGGAGCAGATTCTCTCGAC 2139

2140 ACTGTAAGTCATCAATCATTCTCTTATGTGAATAAAGAGAACTTGAAGAGTTTGTTTTTAACATATTAA 2208

                                                            EcoRV
                                                            |
2209 CTGAGTGTTTTGCATGCAGGTTGAGATAGTGATGGGTTTAGAGGAAGAGTTTGATATCGAAATGGCTGA 2277
                                                            2264

                                                              SstI
                                                              |
2278 AGAGAAAGCTCAGAAGATTGCTACTGTGGAGGAAGCTGCTGAACTCATTGAAGAGCTCGTTCAACTTAA 2346
                                                              2335

2347 GAAGTAATTTTAGTATTAAGAGCAGCCAAGGCTTTGTTGGGTTTGTTGTTTTCATAATCTTCCTGTCAT 2415

2416 TTTCTTTTTCTTTAATGTGTCAAGCGACTCTGTTGGTTTAAAGTAGTATCTGTTTGCCATGGATCTCTC 2484

        SalI                          HindIII
        |                             |
2485 TCTATTTGTCGACTGAAAACTTTTGGTTTACACATGAAAGCTTGTTCTTGTTCTTTCTTAAATCGAAAT 2553
        2493                          2523

2554 GCCAAATGCGAGATTAGGGAATCTTGTATTAACACATACATAAGTCAAAGAGTAGGCCCTAAGATGACA 2622

2623 ATTTATAAACAATCCTATTCACATTGTATATACAGGTTATGATTATTCCCAATCAGCGTCAAAGAATCC 2691
```

FIGURE 9

2692 AGCATCTTTCATCTCTGAATAGTAGACATTCTCCAAGTTCACATCTTCCTCCTGCACCAAAAACCAGTA 2760

2761 CTAAATCATGAACATTGCAATAATCACATGCCTAGGCGAGAGTTTTGGTGATGTGGTGTTAGTGATAGT 2829

2830 GATACTGATGGTGCTAGAGCGGTTAAGAAGGATTAACCTGGAAGAAGTCTGCAAGGAAAGTAACATAGA 2898

2899 GAAGAGGAAGATAGGAGTGGTAACAAACACTTGTGATCCCATACAGCCTCCCAGCATTTTTCAAATGTT 2967

2968 ATTTCCTTACATAAAGAAACAAGAGAAGTCTGACTAGATGATATTTATATAGGATAAGTGTTTTACCAT 3036

3037 AAGCCAAAGTGAGCGCCGTTTGCAAGAGCTAACCAGACAGTACACGTTTGGCATATATCTCATCAACAT 3105

3106 GATCTGAAAAGTAACATATCACAGTTAATGAACACAATGGTTACCTTGAGAAGCAAATCAAGACCTATA 3174

3175 ACAAGCCCAGAGATGAGGAAAGTCCGTGTCAACGCTTCACCGCCATTCGCGTAGTTTCCTTGGAAGACA 3243

3244 AAGGCCACCAACCAAACTTACTTCCAGAAACAACACTCCAAATGTTGTCAACAAAGTCAATAGATTCCA 3312

3313 AACTACTTCGTTACAGGGTTGTATAGATAATATAATAGAATAGTGGGAAGATAGTATAAATAAAATAAA 3381

3382 TAAAAGATCCTATCGGTAAATAGTTTATAATATCGGGGGCGTATATAAAGTATAAAAGAAACTCTTCTC 3450

3451 CAATCCGACCGTTGAAAATCACTCTCAATCTCTGGCGTAACGACCGGATCGTTCGCGCGTAATTTTCGC 3519

3520 TGCTATAAATAGAAACTTTCCTCTTCTGTTTCTCGATCAAAATTTTTTTTGGAAAAAATTAAGTTTGAA 3588

FIGURE 9

```
3589 TCTATCGTAGATGCTGTGACAAAAAAAAATTGTTTTATCGAAGATGAGAAACATGAGGCCTGTTCATGC 3657

                                BamHI
                                |
3658 AAGGAACCAGACCACGGATCCATCTTCGCCGATGATGACGTCTCCTCTGATGAATCGTCACGCACGGAC 3726
                                3674

      BamHI
      |
3727 AGGATCCAACGCTGGACCAGCATCTAACGCCAAGAAAGCACAGACGAAAGCAGCAGCTCAGAGACTCGC 3795
      3729

3796 GGCTGTGATGTCGAACCAAACAGGCGACGATGAAGACAGTGATGATGACCTTTCCTTTGACTACAACGC 3864

                                          BglII
                                          |
3865 TGTCGGAAGCATTGGTCTCGCTGCCGGAAGATCT 3898
                                          3894
```

FIGURE 9

Lambda CGN1-2

NCG-186 Linear LENGTH = 4325

```
                                                   SduI
        XhoI                                    NlaIV
        AvaI                                       HgiJII        HindIII
        |                                       |  |           |
     1 CTCGAGGCAGTCACTAACATGAAGTTTGACGAGGAGCCCAACTATGGGAAGCTTATTTCTCTTTTCGAT   69
        2                                          39          50
                                                36

                                XbaI                     SacI
                                |                        |
    70 ACTCTAATTGAGCCGTGCGCTCTATCTAGACCAATTAGAATTGATGGAGCTCTAAAGGTTGCTGGCTGT  138
                                95                       121

                NdeI                           SspI                      NdeI
                |                              |                         |
   139 TTTCTTGTTCATATGATTAACTTCTAAACTTGTGTATAAATATTCTCTGAAAGTGCTTCTTTTGGCATA  207
                150                            180                       206

                                           Ksp632I
                                           |
   208 TGTAGGTTGGGCAAAAACGAGGAAGATTGCTTCTCAATTTGGAAGAGGATGAACAGCCGAAGAAGAAAA  276
                                           245
```

FIGURE 10

```
                                    XhoII
                                    |
277 TAAGAATAGGCAGTCCTGCTACTCAATGGATCTCAGTCTATAACGGTCGTCGTCCCATGAAACAGAGGT  345
                                    305

                                                             MmeI   EcoRV
                                                             |      |
346 AAAACATTTTTTGCATATACACTTTGAAAGTTCCTCACTAACTGTGTAATCTTTTGGTAGATATCACTA  414
                                                             401    408

                                   SduI
                                MstI
                             BclI HgiAI                    HaeI
                             |  |  |                        |
415 CAATGTCGGAGAGACAA3GGCTGMNCANCATATACAAAAGGGAAATGAAGATGGCCTTTTGATTAGCTG  483
                             437  442                       469
                                439

                                    SduI
                                    HgiJII
                                    |
484 TGTAGCATCAGCAGCTAATCTCTGGGCTCTCATCATGGATGCTGGAACTGGATTCACTTCTCAAGTTTA  552
                                    512
```

FIGURE 10

```
             Cfr10I
          BbvII
          |    |
553 TGAGTTGTCACCGGTCTTCCTACACAAGGTAATAATCAGTTGAAGCAATTAAGAATCAATTTGATTTGT  621
          560
             563

622 AGTAAACTAAGAAGAACTTACCTTATGTTTTCCCCGCAGGACTGGATTATGGAACAATGGGAAAAGAAC  690

                                         SacI
                                         |
691 TACTATATAAGCTCCATAGCTGGTTCAGATAACGGGAGCTCTTTAGTTGTTATGTCAAAAGGTTAGTGT  759
                                         731

                        BbvII
                        |
760 TTAGTGAATAATAAACTTATACCACAAAGTCTTCATTGACTTATTTATATACTTGTTGTGAATTGCTAG  828
                        782

829 GAACTACTTATTCTCAGCAGTCATACAAAGTGAGTGACTCATTTCCGTTCAAGTGGATAAATAAGAAAT  897

898 GGAAAGAAGATTTTCATGTAACCTCCATGACAACTGCTGGTAATCGTTGGGGTGTGGTAATGTCGAGGA  966

                  BclI
                  |
967 ACTCTGGCTTCTCTGATCAGGTAGGTTTTGTCTCTTATTGTCTGGTGTTTTTATTTTCCCCTGATAGT 1035
                  981
```

FIGURE 10

```
1036 CTAATATGATAAACTCTGCGTTGTGAAAGGTGGTGGAGCTTGACTTTTTGTACCCAAGCGATGGGATAC 1104

1105 ATAGGAGGTGGGAGAATGGGTATAGAATAACATCAATGGCAGCAACTGCGGATCAAGCAGCTTTCATAT 1173

      Tth111II                                                          ScaI
      |                                                                 |
1174 TAAGCATACCAAAGCGTAAGATGGTGGATGAAACTCAAGAGACTCTCCGCACCACCGCCTTTCCAAGTA 1242
      1175                                                              1242

                                                 XhoII
                                                 |
1243 CTCATGTCAAGGTTGGTTTCTTTAGCTTTGAACACAGATTTGGATCTTTTTGTTTTGTTTCCATATACT 1311
                                                 1285

1312 TAGGACCTGAGAGCTTTTGGTTGATTTTTTTTTCAGGACAAATGGGCGAAGAATCTGTACATTGCATCA 1380

                                           AflII
                                           |
1381 ATATGCTATGGCAGGACAGTGTGCTGATACACACTTAAGCATCATGTGGAAAGCCAAAGACAATTGGAG 1449
                                           1415

1450 CGAGACTCAGGGTCGTCATAATACCAATCAAAGACGTAAAACCAGACGCAACCTCTTTGGTTGAATGTA 1518

                                                                        SspI
                                                                        |
1519 ATGAAAGGGATGTGTCTTGGTATGTATGTACGAATAACAAAAGAGAAGATGGAATTAGTAGTAGAAATA 1587
                                                                        1587
```

FIGURE 10

```
                                                               EcoRV
                                                               |
1588 TTTGGGAGCTTTTTAAGCCCTTCAAGTGTGCTTTTTATCTTATTGATATCATCCATTTGCGTTGTTTAA 1656
                                                               1635

           XbaI
           |
1657 TGCGTCTCTAGATATGTTCCTATATCTTTCTCAGTGTCTGATAAGTGAAATGTGAGAAAACCATACCAA 1725
           1664

            SspI
            |
1726 ACCAAAATATTCAAATCTTATTTTTAATAATGTTGAATCACTCGGAGTTGCCACCTTCTGTGCCAATTG 1794
            1734                                                     1789

                                                                      EcoRI
                                                                      |
1795 TGCTGAATCTATCACACTAGAAAAAAACATTTCTTCAAGGTAATGACTTGTGGACTATGTTCTGAATTC 1863
                                                                      1859

                                                        Eco57I
                                                        |
1864 TCATTAAGTTTTTATTTTCTGAAGTTTAAGTTTTTACCTTCTGTTTTGAAATATATCGTTCATAAGATG 1932
                                                        1904
```

FIGURE 10

```
                                                SphI
                                                NspI
                                                |
1933 TCACGCCAGGACATGAGCTACACATCGCACATAGCATGCAGATCAGGACGATTTGTCACTCACTTCAAA 2001
                                                1971

                                                    SphI
                                             NdeI   NspI           PmaCI
                        Tth111II              [AvaIII]  SspI AflIII
                        |                     ||     |   |    |  |
2002 CACCTAAGAGCTTCTCTCTCACAGCGCACACACATATGCATGCAATATTTACACGTGATCGCCATGCAA 2070
                        2015                   2037      2048 2053
                                             2036   2044         2056

                                      SecI
                                      |
2071 ATCTCCATTCTCACCTATAAATTAGAGCCTCGGCTTCACTCTTTACTCAAACCAAAACTCATCACTACA 2139
                                      2099

                                         Ksp632I
                                         |
2140 GAACATACACAAATGGCGAACAAGCTCTTCCTCGTCTCGGCAACTCTCGCCTTGTTCTTCCTTCTCACC 2208
                        METAlaAsnLysLeuPheLeuValSerAlaThrLeuAlaLeuPhePheLeuLeuThr
                                         2171
```

FIGURE 10

```
                                    SalI
                                      HindII                     NaeI
                AccI                 AccI                      Cfr10I
                |                   |||                        | |
2209 AATGCCTCCGTCTACAGGACGGTTGTGGAAGTCGACGAAGATGATGCCACAAATCCAGCCGGCCCATTT 2277
     AsnAlaSerValTyrArgThrValValGluValAspGluAspAspAlaThrAsnProAlaGlyProPhe
                2220                 2241                      2267
                                      2242                       2269
                                    2240

                                                    Tth111II
                                                    HindIII          NlaIV
                                                    |                |
2278 AGGATTCCAAAATGTAGGAAGGAGTTTCAGCAAGCACAACACCTGAAAGCTTGCCAACAATGGCTCCAC 2346
     ArgIleProLysCysArgLysGluPheGlnGlnAlaGlnHisLeuLysAlaCysGlnGlnTrpLeuHis
                                                    2325             2342

                     Tth111II             NlaIV                          BbvII
                     |                    |                              |
2347 AAGCAGGCAATGCAGTCCGGTAGTGGTCCAAGCTGGACCCTCGATGGTGAGTTTGATTTTGAAGACGAC 2415
     LysGlnAlaMETGlnSerGlySerGlyProSerTrpThrLeuAspGlyGluPheAspPheGluAspAsp
                     2363                 2384                           2415

                         NlaIV                                        SacI
                           ApaI  GsuI HaeI  NspBII                       Ksp632
                         | |     |    |     |                         |  |
2416 GTGGAGAACCAACAACAGGGCCCGCAGCAGAGGCCACCGCTGCTCCAGCAGTGCTGCAACGAGCTCCAC 2484
     ValGluAsnGlnGlnGlnGlyProGlnGlnArgProProLeuLeuGlnGlnCysCysAsnGluLeuHis
                           2438  2444 2449  2455                      2481
                         2436                                            2484
```

FIGURE 10

```
2485 CAGGAAGAGCCACTTTGCGTTTGCCCAACCTTGAAAGGAGCATCCAAAGCCGTTAAACAACAGATTCGA 2553
     GlnGluGluProLeuCysValCysProThrLeuLysGlyAlaSerLysAlaValLysGlnGlnIleArg

2554 CAACAACAGGGACAACAAATGCAGGGACAGCAGATGCAGCAAGTGATTAGCCGTATCTACCAGACCGCT 2622
     GlnGlnGlnGlyGlnGlnMETGlnGlyGlnGlnMETGlnGlnValIleSerArgIleTyrGlnThrAla

                                                                      SecI
                                                                   BbvII
                                                                   |  |
2623 ACGCACTTACCTAGAGCTTGCAACATCAGGCAAGTTAGCATTTGCCCCTTCCAGAAGACCATGCCTGGG 2691
     ThrHisLeuProArgAlaCysAsnIleArgGlnValSerIleCysProPheGlnLysThrMETProGly
                                                                   2684
                                                                      2687

     NlaIV                                               SecI
       HgiJII                          XhoI              DsaI
       ApaI                            AvaI        AccI
     | |                               |           |   |
2692 CCCGGCTTCTACTAGATTCCAAACGAATATCCTCGAGAGTGTGTATACCACGGTGATATGAGTGTGGTT 2760
     ProGlyPheTyr .
       2694                            2724        2736
     2692                                              2740

                  HpaI
                  HindII
                  |
2761 GTTGATGTATGTTAACACTACATAGTCATGGTGTGTGTTCCATAAATAATGTACTAATGTAATAAGAAC 2829
                  2774
```

FIGURE 10

```
             AccI
             |
2830 TACTCCGTAGACGGTAATAAAAGAGAAGTTTTTTTTTACTCTTGCTACTTTCCTATAAAGTGATGAT 2898
             2838

                                                                  SpeI
                                 VspI                            ScaI
                                 |                               ||
2899 TAACAACAGATACACCAAAAAGAAAACAATTAATCTATATTCACAATGAAGCAGTACTAGTCTATTGAA 2967
                                 2929                            2954
                                                                  2955

         NspI
     AflIII
     |   |
2968 CATGTCAGATTTTCTTTTTCTAAATGTCTAATTAAGCCTTCAAGGCTAGTGATGATAAAAGATCATCCA 3036
     2968
         2972

         XhoII
           NlaIV                        MmeI
         BamHI                          BclI
         | |                            |
3037 ATGGGATCCAACAAAGACTCAAATCTGGTTTTGATCAGATACTTCAAAACTATTTTTGTATTCATTAAA 3105
         3041                           3069
           3043
```

FIGURE 10

```
                                 BbvII                    Tth111
                                 |                        |
3106 TTATGCAAGTGTTCTTTTATTTGGTGAAGACTCTTTAGAAGCAAAGAACGACAAGCAGTAATAAAAAAA 3174
                                 3139                     3174

                                                      VspI
                                                      |
3175 ACAAAGTTCAGTTTTAAGATTTGTTATTGACTTATTGTCATTTGAAAAATATAGTATGATATTAATATA 3243
                                                      3237

        Tth111II                              VspI
        |                                     |
3244 GTTTTATTTATATAATGCTTGTCTATTCAAGATTTGAGAACATTAATATGATACTGTCCACATATCCAA 3312

        3250                                  3287

                             NdeI      Tth111II
                             |         |
3313 TATATTAAGTTTCATTTCTGTTCAAACATATGATAAGATGGTCAAATGATTATGAGTTTTGTTATTTAC 3381
                             3341      3352

                        Eco57I                        Eco57I
                        |                             |
3382 CTGAAGAAAAGATAAGTGAGCTTCGAGTTTCTGAAGGGTACGTGATCTTCATTTCTTGGCTAAAAGCGA 3450
                        3404                          3434

3451 ATATGACATCACCTAGAGAAAGCCGATAATAGTAAACTCTGTTCTTGGTTTTTGGTTTAATCAAACCGA 3519
```

FIGURE 10

```
                                                   Tth111II
      Cfr10I                                       NdeI
      |                                            ||
3520 ACCGGTAGCTGAGTGTCAAGTCAGCAAACATCGCAAACCATATGTCAATTCGTTAGATTCCCGGTTTAA 3588
      3521                                         3560
                                                    3561

          Cfr10I
          |
3589 GTTGTAAACCGGTATTTCATTTGGTGAAAACCCTAGAAGCCAGCCANCCTTTTTAATCTAATTTTTGCA 3657
          3597

                                                                 NlaIV
                                                                 HindII
                                                                HgiCI
                                                                 BspHI
                                                                |||
3658 AACGAGAAGTCACCACACCTCTCCACTAAAACCCTGAACCTTACTGAGAGAAGCAGAGNCANNAAAGAA 3726
                                                                 3717
                                                                3716
                                                                 3718

           Eco31I          PmaCI                  Ksp632I
           |               |                      |
3727 CAAATAAAACCCGAAGATGAGACCACCACGTGCGGCGGGACGTTCAGGGGACGGGGAGGAAGAGAATGR 3795
           3740            3756                   3781

3796 CGGCGG5MNTTTGGTGGCGGCGGCGGACGTTTTGGTGGCGGCGGTGGACGTTTTGGTGGCGGCGGTGGA 3864
```

FIGURE 10

```
                EcoRV
                |
3865 CCTTTGGTGGTGGATATCGTGACGAAGGACCTCCCAGTGAAGTCATTGGTTCGTTTACTCTTTTCTTAG 3933
                3880

                                            HindIII
                                               AflII
                                            |  |
3934 TCGAATCTTATTCTTGCTCTGCTCGTTGTTTTACCGATAAAGCTTAAGACTTTATTGATAAAGTTCTCA 4002
                                               3977
                                            3974

4003 GCTTTGAATGTGAATGAACTGTTTCCTGCTTATTAGTGTTCCTTTGTTTTGAGTTGAATCACTGTCTTA 4071

4072 GCACTTTTGTTAGATTCATCTTTGTGTTTAAGTTAAAAGGTAGAAACTTTGTGACTTGTCTCCGTTATG 4140

         HpaI
         HindII                        Tth111II
         |                             |
4141 ACAAGGTTAACTTTGTTGGTTATAACAGAAGTTGCGACCTTTCTCCATGCTTGTGAGGGTGATGCTGTG 4209
         4149                          4179

                      XhoII
                      |
4210 GACCAAGCTCTCTCAGGCGAAGATCCCTTACTTCAATGCCCCAATCTACTTGGAAAACAAGACACAGAT 4278
                      4231
```

FIGURE 10

```
                                               SalI
                                             PstI
                     HindIII                   HindII
                XhoII   BspMI                 AccI EcoRI
                |      | |          |  |||    |
4279 TGGGAAAGTTGATGAGATCCAAGCTTGGGCTGCAGGTCGACGAATTC 4325
                4294    4302              4316 4321
                     4300                  4317
                                    4313
                                      4315
```

FIGURE 10